# EUROPEAN PATENT APPLICATION

(11) **EP 2 883 883 A1**
(43) Date of publication of application: **17.06.2015**
(21) Application number: 13197532.8
(22) Date of filing: 16.12.2013
(51) Int. Cl.: C07K 14/705, A61P 9/10

(54) **Therapeutic targets and agents useful in treating ischemia reperfusion injury**

(71) Applicant: Cardio3 Biosciences S.A., 1435 Mont-Saint-Guibert (BE)
(72) Inventor: De Waele, Peter, 9080 Lochristi (BE); Daro, Dorothée, 1495 Marbais (BE)
(74) Representative: De Clercq, Ann G. Y.

(57) **Abstract**

The application teaches a therapeutic agent for use in the treatment of cardiac warm ischemia reperfusion injury, wherein said therapeutic agent is capable of interacting with at least one of the CD80 or CD86 polypeptides, and wherein said interaction interferes with the binding of the at least one of the CD80 or CD86 polypeptides to the CD28 polypeptide. The application also teaches a therapeutic agent for use in the treatment of cardiac warm ischemia reperfusion injury, wherein said therapeutic agent is capable of preventing or inhibiting the expression of at least one of the CD80 or CD86 polypeptides by a cell or on the surface of a cell.

SEQUENCE LISTING

<110>  Cardio3 Biosciences S.A.

<120>  Therapeutic targets and agents useful in treating ischemia
       reperfusion injury

<130>  C3B-001-EP-PRIO

<160>  4

<170>  PatentIn version 3.5

<210>  1
<211>  76
<212>  PRT
<213>  artificial sequence

<220>
<223>  fragment of antibody


<220>
<221>  MISC_FEATURE
<222>  (3)..(3)
<223>  X is G, A, V, L, I, P, F, M, W, C, N, Q, S, T, Y, D, E, K, R or
       H, preferably G, A, V, L, P, F, M, W, C, N, Q, S, T, Y, D, E, K,
       R or H

<220>
<221>  MISC_FEATURE
<222>  (11)..(11)
<223>  X is G, A, V, L, I, P, F, M, W, C, N, Q, S, T, Y, D, E, K, R or H

<220>
<221>  MISC_FEATURE
<222>  (19)..(19)
<223>  X is G, A, V, L, I, P, F, M, W, C, N, Q, S, T, Y, D, E, K, R or H

<220>
<221>  MISC_FEATURE
<222>  (22)..(22)
<223>  X is G, A, V, L, I, P, F, M, W, C, N, Q, S, T, Y, D, E, K, R or H

<220>
<221>  MISC_FEATURE
<222>  (53)..(53)
<223>  X is G, A, V, L, I, P, F, M, W, C, N, Q, S, T, Y, D, E, K, R or H

<400>  1

Phe Ser Xaa Ser Arg Tyr Ser Val Tyr Trp Xaa Arg Gln Pro Pro Gly
1               5                   10                  15


Lys Gly Xaa Glu Trp Xaa Gly Met Met Trp Gly Gly Gly Ser Thr Asp
            20                  25                  30


Tyr Ser Thr Ser Leu Lys Ser Arg Leu Thr Ile Ser Lys Asp Thr Ser
        35                  40                  45

Lys Ser Gln Val Xaa Leu Lys Met Asn Ser Leu Arg Thr Asp Asp Thr
    50                  55                  60

Ala Met Tyr Tyr Cys Val Arg Thr Asp Gly Asp Tyr
65                  70                  75

<210>  2
<211>  113
<212>  PRT
<213>  artificial sequence

<220>
<223>  fragment of antibody

<220>
<221>  MISC_FEATURE
<222>  (29)..(29)
<223>  X is G, A, V, L, I, P, F, M, W, C, N, Q, S, T, Y, D, E, K, R or
       H, preferably G, A, V, L, P, F, M, W, C, N, Q, S, T, Y, D, E, K,
       R or H

<220>
<221>  MISC_FEATURE
<222>  (37)..(37)
<223>  X is G, A, V, L, I, P, F, M, W, C, N, Q, S, T, Y, D, E, K, R or H

<220>
<221>  MISC_FEATURE
<222>  (45)..(45)
<223>  X is G, A, V, L, I, P, F, M, W, C, N, Q, S, T, Y, D, E, K, R or H

<220>
<221>  MISC_FEATURE
<222>  (48)..(48)
<223>  X is G, A, V, L, I, P, F, M, W, C, N, Q, S, T, Y, D, E, K, R or H

<220>
<221>  MISC_FEATURE
<222>  (79)..(79)
<223>  X is G, A, V, L, I, P, F, M, W, C, N, Q, S, T, Y, D, E, K, R or H

<400>  2

Gln Val Lys Leu Gln Glu Ser Gly Pro Gly Leu Val Lys Pro Ser Glu
1               5                   10                  15

Thr Leu Ser Ile Thr Cys Thr Val Ser Gly Phe Ser Xaa Ser Arg Tyr
            20                  25                  30

Ser Val Tyr Trp Xaa Arg Gln Pro Pro Gly Lys Gly Xaa Glu Trp Xaa
        35                  40                  45

Gly Met Met Trp Gly Gly Gly Ser Thr Asp Tyr Ser Thr Ser Leu Lys
    50                  55                  60

Ser Arg Leu Thr Ile Ser Lys Asp Thr Ser Lys Ser Gln Val Xaa Leu

65                  70                  75                  80

Lys Met Asn Ser Leu Arg Thr Asp Asp Thr Ala Met Tyr Tyr Cys Val
                85                  90                  95

Arg Thr Asp Gly Asp Tyr Trp Gly Gln Gly Thr Thr Val Thr Val Ser
            100                 105                 110

Ser

<210>  3
<211>  89
<212>  PRT
<213>  fragment of antibody

<220>
<221>  MISC_FEATURE
<222>  (1)..(1)
<223>  X is N, Q, S, T, Y, W or C

<220>
<221>  MISC_FEATURE
<222>  (4)..(4)
<223>  X is D, E, N, Q, S, T, Y, W or C

<220>
<221>  MISC_FEATURE
<222>  (5)..(5)
<223>  X is N, Q, S, T, Y, G, A, V, L, I, P, F, M, W or C

<220>
<221>  MISC_FEATURE
<222>  (17)..(17)
<223>  X is G, A, V, L, I, P, F, M

<220>
<221>  MISC_FEATURE
<222>  (75)..(75)
<223>  X is G, A, V, L, I, P, F, M

<220>
<221>  MISC_FEATURE
<222>  (79)..(79)
<223>  X is N, Q, S, T, Y, G, A, V, L, I, P, F, M, W or C

<400>  3

Xaa Leu Gly Xaa Xaa Ala Ser Ile Ser Cys Arg Ser Ser Gln Ser Leu
1               5                   10                  15

Xaa Asn Ser Asn Gly Asn Thr Tyr Leu His Trp Tyr Leu Gln Arg Pro
            20                  25                  30

Gly Gln Ser Pro Arg Leu Leu Ile Tyr Lys Val Ser Asn Arg Phe Ser
        35                  40                  45

Gly Val Pro Asp Arg Phe Ser Gly Ser Gly Ser Gly Thr Asp Phe Thr
    50                  55                  60

Leu Lys Ile Ser Arg Val Glu Ala Glu Asp Xaa Gly Val Tyr Xaa Cys
65                  70                  75                  80

Ser Gln Ser Thr His Val Pro Trp Thr
                85

<210>  4
<211>  113
<212>  PRT
<213>  fragment of antibody

<220>
<221>  MISC_FEATURE
<222>  (14)..(14)
<223>  X is N, Q, S, T, Y, W or C

<220>
<221>  MISC_FEATURE
<222>  (17)..(17)
<223>  X is D, E, N, Q, S, T, Y, W or C

<220>
<221>  MISC_FEATURE
<222>  (18)..(18)
<223>  X is N, Q, S, T, Y, G, A, V, L, I, P, F, M, W or C

<220>
<221>  MISC_FEATURE
<222>  (30)..(30)
<223>  X is G, A, V, L, I, P, F, M

<220>
<221>  MISC_FEATURE
<222>  (88)..(88)
<223>  X is G, A, V, L, I, P, F, M

<220>
<221>  MISC_FEATURE
<222>  (92)..(92)
<223>  X is N, Q, S, T, Y, G, A, V, L, I, P, F, M, W or C

<400>  4

Glu Leu Gln Leu Thr Gln Ser Pro Leu Ser Leu Pro Val Xaa Leu Gly
1               5                   10                  15

Xaa Xaa Ala Ser Ile Ser Cys Arg Ser Ser Gln Ser Leu Xaa Asn Ser
            20                  25                  30

Asn Gly Asn Thr Tyr Leu His Trp Tyr Leu Gln Arg Pro Gly Gln Ser
        35                  40                  45

Pro Arg Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Xaa Gly Val Tyr Xaa Cys Ser Gln Ser
                85                  90                  95

Thr His Val Pro Trp Thr Phe Gly Gly Gly Thr Lys Leu Glu Ile Lys
            100                 105                 110

Arg

## Description

### FIELD

The invention is broadly in the medical field, more precisely in the field of treatment of heart ailments. In particular, the invention concerns therapeutic targets and therapeutic agents useful in the treatment of ischemia reperfusion injury, more precisely cardiac ischemia reperfusion injury, even more precisely cardiac warm ischemia reperfusion injury.

### BACKGROUND

The current interventional treatment of acute myocardial infarction (AMI), which focuses on restoring the blood flow to the myocardium (i.e., cardiac reperfusion) through thrombolytic therapy or interventional procedures, such as percutaneous coronary angioplasty, has significantly improved the clinical outcome of AMI by reducing both infarct size and mortality. However, cardiac reperfusion paradoxically causes serious adverse effects that arise from cardiac repair pathways occurring after infarct, which can lead to left ventricular remodelling and heart failure. This damage is often referred to as ischemia reperfusion injury (IRI), and is mainly responsible for the chronic complications observed in patients who survive severe AMI.

IRI is also observed in patients who undergo artery bypass grafting procedures, cardiac transplants and other surgical procedures that involve temporarily stopping the blood flow to the myocardium, and then restoring the blood flow.

The precise mechanism of IRI is not fully known yet, but it seems to involve amongst others the activation of a systemic inflammatory response, which particularly affects the already injured cardiomyocytes and endothelial cells in the ischemic zone, resulting in cell and tissue damage and ultimately, in loss of function.

There exists some experimental and clinical evidence suggesting a role for inflammation in cardiovascular diseases, and anti-inflammatory agents have been hypothesised for the treatment of cardiovascular diseases. For example, WO2005/016266 proposes the treatment of cardiovascular diseases using soluble CTLA-4 molecules that block endogenous B7 molecules, thereby suppressing T-cell responses.

The proliferation of naive T lymphocytes and their differentiation into effector cells require antigen recognition and additional signals provided by co-stimulatory molecules expressed on antigen presenting cells (APCs). Recognition of antigens can occur directly when the antigens are associated with the major histocompatibility complex (MHC) on the surface of foreign cells or tissues, or indirectly when the antigens are processed and then associated with the MHC on the surface of antigen presenting cells (APCs). Resting T lymphocytes that recognize such antigen-presenting MHC complexes become activated via association of these complexes with the T cell receptor. Such activated T cells require an additional signal (i.e., co-stimulation) to proliferate and become functional. The best-defined co-stimulatory pathways are the B7:CD28 pathway and the CD40:CD40 ligand (CD40L) pathway. The two known members of the B7 family, B7-1 (CD80) and B7-2(CD86), are expressed on APCs, and their expression is enhanced by microbes and innate immune responses. Recognition of B7 by CD28, which is constitutively expressed on the majority of T lymphocytes, appears to initiate the T cell response. In contrast, binding of CD80 and CD86 to the inhibitory receptor CTLA-4 down-regulates the T cell response. CD40 is constitutively expressed on APCs, and CD40L is induced on T cells upon antigen recognition. The interaction between CD40 and CD40L also enhances the T cell response.

Since the total infarct size is considered to be a critical determinant of the patient's risk to develop heart failure, and considering that cardiovascular diseases in general, and acute myocardial infarction in particular, are leading causes of death in the western world, there exists a pressing need for additional therapeutic targets useful in the treatment of cardiac ischemia reperfusion injury, more particularly cardiac warm ischemia reperfusion injury.

### SUMMARY

The inventors have realised the unexpected finding that inhibition of the T cell response has a beneficial effect in the treatment of cardiac warm ischemia reperfusion injury.

In particular, the inventors have put forward the co-stimulatory pathway B7:CD28, alone or in combination with the co-stimulatory pathway CD40:CD40L, as a useful and promising target for the treatment of cardiac warm ischemia reperfusion injury. Expanding on these findings, the inventors in particular recognised the co-stimulatory molecules CD80 (B7-1) and CD86 (B7-2) as valuable targets for the treatment of cardiac warm ischemia reperfusion injury.

Thus, among others the following aspects and embodiments are provided in accordance with the present invention:
A therapeutic agent for use in the treatment of cardiac warm ischemia reperfusion injury, wherein the therapeutic agent is capable of interacting with at least one of the CD80 or CD86 polypeptides, and wherein said interaction interferes with the binding of the at least one of the CD80 or CD86 polypeptides to the CD28 polypeptide.

A method for treating cardiac warm ischemia reperfusion injury in a subject in need of such treatment, comprising administering to said subject a therapeutically effective amount of a therapeutic agent, wherein the agent is capable of interacting with at least one of the CD80 or CD86 polypeptides, and wherein said interaction interferes with the binding of at least one of the CD80 or CD86 polypeptides to the CD28 polypeptide.

Use of a therapeutic agent, wherein the agent is capable of interacting with at least one of the CD80 or CD86 polypeptides, and wherein said interaction interferes with the binding of the at least one of the CD80 or CD86 polypeptides to the CD28 polypeptide, for the manufacture of a medicament for the treatment of cardiac warm ischemia reperfusion injury.

A therapeutic agent for use in the treatment of cardiac warm ischemia reperfusion injury, wherein the therapeutic agent is capable of preventing or inhibiting the expression of at least one of the CD80 or CD86 polypeptides by a cell or capable of preventing or inhibiting the presentation of at least one of the CD80 or CD86 polypeptides on the surface of a cell.

A method for treating cardiac warm ischemia reperfusion injury in a subject in need of such treatment, comprising administering to said subject a therapeutically effective amount of a therapeutic agent, wherein the therapeutic agent is capable of preventing or inhibiting the expression of at least one of the CD80 or CD86 polypeptides by a cell or capable of preventing or inhibiting the presentation of at least one of the CD80 or CD86 polypeptides on the surface of a cell.

Use of a therapeutic agent, wherein the therapeutic agent is capable of preventing or inhibiting the expression of at least one of the CD80 or CD86 polypeptides by a cell or capable of preventing or inhibiting the presentation of at least one of the CD80 or CD86 polypeptides on the surface of a cell, for the manufacture of a medicament for the treatment of cardiac warm ischemia reperfusion injury.

The above and further aspects and preferred embodiments of the invention are described in the following sections and in the appended claims. The subject matter of appended claims is hereby specifically incorporated in this specification.

### BRIEF DESCRIPTION OF DRAWINGS

Figure 1 illustrates an example of assessment of infarct size by creatine kinase (CKMB) measurement in the blood of a subject shortly post-infarction.
Figure 2 illustrates an example of assessment of infarct size by cardiac troponin I (cTnI) measurement in the blood of a subject shortly post-infarction.
Figure 3 illustrates an example of measurement of ejection fraction (EF) over time by magnetic resonance imaging (MRI).
Figure 4 illustrates an example of measurement of left ventricular end diastolic volume (LVEDV) over time by magnetic resonance imaging (MRI).
Figure 5 illustrates an example of measurement of left ventricular end systolic volume (LVESV) over time by MRI.
Figure 6 illustrates an example of measurement of scar size over time by MRI.
Figure 7 illustrates an example of scar size evaluation at necropsy.

### DESCRIPTION OF EMBODIMENTS

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. The terms also encompass "consisting of" and "consisting essentially of", which enjoy well-established meanings in patent terminology.

The recitation of numerical ranges by endpoints includes all numbers and fractions subsumed within the respective ranges, as well as the recited endpoints.

The terms "about" or "approximately" as used herein when referring to a measurable value such as a parameter, an amount, a temporal duration, and the like, are meant to encompass variations of and from the specified value, such as variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" or "approximately" refers is itself also specifically, and preferably, disclosed.

Whereas the terms "one or more" or "at least one", such as one or more members or at least one member of a group of members, is clear *per se,* by means of further exemplification, the term encompasses inter alia a reference to any one of said members, or to any two or more of said members, such as, e.g., any ≥3, ≥4, ≥5, ≥6 or ≥7 etc. of said members, and up to all said members. In another example, "one or more" or "at least one" may refer to 1, 2, 3, 4, 5, 6, 7 or more.

The discussion of the background to the invention herein is included to explain the context of the invention. This is not to be taken as an admission that any of the material referred to was published, known, or part of the common general knowledge in any country as of the priority date of any of the claims.

Throughout this disclosure, various publications, patents and published patent specifications are referenced by an identifying citation. All documents cited in the present specification are hereby incorporated by reference in their entirety. In particular, the teachings or sections of such documents herein specifically referred to are incorporated by reference.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the invention. When specific terms are defined in connection with a particular aspect of the invention or a particular embodiment of the invention, such connotation is meant to apply throughout this specification, i.e., also in the context of other aspects or embodiments of the invention, unless otherwise defined.

In the following passages, different aspects or embodiments of the invention are defined in more detail. Each aspect or embodiment so defined may be combined with any other aspect(s) or embodiment(s) unless clearly indicated to the contrary. In particular, any feature indicated as being preferred or advantageous may be combined with any other feature or features indicated as being preferred or advantageous.

Reference throughout this specification to "one embodiment", "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the appended claims, any of the claimed embodiments can be used in any combination.

As noted above, the inventors recognised the co-stimulatory pathway B7:CD28, alone or in combination with the co-stimulatory pathway CD40:CD40L, as a useful and promising target for the treatment of cardiac warm ischemia reperfusion injury (abbreviated herein as CWIRI). As used in the art and herein, the term "co-stimulatory pathway" denotes a T cell co-stimulatory pathway and refers to the interaction of a non-antigen molecule (i.e., co-stimulatory molecule) present on the surface of an antigen presenting cell with its respective binding partner on a T cell, which interaction together with the major histocompatibility complex (MHC)-antigen-T cell receptor interaction is required to induce proliferation and differentiation of the T cell. Hence, inhibition of these T cell responses has been unexpectedly realised by the present inventors to have a beneficial effect in the treatment of cardiac warm ischemia reperfusion injury.

Accordingly, an aspect of the invention relates to a therapeutic agent for use in the treatment of cardiac warm ischemia reperfusion injury, wherein the therapeutic agent is capable of interacting with at least one of the CD80 or CD86 polypeptides, and wherein said interaction interferes with the binding of the at least one of the CD80 or CD86 polypeptides to the CD28 polypeptide.

Another aspect of the invention relates to a therapeutic agent for use in the treatment of cardiac warm ischemia reperfusion injury, wherein the therapeutic agent is capable of preventing or inhibiting the expression of at least one of the CD80 or CD86 polypeptides by a cell or capable of preventing or inhibiting the presentation of at least one of the CD80 or CD86 polypeptides on the surface of a cell.

As used herein, the term "agent" broadly refers to any chemical (e.g., inorganic or organic), biochemical or biological substance, molecule or macromolecule (e.g., biological macromolecule), a combination or mixture thereof, a sample of undetermined composition, or an extract made from biological materials such as bacteria, plants, fungi, or animal cells or tissues. Preferred though non-limiting "agents" include nucleic acids, oligonucleotides, ribozymes, polypeptides or proteins, peptides, peptidomimetics, antibodies and fragments and derivatives thereof, aptamers, photoaptamers, chemical substances, preferably organic molecules, more preferably small organic molecules, lipids, carbohydrates, polysaccharides, etc., and any combinations thereof.

As used herein, the term "therapeutic agent" is used interchangeably with "drug", and refers to an agent as taught herein used in the treatment, cure, prevention, or diagnosis of a disease.

As used herein, the terms "treat", "treating", or "treatment", encompass both the therapeutic treatment of an already developed disease or condition, in particular the therapy of CWIRI, as well as prophylactic or preventative measures, wherein the aim is to prevent or lessen the chances of incidence of an undesired affliction, in particular the prevention of CWIRI. Beneficial or desired clinical results may include, without limitation, preventing injury or damage of cardiac tissue, diminishment of extent of injury or damage of cardiac tissue, not worsening injury or damage of cardiac tissue, healing of the injured or damaged cardiac tissue, reducing scar size, and the like. "Treatment" can also mean prolonging survival as compared to expected survival if not receiving treatment.

As used herein, the term "ischemia reperfusion injury" refers to the tissue or organ damage caused when blood supply returns (i.e., re-oxygenation) to the tissue or organ after a period of ischemia or lack of oxygen in the tissue or organ. By means of explanation, the absence of oxygen and nutrients from blood during the ischemic period creates a condition in which the restoration of circulation results in inflammation and oxidative damage through the induction of oxidative stress, rather than restoration of normal function. Oxidative stresses associated with reperfusion may cause damage to the affected tissues or organs. Ischemia reperfusion injury is thus characterized biochemically by a depletion of oxygen during an ischemic event followed by re-oxygenation and the concomitant generation of reactive oxygen species during reperfusion.

An ischemia reperfusion injury can be caused, for example, by restoration of blood flow following an ischemic infarction, such as a cerebral infarction, a myocardial infarction or a pulmonary infarction, venous or arterial occlusion, or restenosis; restoration of blood flow accompanying treatment of such infarctions, occlusions or restenoses; restoration of blood flow following cardiovascular arrest; restoration of blood flow following a trauma; or consequent to one or more surgical procedures or other therapeutic interventions that restore blood flow to a tissue or organ that has been subjected to a diminished supply of blood. Such surgical procedures include, for example, coronary artery bypass graft surgery, coronary angioplasty, vessel ligation and reperfusion during organ transplant surgery and the like.

The term "warm ischemia reperfusion injury" more specifically refers to ischemia reperfusion injury in a tissue or organ which has been maintained at or exposed to substantially physiological temperatures during the period of ischemia. Physiological temperatures may most typically refer to normothermia. By means of an example, in human subjects normothermia may specifically denote temperatures between about 36.00°C and about 37.50°C. Physiological temperatures may, however, also encompass hyperthermia, particularly where the extent of hyperthermia is compatible with the survival of the organism. By means of an example, in human subjects hyperthermia may specifically denote temperatures between about 37.51°C and about 41.00°C, preferably between about 37.51°C and about 40.00°C, more preferably between about 37.51°C and about 39.00°C, even more preferably between about 37.51°C and about 38.50°C. Physiological temperatures may also encompass hypothermia, particularly where the extent of hypothermia is compatible with the survival of the organism. By means of an example, in human subjects hypothermia may specifically denote temperatures between about 24.00°C and about 35.99°C, preferably between about 28.00°C and about 35.99°C, more preferably between about 31.00°C and about 35.99°C, even more preferably between about 32.00°C or about 33.00°C or about 34.00°C and about 35.99°C, still more preferably between about 35.00°C and about 35.99°C. The skilled person can readily ascertain physiological temperatures of warm-blooded animals, in particular mammals, from literature or by simple temperature measurements.

By means of an example, warm ischemia reperfusion injury may ensue upon reperfusion when the tissue or organ is present in or is not removed from the body during the period of ischemia. By means of another example, warm ischemia reperfusion injury may ensue upon reperfusion when the tissue or organ is not refrigerated during the period of ischemia, for example when the tissue or organ is temporarily removed from the body during that period. Refrigeration in this connection may in particular mean keeping the tissue or organ at (exposing the tissue or organ to) temperatures between about 2°C and about 8°C, such as between about 4°C and about 6°C.

The phrase "cardiac warm ischemia reperfusion injury" thus broadly encompasses warm ischemia reperfusion injury of the heart tissue or organ. More particularly, the phrase may refer to warm ischemia reperfusion injury of the muscular tissue of the heart (myocardium). Cardiac warm ischemia reperfusion injury as intended herein may for example be consequent to myocardial infarction, which may be chronic myocardial infarction or preferably acute (i.e., sudden and serious) myocardial infarction (STEMI), possibly exacerbated by treatment of the myocardial infarction aimed at restoring blood flow to the ischemic areas of the heart (e.g., anticoagulant therapy), cardiovascular arrest, coronary artery bypass graft surgery, coronary angioplasty, transient vasospasm, acute coronary syndromes (NSTEMI), cardioplegia, vessel ligation and reperfusion during heart transplant surgery and the like.

Accordingly, in certain embodiments, the cardiac warm ischemia reperfusion injury may be consequent to myocardial infarction, chronic myocardial infarction, acute myocardial infarction (STEMI), treatment of the myocardial infarction aimed at restoring blood flow to the ischemic areas of the heart, cardiovascular arrest, coronary artery bypass graft surgery, coronary angioplasty, transient vasospasm, acute coronary syndromes (NSTEMI), cardioplegia, or vessel ligation and reperfusion during heart transplant surgery.

Accordingly, in certain embodiments, the cardiac warm ischemia reperfusion injury may be consequent to acute myocardial infarction.

The term "interact" as used herein refers to the act or process in which two or more, such as two, agents influence each other. The interaction between agents, such as between the therapeutic agent as described in the present application and the at least one of the CD80 or CD86 polypeptides, or between the second therapeutic agent as described in the present application and the CD40 polypeptide, may be covalent (i.e., mediated by one or more chemical bonds that involve the sharing of electron pairs between atoms) or, more typically, non-covalent (i.e., mediated by non-covalent forces, such as for example, hydrogen bridges, dipolar interactions, van der Waals interactions, and the like).

Typically, an agent that interacts with another agent (which may be conveniently denoted as "a target") may thus bind to said another agent (target), such as the therapeutic agent as described in the present application may bind to the at least one of the CD80 or CD86 polypeptides, or the second therapeutic agent as described in the present application may bind to the CD40 polypeptide, more typically bind to said another agent with an affinity constant (K_{A}) of such binding K_{A} ≥ 1×10⁵ M⁻¹, preferably K_{A} ≥ 1×10⁶ M⁻¹, more preferably K_{A} ≥ 1×10⁷ M⁻¹, yet more preferably K_{A} ≥ 1×10⁸ M⁻¹, even more preferably K_{A} ≥ 1×10⁹ M⁻¹, and still more preferably K_{A} ≥ 1×10¹⁰ M⁻¹ or K_{A} ≥ 1×10¹¹ M⁻¹, wherein K_{A} = [A_T]/[ A][T], A denotes the agent, T denotes the target. Determination of K_{A} can be carried out by methods known in the art, such as for example, using bio-layer interferometry (BLI), equilibrium dialysis and Scatchard plot analysis.

Preferably, the interaction (such as binding) between an agent and a target, such as the interaction between the therapeutic agent as described in the present application and the at least one of the CD80 or CD86 polypeptides, or the interaction between the second therapeutic agent as described in the present application and the CD40 polypeptide, will be specific or selective, i.e., the agent will not substantially interact (such as bind) with random, unrelated molecules or substances. Hereby, the agent will also not substantially alter the activity and the level of such random, unrelated molecules or substances. Binding of an agent to a target may be evaluated *inter alia* using conventional interaction-querying methods, such as co-immunoprecipitation, immunoassay methods, chromatography methods, gel elecrophoresis methods, yeast two hybrid methods, or combinations thereof.

The interaction (such as binding) between an agent and a target, such as the interaction between the therapeutic agent as described in the present application and the at least one of the CD80 or CD86 polypeptides, or the interaction between the second therapeutic agent as described in the present application and the CD40 polypeptide, will desirably alter the activity or the level or both of said target. The reference to the "activity" is to be interpreted broadly and may generally encompass any one or more aspects of the biological activity of the target at any level (e.g., molecular, cellular and/or physiological), such as without limitation any one or more aspects of its biochemical activity, enzymatic activity, signalling activity, interaction activity, ligand activity, receptor activity or structural activity, e.g., within a cell, tissue, organ or an organism. By means of an example and not limitation, reference to the activity of the CD80 or CD86 polypeptides may particularly denote the activity of the CD80 or CD86 polypeptides as ligands, i.e., their ability to bind to one or more cognate receptors, in particular to the CD28 polypeptide, or to CTLA-4 polypeptide, or to both the CD28 and CTLA-4 polypeptides, and/or the of the CD80 or CD86 polypeptides as signalling molecules, i.e., their ability to participate in one or more cellular signalling pathways, in particular in the B7:CD28 co-stimulatory pathway. The reference to the "level" is to be interpreted broadly and may generally encompass the quantity and/or the availability (e.g., availability for performing its biological activity) of the target, e.g., within a cell, tissue, organ or an organism.

In particular, as intended in certain aspects, the interaction between the therapeutic agent as described in the present application and the at least one of the CD80 or CD86 polypeptides interferes with the binding of the at least one of the CD80 or CD86 polypeptides to the CD28 polypeptide, i.e., with the capability of the at least one of the CD80 or CD86 polypeptides to bind to the CD28 polypeptide. Thereby, the interaction between the therapeutic agent as described in the present application and the at least one of the CD80 or CD86 polypeptides can inhibit or prevent the production of the B7:CD28 co-stimulatory signal, and the proliferation and differentiation of T-cells. The phrase "interfere with" as used herein is intended to be synonymous with terms such as decrease", "reduce", "diminish", "inhibit", "disrupt", or "disturb", and denotes a qualitative or quantitative decrease of the binding that is being interfered with. The term encompasses any extent of such interference. For example, where the decrease in the binding affects a determinable or measurable variable, the interference may encompass a decrease in the value of said variable (commensurate with a decrease in the binding of at least one of the CD80 or CD86 polypeptides to the CD28 polypeptide) of at least about 10%, e.g., of at least about 20%, of at least about 30%, e.g., of at least about 40%, of at least about 50%, e.g., of at least about 60%, of at least about 70%, e.g., of at least about 80%, of at least about 90%, e.g., of at least about 95%, such as of at least about 96%, 97%, 98%, 99% or even of 100%, compared to a reference situation without said interference. Suitable molecular tests for measuring the binding of polypeptides, such as the binding of the at least one of the CD80 or CD86 polypeptides to the CD28 polypeptide, and for measuring the extent to which agents may interfere with such binding, are available to the skilled person. Further, suitable *in vitro* cellular tests for measuring the extent to which agents may interfere with binding of the at least one of the CD80 or CD86 polypeptides to the CD28 polypeptide may include testing the ability of such agents to interfere with induction of the T cell response in one-way allogeneic mixed lymphocytes reactions (one suitable endpoint in such tests being the EC50 value).

Similarly, as intended in certain embodiments, the interaction between the second therapeutic agent as described in the present application and the CD40 polypeptide interferes with the binding of the CD40 polypeptide to the CD40 ligand (CD40L) polypeptide, i.e., with the capability of the CD40 polypeptide to bind to the CD40L polypeptide. Any extent of such interference is encompassed. For example, where the decrease in the binding affects a determinable or measurable variable, the interference may encompass a decrease in the value of said variable (commensurate with a decrease in the binding of the CD40 polypeptide to the CD40L polypeptide) of at least about 10%, e.g., of at least about 20%, of at least about 30%, e.g., of at least about 40%, of at least about 50%, e.g., of at least about 60%, of at least about 70%, e.g., of at least about 80%, of at least about 90%, e.g., of at least about 95%, such as of at least about 96%, 97%, 98%, 99% or even of 100%, compared to a reference situation without said interference. Suitable molecular tests for measuring the binding of polypeptides, such as the binding of the CD40 polypeptide to the CD40L polypeptide, and for measuring the extent to which agents may interfere with such binding, are available to the skilled person. Further, suitable *in vitro* cellular tests for measuring the extent to which agents may interfere with binding of the CD40 polypeptide to the CD40L polypeptide may include testing the ability of such agents to interfere with induction of the T cell response in one-way allogeneic mixed lymphocytes reactions (one suitable endpoint in such tests being the EC50 value).

As used herein, the terms "cluster of differentiation 80" or "CD80" or B7-1 refer to a protein primarily found on antigen presenting cells (APCs) that provides a co-stimulatory signal necessary for T-cell activation and survival. The term encompasses CD80 expressed in APCs, as well as in any other cell type, such as, e.g., in endothelial cells. CD80 is the ligand for two different proteins on the T-cell surface, CD28 and CTLA-4.

The term "antigen presenting cell" is understood by those skilled in the art and by means of further explanation refers to any cell capable of displaying on its surface an antigen (such as in particular a foreign (exogenous) antigen) in association with a major histocompatibility complex (MHC) molecule (MHC class I or II) or portion thereof, or, one or more non-classical MHC molecules, or a portion thereof, such as to present the antigen in the MHC molecule context to T-cells. Antigen presenting cells are thus suitably also capable of processing proteins and polypeptides into peptides that may be presented in the context of MHC molecules. Antigen presenting cells (APCs) may include but are not limited to dendritic cells, macrophages and B-cells. The term "T-cell" is understood by those skilled in the art and by means of further explanation refers to a type of thymus-derived white blood cell that participates in a variety of cell-mediated immune reactions. The term may in particular but without limitation refer to CD8+ or CD4+ T-cells capable of lysing target cells or providing effector or helper functions, such as cytokine secretion, which can result in the death of target cells or the generation or enhancement of anti-target effector activity.

Exemplary but non-limiting *CD80* mRNA includes human *CD80* mRNA having nucleic acid sequence as annotated under Genbank (http://www.ncbi.nlm.nih.gov/) accession number NM_005191.3 (the number following the period denotes the Genbank sequence version). Exemplary but non-limiting CD80 protein includes human CD80 protein (precursor) having amino acid sequence as annotated under Genbank accession number NP_005182.1.

A skilled person can appreciate that in some instances sequences referred to herein may be of precursors (e.g., preproteins) and may include parts which are processed away from the mature proteins or polypeptides. A skilled person can also appreciate that any and all isoforms of proteins or polypeptides are to be encompassed herein, even where sequences referred to only illustrate one of such isoforms.

As used herein, the terms "cluster of differentiation 86" or "CD86" or B7-2 refer to a protein primarily found on antigen presenting cells that provides a co-stimulatory signal necessary for T-cell activation and survival. The term encompasses CD86 expressed in APCs, as well as in any other cell type, such as, e.g., in endothelial cells. CD86 is the ligand for two different proteins on the T-cell surface, CD28 and CTLA-4. Exemplary but non-limiting *CD86* gene includes human *CD86* gene having nucleic acid sequence as annotated under Genbank accession number EF064748.1. Exemplary but non-limiting *CD86* mRNA includes human *CD86* mRNA having nucleic acid sequence as annotated under Genbank accession number U04343.3. Exemplary but non-limiting CD86 protein includes human CD86 protein having amino acid sequence as annotated under Genbank accession number ABK41931.1.

CD80 and CD86 polypeptides are collectively termed "co-stimulatory molecules" and their presence is required (signal 2) alongside antigenic peptide (signal 1) to obtain efficient stimulation of antigen reactive T-cells. The presence of co-stimulatory molecules is sensed by receptors on the surface of the T-cell.

As used herein, the terms "cluster of differentiation 28" or "CD28" refer to one of the molecules expressed on T-cells that provide co-stimulatory signals, which are required for T-cell activation. CD28 is the receptor for CD80 and CD86. Exemplary but non-limiting *CD28* gene includes human *CD28* gene having nucleic acid sequence as annotated under Genbank accession number NG_029618.1. Exemplary but non-limiting *CD28* mRNA includes human *CD28* mRNA having nucleic acid sequence as annotated under Genbank accession number NM_006139.3, NM_001243077.1, or NM_001243078.1. Exemplary but non-limiting CD28 protein includes human CD28 protein (precursor) having amino acid sequence as annotated under Genbank accession number NP_006130.1, NP_001230006.1, or NP_001230007.1.

As used herein, the terms "cytotoxic T-lymphocyte antigen 4", "CTLA-4", "CTLA4", "cluster of differentiation 152" or "CD152", refers to a molecule expressed on the surface of T-cells that provide co-stimulatory signals, required for T-cell activation. CTLA4 is a receptor for CD80 and CD86. Exemplary but non-limiting *CTLA4* gene includes human *CTLA4* gene having nucleic acid sequence as annotated under Genbank accession number NG_011502.1. Exemplary but non-limiting *CTLA4* mRNA includes human *CTLA4* mRNA having nucleic acid sequence as annotated under Genbank accession number NM_001037631.2, or NM_005214.4. Exemplary but non-limiting CTLA4 protein includes human CTLA4 protein (precursor) having amino acid sequence as annotated under Genbank accession number NP_001032720.1, or NP_005205.2.

As used herein, the terms "cluster of differentiation 40" or "CD40" refer to CD40 co-stimulatory molecule expressed on activated antigen presenting cells. CD40 can bind CD154 (CD40L) on T cells. Exemplary but non-limiting *CD40* gene includes human *CD40* gene having nucleic acid sequence as annotated under Genbank accession number DQ871604.1. Exemplary but non-limiting *CD40* mRNA includes human *CD40* mRNA having nucleic acid sequence as annotated under Genbank accession number NM_001250.4, or NM_152854.2. Exemplary but non-limiting CD40 protein includes human CD40 protein (precursor) having amino acid sequence as annotated under Genbank accession number NP_001241.1, or NP_690593.1.

As used herein, the terms "CD40L", "CD40 ligand", "cluster of differentiation 154" or "CD154" refer to a protein that is primarily expressed on T cells that can bind CD40. Exemplary but non-limiting *CD40L* gene includes human *CD40L* gene having nucleic acid sequence as annotated under Genbank accession number NG_007280.1. Exemplary but non-limiting *CD40L* mRNA includes human *CD40L* mRNA having nucleic acid sequence as annotated under Genbank accession number NM_000074.2. Exemplary but non-limiting CD40L protein includes human CD40L protein having amino acid sequence as annotated under Genbank accession number NP_000065.1.

As used herein, the reference to any one nucleic acid or protein corresponds to the nucleic acid, protein, polypeptide or peptide commonly known under the respective designations in the art. The terms encompass such nucleic acids, proteins, polypeptides or peptides of any organism where found, and particularly of animals, preferably warm-blooded animals, more preferably vertebrates, yet more preferably mammals, including humans and non-human mammals, still more preferably of humans. The terms particularly encompass such nucleic acids, proteins, polypeptides or peptides with a native sequence, i.e., ones of which the primary sequence is the same as that of the nucleic acids, proteins, polypeptides or peptides found in or derived from nature. A skilled person understands that native sequences may differ between different species due to genetic divergence between such species. Moreover, native sequences may differ between or within different individuals of the same species due to normal genetic diversity (variation) within a given species. Also, native sequences may differ between or even within different individuals of the same species due to post-transcriptional or post-translational modifications. Any such variants or isoforms of nucleic acids, proteins, polypeptides or peptides are intended herein. Accordingly, all sequences of nucleic acids, proteins, polypeptides or peptides found in or derived from nature are considered "native". The terms encompass the nucleic acids, proteins, polypeptides or peptides when forming a part of a living organism, organ, tissue or cell, when forming a part of a biological sample, as well as when at least partly isolated from such sources. The terms also encompass the nucleic acids, proteins, polypeptides or peptides when produced by recombinant or synthetic means.

Unless otherwise apparent from the context, reference herein to any nucleic acid, protein, polypeptide or peptide may generally also encompass modified forms of said nucleic acid, protein, polypeptide or peptide such as bearing post-expression or chemical modifications including, for example, phosphorylation, glycosylation, lipidation, methylation, cysteinylation, sulphonation, glutathionylation, acetylation, oxidation of methionine to methionine sulphoxide or methionine sulphone, and the like.

A nucleic acid, protein, polypeptide or peptide may be preferably human, i.e., their primary sequence may be the same as a corresponding primary sequence of or present in a naturally occurring human nucleic acid, protein, polypeptide or peptide. Hence, the qualifier "human" in this connection relates to the primary sequence of the respective nucleic acid, protein, polypeptide or peptide, rather than to its origin or source. For example, such nucleic acid, protein, polypeptide or peptide may be present in or isolated from samples of human subjects or may be obtained by other means (e.g., by recombinant expression, cell-free translation or non-biological peptide synthesis).

The reference herein to any nucleic acid, protein, polypeptide or peptide may also encompass fragments thereof. The term "fragment" of a nucleic acid generally refers to 5'- and/or 3'-terminally deleted or truncated forms of said nucleic acid. The term "fragment" of a protein, polypeptide or peptide generally refers to N-terminally and/or C-terminally deleted or truncated forms of said protein, polypeptide or peptide. Without limitation, a fragment of a nucleic acid, protein, polypeptide or peptide may represent at least about 5%, or at least about 10%, e.g., ≥ 20%, ≥ 30% or ≥ 40%, such as preferably ≥ 50%, e.g., ≥ 60%, ≥ 70% or ≥ 80%, or more preferably ≥ 90% or ≥ 95% of the nucleotide sequence of said nucleic acid or of the amino acid sequence of said protein, polypeptide or peptide.

The reference herein to any nucleic acid, protein, polypeptide or peptide may also encompass variants thereof. The term "variant" of a nucleic acid, protein, polypeptide or peptide refers to nucleic acids, proteins, polypeptides or peptides the sequence (i.e., nucleotide sequence or amino acid sequence, respectively) of which is substantially identical (i.e., largely but not wholly identical) to the sequence of said recited nucleic acid, protein or polypeptide, e.g., at least about 80% identical or at least about 85% identical, e.g., preferably at least about 90% identical, e.g., at least 91% identical, 92% identical, more preferably at least about 93% identical, e.g., at least 94% identical, even more preferably at least about 95% identical, e.g., at least 96% identical, yet more preferably at least about 97% identical, e.g., at least 98% identical, and most preferably at least 99% identical. Preferably, a variant may display such degrees of identity to a recited nucleic acid, protein, polypeptide or peptide when the whole sequence of the recited nucleic acid, protein, polypeptide or peptide is queried in the sequence alignment (i.e., overall sequence identity). Also included among fragments and variants of a nucleic acid, protein, polypeptide or peptide are fusion products of said nucleic acid, protein, polypeptide or peptide with another, usually unrelated, nucleic acid, protein, polypeptide or peptide.

Sequence identity may be determined using suitable algorithms for performing sequence alignments and determination of sequence identity as known *per se.* Exemplary but non-limiting algorithms include those based on the Basic Local Alignment Search Tool (BLAST) originally described by Altschul et al. 1990 (J Mol Biol 215: 403-10), such as the "Blast 2 sequences" algorithm described by Tatusova and Madden 1999 (FEMS Microbiol Lett 174: 247-250), for example using the published default settings or other suitable settings (such as, e.g., for the BLASTN algorithm: cost to open a gap = 5, cost to extend a gap = 2, penalty for a mismatch = -2, reward for a match = 1, gap x_dropoff= 50, expectation value = 10.0, word size = 28; or for the BLASTP algorithm: matrix = Blosum62, cost to open a gap = 11, cost to extend a gap = 1, expectation value = 10.0, word size = 3).

A variant of a nucleic acid, protein, polypeptide or peptide may be a homologue (e.g., orthologue or paralogue) of said nucleic acid, protein, polypeptide or peptide. As used herein, the term "homology" generally denotes structural similarity between two macromolecules, particularly between two nucleic acids, proteins or polypeptides, from same or different taxons, wherein said similarity is due to shared ancestry.

Where the present specification refers to or encompasses fragments and/or variants of nucleic acids, proteins, polypeptides or peptides, this preferably denotes variants and/or fragments which are "functional", i.e., which at least partly retain the biological activity or intended functionality of the respective nucleic acids, proteins, polypeptides or peptides. By means of an example and not limitation, a functional fragment and/or variant of an antisense agent or RNAi agent shall at least partly retain the functionality of said agent, i.e., its ability to reduce or abolish the expression of a target molecule (gene). By means of another example and not limitation, a functional fragment and/or variant of a CD80 or CD86 polypeptide shall at least partly retain the biological activity of CD80 or CD86, respectively, such as, e.g., ability to bind to one or more cognate receptors, more particularly CD28 and/or CTLA-4, or to participate in one or more cellular pathways, more particularly in the genesis of the co-stimulatory signal necessary for the activation of T-cells, *etc.* Preferably, where the biological activity can be expressed as a determinable or measurable variable, for example by using a suitable quantitative test to determine or measure the biological activity, then a functional fragment and/or variant may retain at least about 20%, e.g., at least 30%, or at least about 40%, or at least about 50%, e.g., at least 60%, more preferably at least about 70%, e.g., at least 80%, yet more preferably at least about 85%, still more preferably at least about 90%, and most preferably at least about 95% or even about 100% or higher of the intended biological activity or functionality compared to the corresponding nucleic acid, protein, polypeptide or peptide.

The term "expression" of a polypeptide by a cell generally refers to the production of said polypeptide by the cell. As commonly known, the expression of polypeptides by cells involves several successive molecular mechanisms, more particularly but without limitation, the transcription of a gene encoding said polypeptide into RNA, the polyadenylation and where applicable splicing and/or other post-transcriptional modifications of the RNA into mRNA, the localisation of the mRNA into cell cytoplasm, where applicable other post-transcriptional modifications of the mRNA, the translation of the mRNA into a polypeptide chain, where applicable post-translational modifications of the polypeptide, and folding of the polypeptide chain into the mature conformation of the polypeptide. For compartmentalised polypeptides, such as secreted polypeptides and transmembrane polypeptides, the production process further involves trafficking of the polypeptides, i.e., the cellular mechanism by which polypeptides are transported to the appropriate sub-cellular compartment or organelle, membrane, e.g. the plasma membrane, or outside the cell.

The term "prevent or inhibit the expression of a polypeptide" thus denotes achieving a qualitative or quantitative reduction of the production of the polypeptide by a cell. Such prevention or inhibition may impinge on any one or more stages of the expression process, so long as it results in reduced amount of the mature, biologically active polypeptide produced by the cell. The term encompasses any extent of such prevention or inhibition. For example, where the reduction in the amount of the polypeptide expressed by a cell can be expressed as a determinable or measurable variable, for example by using a suitable quantitative test to determine or measure the amount polypeptide, the term may encompass a reduction in the amount of the polypeptide expressed by a cell by at least about 10%, e.g., by at least about 20%, by at least about 30%, e.g., by at least about 40%, by at least about 50%, e.g., by at least about 60%, by at least about 70%, e.g., by at least about 80%, by at least about 90%, e.g., by at least about 95%, such as by at least about 96%, 97%, 98%, 99% or even of 100%, compared to a reference situation without said prevention or inhibition. Many suitable tests to measure polypeptide amounts expressed by cells are available, for example based on quantitative biochemical assay methods, immunoassay methods, mass spectrometry methods, or chromatography methods, or combinations thereof.

Preferably, the prevention or inhibition of the expression of a polypeptide, such as the prevention or inhibition of the expression of the at least one of the CD80 or CD86 polypeptides, or the prevention or inhibition of the expression of the CD40 polypeptide, will be specific or selective, i.e., the agent effecting the same will not substantially prevent or inhibit the expression of random, unrelated polypeptides.

Polypeptides such as receptors or ligands, including *inter alia* CD80, CD86, CD28, CD40 and CD40L, are typically presented on the surface (i.e., on the plasma membrane) of cells expressing them in order to perform their biological functions (binding to cognate receptors or ligands to effect signalling activities). Accordingly, the term "prevent or inhibit the presentation of a polypeptide on the surface of a cell" denotes achieving a qualitative or quantitative reduction of the presentation of the mature, biologically active polypeptide on the surface of the cell. Such prevention or inhibition may impinge on any one or more stages of the trafficking or translocation process, as long as it results in reduced amount of the mature, biologically active polypeptide presented on the surface of the cell. The term encompasses any extent of such prevention or inhibition.

For example, where the prevention or inhibition in the amount of the polypeptide presented on the surface of the cell can be expressed as a determinable or measurable variable, for example by using a suitable quantitative test to determine or measure the amount of the polypeptide on the cell surface, the term may encompass a reduction in the amount of the polypeptide presented on the surface of a cell by at least about 10%, e.g., by at least about 20%, by at least about 30%, e.g., by at least about 40%, by at least about 50%, e.g., by at least about 60%, by at least about 70%, e.g., by at least about 80%, by at least about 90%, e.g., by at least about 95%, such as by at least about 96%, 97%, 98%, 99% or even of 100%, compared to a reference situation without said prevention or inhibition. Many suitable tests to measure polypeptide amounts expressed on the surface of cells are available, for example based on quantitative biochemical assay methods, immunoassay methods, quantitative fluorescence activated cell sorting methods, or combinations thereof.

Preferably, the prevention or inhibition of the presentation of a polypeptide on the surface of a cell, such as the prevention or inhibition of the presentation of the at least one of the CD80 or CD86 polypeptides on the surface of a cell, or the prevention or inhibition of the presentation of the CD40 polypeptide on the surface of a cell, will be specific or selective, i.e., the agent effecting the same will not substantially prevent or inhibit the presentation of random, unrelated polypeptides on the surface of the cell.

In certain embodiments, the therapeutic agent capable of interacting with the at least one of the CD80 or CD86 polypeptides may be a therapeutic agent capable of interacting with the CD80 polypeptide, wherein said interaction interferes with the binding of the CD80 polypeptide to the CD28 polypeptide. In certain embodiments, the therapeutic agent capable of interacting with the at least one of the CD80 or CD86 polypeptides may be a therapeutic agent capable of interacting with the CD80 polypeptide but not with the CD86 polypeptide, wherein said interaction interferes with the binding of the CD80 polypeptide to the CD28 polypeptide.

In certain embodiments, the therapeutic agent capable of interacting with the at least one of the CD80 or CD86 polypeptides may be a therapeutic agent capable of interacting with the CD86 polypeptide, wherein said interaction interferes with the binding of the CD86 polypeptide to the CD28 polypeptide.

In certain embodiments, the therapeutic agent capable of interacting with the at least one of the CD80 or CD86 polypeptides may be a therapeutic agent capable of interacting with the CD86 polypeptide but not with the CD80 polypeptide, wherein said interaction interferes with the binding of the CD86 polypeptide to the CD28 polypeptide.

In certain embodiments, the therapeutic agent capable of interacting with the at least one of the CD80 or CD86 polypeptides may be a therapeutic agent capable of interacting with the CD80 polypeptide and with the CD86 polypeptide, wherein said interaction interferes with the binding of the CD80 polypeptide and the CD86 polypeptide to the CD28 polypeptide.

In certain embodiments, the therapeutic agent is capable of interacting with at least one of the CD80 or CD86 polypeptides, wherein said interaction interferes with the binding of the at least one of the CD80 or CD86 polypeptides to the CD28 polypeptide and to the CTLA-4 polypeptide.

In certain embodiments, the therapeutic agent is capable of interacting with at least one of the CD80 or CD86 polypeptides, wherein said interaction interferes with the binding of the at least one of the CD80 or CD86 polypeptides to the CD28 polypeptide but not to the CTLA-4 polypeptide.

In certain embodiments, the therapeutic agent capable of preventing or inhibiting the expression of at least one of the CD80 or CD86 polypeptides by a cell or capable of preventing or inhibiting the presentation of at least one of the CD80 or CD86 polypeptides on the surface of a cell may be a therapeutic agent capable of preventing or inhibiting the expression of the CD80 polypeptide by the cell or capable of preventing or inhibiting the presentation of the CD80 polypeptide on the surface of the cell.

In certain embodiments, the therapeutic agent capable of preventing or inhibiting the expression of at least one of the CD80 or CD86 polypeptides by a cell or capable of preventing or inhibiting the presentation of at least one of the CD80 or CD86 polypeptides on the surface of a cell may be a therapeutic agent capable of preventing or inhibiting the expression of the CD80 polypeptide but not the CD86 polypeptide by the cell or capable of preventing or inhibiting the presentation of the CD80 polypeptide but not the CD86 polypeptide on the surface of the cell.

In certain embodiments, the therapeutic agent capable of preventing or inhibiting the expression of at least one of the CD80 or CD86 polypeptides by a cell or capable of preventing or inhibiting the presentation of at least one of the CD80 or CD86 polypeptides on the surface of a cell may be a therapeutic agent capable of preventing or inhibiting the expression of the CD86 polypeptide by the cell or capable of preventing or inhibiting the presentation of the CD86 polypeptide on the surface of the cell.

In certain embodiments, the therapeutic agent capable of preventing or inhibiting the expression of at least one of the CD80 or CD86 polypeptides by a cell or capable of preventing or inhibiting the presentation of at least one of the CD80 or CD86 polypeptides on the surface of a cell may be a therapeutic agent capable of preventing or inhibiting the expression of the CD86 polypeptide but not the CD80 polypeptide by the cell or capable of preventing or inhibiting the presentation of the CD86 polypeptide but not the CD80 polypeptide on the surface of the cell.

In certain embodiments, the therapeutic agent capable of preventing or inhibiting the expression of at least one of the CD80 or CD86 polypeptides by a cell or capable of preventing or inhibiting the presentation of at least one of the CD80 or CD86 polypeptides on the surface of a cell may be a therapeutic agent capable of preventing or inhibiting the expression of the CD80 polypeptide and the CD86 polypeptide by the cell or capable of preventing or inhibiting the presentation of the CD80 polypeptide and the CD86 polypeptide on the surface of the cell.

In further embodiments, the at least one of the CD80 or CD86 polypeptides may be comprised by a cell and the CD28 polypeptide may be comprised by another cell.

In particular, the at least one of the CD80 or CD86 polypeptides may be comprised by an antigen presenting cell (APC) and the CD28 polypeptide may be comprised by a T-cell, whereby the action of the therapeutic agent as disclosed herein capable of interacting with the at least one of the CD80 or CD86 polypeptides will interfere with the co-stimulatory pathway B7:CD28, resulting in inhibition of the T cell responses. Accordingly, such embodiments provide for a therapeutic agent for use in the treatment of cardiac warm ischemia reperfusion injury, wherein the therapeutic agent is capable of interacting with at least one of the CD80 or CD86 polypeptides comprised by an antigen presenting cell, and wherein said interaction interferes with the binding of the at least one of the CD80 or CD86 polypeptides comprised by the antigen presenting cell to the CD28 polypeptide comprised by a T-cell.

In embodiments, the at least one of the CD80 or CD86 polypeptides may be comprised by an endothelial cell and the CD28 polypeptide may be comprised by a T-cell, whereby the action of the therapeutic agent as disclosed herein capable of interacting with the at least one of the CD80 or CD86 polypeptides will interfere with the adherence of the T-cell to the endothelium. Accordingly, such embodiments provide for a therapeutic agent for use in the treatment of cardiac warm ischemia reperfusion injury, wherein the therapeutic agent is capable of interacting with at least one of the CD80 or CD86 polypeptides comprised by an endothelial cell, and wherein said interaction interferes with the binding of the CD28 polypeptide comprised by a T-cell to the at least one of the CD80 or CD86 polypeptides comprised by the endothelial cell.

In further embodiments, the at least one of the CD80 or CD86 polypeptides may be comprised by an antigen presenting cell (APC), whereby the action of the therapeutic agent as disclosed herein capable of preventing or inhibiting the expression of the at least one of the CD80 or CD86 polypeptides by the cell or capable of preventing or inhibiting the presentation of at least one of the CD80 or CD86 polypeptides on the surface of the cell will interfere with the co-stimulatory pathway B7:CD28, resulting in inhibition of the T cell responses. Accordingly, such embodiments provide for a therapeutic agent for use in the treatment of cardiac warm ischemia reperfusion injury, wherein the therapeutic agent is capable of preventing or inhibiting the expression of at least one of the CD80 or CD86 polypeptides by an antigen presenting cell or capable of preventing or inhibiting the presentation of at least one of the CD80 or CD86 polypeptides on the surface of an antigen presenting cell.

In embodiments, the at least one of the CD80 or CD86 polypeptides may be comprised by an endothelial cell, whereby the action of the therapeutic agent as disclosed herein capable of preventing or inhibiting the expression of the at least one of the CD80 or CD86 polypeptides by the cell or capable of preventing or inhibiting the presentation of at least one of the CD80 or CD86 polypeptides on the surface of the cell will interfere with the adherence of the T-cell to the endothelium. Accordingly, such embodiments provide for a therapeutic agent for use in the treatment of cardiac warm ischemia reperfusion injury, wherein the therapeutic agent is capable of preventing or inhibiting the expression of at least one of the CD80 or CD86 polypeptides by an endothelial cell or capable of preventing or inhibiting the presentation of at least one of the CD80 or CD86 polypeptides on the surface of an endothelial cell.

In certain embodiments, the therapeutic agent as intended herein may be selected from the group comprising, consisting essentially of, or consisting of a protein, a polypeptide, a peptide, a peptidomimetic, a nucleic acid, a small organic molecule, and a compound or combination of any two or more thereof.

The term "protein" as used herein generally encompasses macromolecules comprising one or more polypeptide chains, i.e., polymeric chains of amino acid residues linked by peptide bonds. The term may encompass naturally, recombinantly, semi-synthetically or synthetically produced proteins. The term also encompasses proteins that carry one or more co- or post-expression-type modifications of the polypeptide chain(s), such as, without limitation, glycosylation, acetylation, phosphorylation, sulfonation, methylation, ubiquitination, signal peptide removal, N-terminal Met removal, conversion of pro-enzymes or pre-hormones into active forms, etc. The term further also includes protein variants or mutants which carry amino acid sequence variations vis-à-vis a corresponding native proteins, such as, e.g., amino acid deletions, additions and/or substitutions. The term contemplates both full-length proteins and protein parts or fragments, e.g., naturally-occurring protein parts that ensue from processing of such full-length proteins.

The term "polypeptide" as used herein generally encompasses polymeric chains of amino acid residues linked by peptide bonds. Hence, insofar a protein is only composed of a single polypeptide chain, the terms "protein" and "polypeptide" may be used interchangeably herein to denote such a protein. The term is not limited to any minimum length of the polypeptide chain. The term may encompass naturally, recombinantly, semi-synthetically or synthetically produced polypeptides. The term also encompasses polypeptides that carry one or more co- or post-expression-type modifications of the polypeptide chain, such as, without limitation, glycosylation, acetylation, phosphorylation, sulfonation, methylation, ubiquitination, signal peptide removal, N-terminal Met removal, conversion of pro-enzymes or pre-hormones into active forms, etc. The term further also includes polypeptide variants or mutants which carry amino acid sequence variations vis-à-vis a corresponding native polypeptide, such as, e.g., amino acid deletions, additions and/or substitutions. The term contemplates both full-length polypeptides and polypeptide parts or fragments, e.g., naturally-occurring polypeptide parts that ensue from processing of such full-length polypeptides.

The term "peptide" as used herein preferably refers to a polypeptide as used herein consisting essentially of 50 amino acids or less, e.g., 45 amino acids or less, preferably 40 amino acids or less, e.g., 35 amino acids or less, more preferably 30 amino acids or less, e.g., 25 or less, 20 or less, 15 or less, 10 or less or 5 or less amino acids.

The term "peptidomimetic" refers to a non-peptide agent that is a topological analogue of a corresponding peptide. Methods of rationally designing peptidomimetics of peptides are known in the art. For example, by means of a guidance, the rational design of three peptidomimetics based on the sulphated 8-mer peptide CCK26-33, and of two peptidomimetics based on the 11-mer peptide Substance P, and related peptidomimetic design principles, are described in Horwell 1995 (Trends Biotechnol 13: 132-134).

The term "nucleic acid" as used herein typically refers to a polymer (preferably a linear polymer) of any length composed essentially of nucleoside units. A nucleoside unit commonly includes a heterocyclic base and a sugar group. Heterocyclic bases may include *inter alia* purine and pyrimidine bases such as adenine (A), guanine (G), cytosine (C), thymine (T) and uracil (U) which are widespread in naturally-occurring nucleic acids, other naturally-occurring bases (e.g., xanthine, inosine, hypoxanthine) as well as chemically or biochemically modified (e.g., methylated), non-natural or derivatised bases. Exemplary modified nucleobases include without limitation 5-substituted pyrimidines, 6-azapyrimidines and N-2, N-6 and O-6 substituted purines, including 2-aminopropyladenine, 5- propynyluracil and 5-propynylcytosine. In particular, 5-methylcytosine substitutions have been shown to increase nucleic acid duplex stability and may be preferred base substitutions in for example antisense agents, even more particularly when combined with 2'-O-methoxyethyl sugar modifications. Sugar groups may include *inter alia* pentose (pentofuranose) groups such as preferably ribose and/or 2-deoxyribose common in naturally-occurring nucleic acids, or arabinose, 2-deoxyarabinose, threose or hexose sugar groups, as well as modified or substituted sugar groups (such as without limitation 2'-O-alkylated, e.g., 2'-O-methylated or 2'-O-ethylated sugars such as ribose; 2'-O-alkyloxyalkylated, e.g., 2'-O-methoxyethylated sugars such as ribose; or 2'-O,4'-C-alkylene-linked, e.g., 2'-O,4'-C-methylene-linked or 2'-O,4'-C-ethylene-linked sugars such as ribose; 2'-fluoro-arabinose, *etc.).* Nucleoside units may be linked to one another by any one of numerous known inter-nucleoside linkages, including *inter alia* phosphodiester linkages common in naturally-occurring nucleic acids, and further modified phosphate- or phosphonate-based linkages such as phosphorothioate, alkyl phosphorothioate such as methyl phosphorothioate, phosphorodithioate, alkylphosphonate such as methylphosphonate, alkylphosphonothioate, phosphotriester such as alkylphosphotriester, phosphoramidate, phosphoropiperazidate, phosphoromorpholidate, bridged phosphoramidate, bridged methylene phosphonate, bridged phosphorothioate; and further siloxane, carbonate, sulfamate, carboalkoxy, acetamidate, carbamate such as 3'-N-carbamate, morpholino, borano, thioether, 3'-thioacetal, and sulfone inter-nucleoside linkages. Preferably, inter-nucleoside linkages may be phosphate-based linkages including modified phosphate-based linkages, such as more preferably phosphodiester, phosphorothioate or phosphorodithioate linkages or combinations thereof. The term "nucleic acid" also encompasses any other nucleobase containing polymers such as nucleic acid mimetics, including, without limitation, peptide nucleic acids (PNA), peptide nucleic acids with phosphate groups (PHONA), locked nucleic acids (LNA), morpholino phosphorodiamidate-backbone nucleic acids (PMO), cyclohexene nucleic acids (CeNA), tricyclo-DNA (tcDNA), and nucleic acids having backbone sections with alkyl linkers or amino linkers (see, e.g., Kurreck 2003 (Eur J Biochem 270: 1628-1644)). "Alkyl" as used herein particularly encompasses lower hydrocarbon moieties, e.g., C1-C4 linear or branched, saturated or unsaturated hydrocarbon, such as methyl, ethyl, ethenyl, propyl, 1-propenyl, 2-propenyl, and isopropyl. Nucleic acids as intended herein may include naturally occurring nucleosides, modified nucleosides or mixtures thereof. A modified nucleoside may include a modified heterocyclic base, a modified sugar moiety, a modified inter-nucleoside linkage or a combination thereof. The term "nucleic acid" further preferably encompasses DNA, RNA and DNA/RNA hybrid molecules, specifically including hnRNA, pre-mRNA, mRNA, cDNA, genomic DNA, amplification products, oligonucleotides, and synthetic (e.g., chemically synthesised) DNA, RNA or DNA/RNA hybrids. A nucleic acid can be naturally occurring, e.g., present in or isolated from nature, can be recombinant, i.e., produced by recombinant DNA technology, and/or can be, partly or entirely, chemically or biochemically synthesised. A "nucleic acid" can be double-stranded, partly double stranded, or single-stranded. Where single-stranded, the nucleic acid can be the sense strand or the antisense strand. In addition, nucleic acid can be circular or linear. Nucleic acids and particularly antisense oligonucleotides or RNAi agents, including small interfering RNA, may be herein denoted as comprising uracil (U) bases. It shall be appreciated that U may be optionally substituted by thymine (T) in (at least some) such nucleic acids and agents. For example, as 2'-O-methyl phosphorothioate antisense oligonucleotides are more 'RNA-like', U may be used and denoted in such molecules. With other antisense chemistries, such as peptide nucleic acids or morpholino backbones, T bases may be preferably denoted and used.

The term "small organic molecule" encompasses organic compounds with a size comparable to those organic molecules generally used in pharmaceuticals. The term excludes biological macromolecules (e.g., proteins, nucleic acids, *etc.).* Preferred small organic molecules range in size up to about 5000 Da, e.g., up to about 4000, preferably up to 3000 Da, more preferably up to 2000 Da, even more preferably up to about 1000 Da, e.g., up to about 900, 800, 700, 600 or up to about 500 Da.

In certain embodiment, a small organic molecule suitable as a therapeutic agent herein may include heterocyclic compounds as described in WO2005116033, WO2005046679, WO2003004495, WO2004048378 and WO 2004281011, such as RhuDex® (Medigene), which binds to CD80.

In embodiments, the small molecule may be a compound of formula (I) or (II) or a pharmaceutically acceptable salt, hydrate or solvate of any of them: 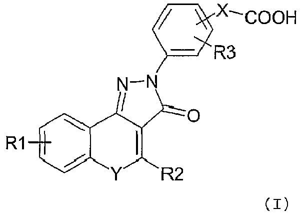 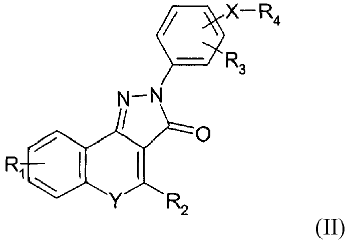 wherein
X represents a bond or a divalent radical of formula -(Z)ₙ-(Alk)- or -(Alk)-(Q)ₙ-; -NH-C(O)-Alk-, -NH-C(O)-Alk-O-Alk- or C(O)-NH; wherein Q represents -O-, -S- or -NH-, and n is 0 or 1; wherein Alk is an optionally substituted divalent straight or branched C₁-C₁₂ alkylene, or C₂-C₁₂ alkenylene, or C₂-C₁₂ alkynylene radical or a divalent C₃-C₁₂ carbocyclic radical, preferably Alk is an C₁-C₆alkylene; wherein any of which radicals may contain one or more -O-, -S- or -N(R₈)- links wherein R₈ represents H or C₁-C₄ alkyl, C₃-C₄ alkenyl, C₃-C₄ alkynyl, or C₃-C₆ cycloalkyl;
Y represents -O-, -S-, or -N(R₅)- in formula (I), Y represents -O-, -S-, N-oxide, or -N(R₅)- in formula (II) wherein R₅ represents H or C₁-C₆ alkyl;
R₁ represents H; F; Cl ; Br ; -NO₂; -CN ; C₁-C₆ alkyl optionally substituted by F or Cl; or C₁- C₆ alkoxy optionally substituted by F in formulas (I) and (II);
R₂ represents H, or optionally substituted C₁-C₆ alkyl, C₃-C₇ cycloalkyl or optionally substituted phenyl in formulas (I) and (II);
R₃ represents H; F; Cl ; Br; -NO₂; -CN ; C₁-C₆ alkyl optionally substituted by F or Cl; or C₁- C₆ alkoxy optionally substituted by F in formulas (I) and (II)
R₄ represents -C(=O)NR₆R₇, -NR₇C(=O)R₆, -NR₇C(=O)OR₆, -NHC(=O)NHR₆ or -NHC(=S)NHR₆ in formula (II) wherein
R₆ represents H, or a radical of formula -(Alk)_{b}-Q wherein
b is 0 or 1 and
Alk is an optionally substituted divalent straight chain or branched C₁-C₁₂ alkylene, C₂-C₁₂ alkenylene or C₂- C₁₂ alkynylene radical which may be interrupted by one or more non-adjacent -O-, -S- or -N(R₈)-radicals wherein R₈ represents H or C₁-C₄ alkyl, C₃-C₄ alkenyl, C₃-C₄ alkynyl, or C₃-C₆ cycloalkyl, and
Q represents H; -CF₃; -OH; -SH ; -NR₈R₈ wherein each R₈ may be the same or different; an ester group; or an optionally substituted phenyl, C₃-C₇ cycloalkyl, C₅-C₇ cycloalkenyl or heterocyclic ring having from 5 to 8 ring atoms; and
R₇ represents H or C₁-C₆ alkyl; or when taken together with the atom or atoms to which they are attached R₆ and R₇ form an optionally substituted heterocyclic ring having from 5 to 8 ring atoms.

In embodiments, the small molecule may be a compound of formula (III), or a pharmaceutically acceptable salt, hydrate or solvate thereof: 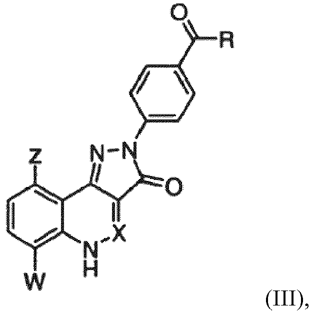 where R is OH, W is F, Z is H and X is CH.

Further examples of small molecules suitable for use herein include:
{[3-((3-Oxo-3, 5-dihydro-pyrazolo [4, 3-c] quinolin-2-yl)- phenylcarbamoyl]-methoxy}-acetic acid,
N- [4- (3-Oxo-3, 5-dihydro-pyrazolo [4, 3-c] quinolin-2-yl)- phenyl]-succinamic acid,
4- [4- (3-Oxo-3, 5-dihydro-pyrazolo [4, 3-c] quinolin-2-yl)- phenylcarbamoyl]-butyric acid,
{[4-((3-Oxo-3, 5-dihydro-pyrazolo [4, 3-c] quinolin-2-yl)- phenylcarbamoyl]-methoxy}-acetic acid,
4- [4- (3-Oxo-3, 5-dihydro-pyrazolo [4, 3-c] quinolin-2-yl)- phenylcarbamoyl]-2-phenyl-butyric acid,
N- [3- (3-Oxo-3, 5-dihydro-pyrazolo [4, 3-c] quinolin-2-yl)- phenyl]-succinamic acid,
2-{[4-(3-Oxo-3,5-dihydro-pyrazolo [4, 3-c] quinolin-2-yl)- phenylcarbamoyl]-methyl}-benzoic acid,
2-Chloro-4-(3-oxo-3, 5-dihydro-pyrazolo [4, 3-c] quinolin-2- yl)-benzoic acid,
4-((6, 8-Dimethyl-3-oxo-3, 5-dihydro-pyrazolo [4,3c] quinolin-2-yl)-benzoic acid,
4- ((8-Methoxy-6-methyl-3-oxo-3, 5-dihydro-pyrazolo [4,3c] quinolin-2-yl)-benzoic acid,
4- ((6, 8-Dimethoxy-3-oxo-3,5-dihydro-pyrazolo [4,3c] quinolin-2-yl)-benzoic acid,
4- ((7, 9-Dimethoxy-3-oxo-3,5-dihydro-pyrazolo [4,3c] quinolin-2-yl)-benzoic acid,
4- ((6-Methyl-3-oxo-8-trifluoromethyl-3, 5-dihydropyrazolo [4, 3-c] quinolin-2-yl)-benzoic acid,
4- ((7, 9-Dichloro-3-oxo-3, 5-dihydro-pyrazolo [4,3c]quinolin-2-yl)-benzoic acid,
[4- ((3-Oxo-3, 5-dihydro-pyrazolo [4, 3-c] quinolin-2-yl)- phenyl]-propionic acid,
[4- ((3-Oxo-3, 5-dihydro-pyrazolo [4, 3-c] quinolin-2-yl)- phenyl]-acetic acid,
4- ((4-Methyl-3-oxo-3H-chromeno [4, 3-c] pyrazol-2-yl)-benzoic acid,
4- ((3-Oxo-3H-thiochromeno [4, 3-c] pyrazol-2-yl) benzoic acid,
4- ((5-Methyl-3-oxo-3, 5-dihydro-pyrazolo [4, 3-c] quinolin-2- yl)-benzoic acid,
2- [4- (3-Oxo-3, 5-dihydro-pyrazolo [4,3-c] quinolin-2-yl) benzoylamino]-3-phenyl-propionic acid, and
2- [4- (3-Oxo-3, 5-dihydro-pyrazolo [4,3-c] quinolin-2-yl) benzoylamino]-2-acetic acid.

In embodiments, the small molecule may be a compound of formula (IV), (V), (VI), or (VII) or a pharmaceutically acceptable salt, hydrate or solvate of any of them: 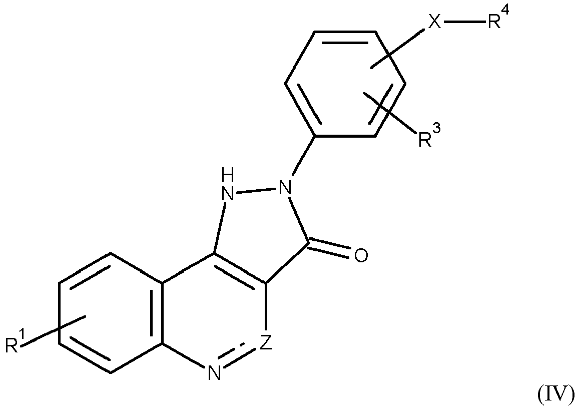 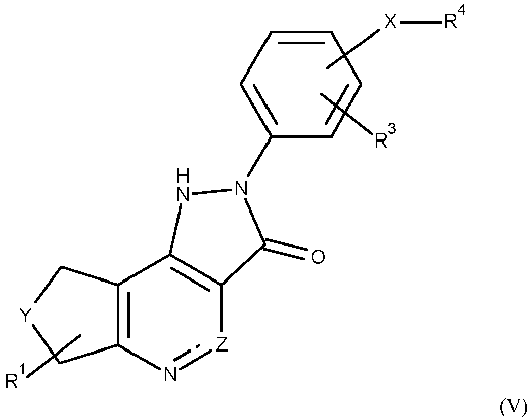 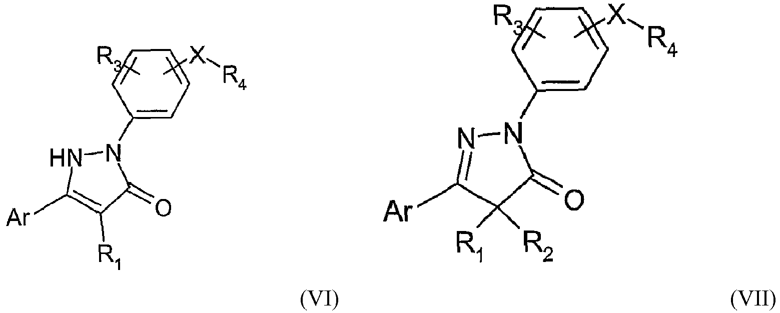 wherein R₁ represents H; F; Cl; Br; -NO₂; CN; C₁-C₆ alkyl optionally substituted by F or Cl; or C₁-C₆ alkoxy optionally substituted by F in formula (IV), R₁ represents H; F; methyl, trifuoromethyl, methoxy or triflioromethoxy in formula (V), R₁ represents H, or C₁-C₆ alkyl in formulas (VI) and (VII);
R₂ represents H or C₁-C₆alkyl in formula (VII);
R₃ represents H; F; Cl; Br; -NO₂; CN; C₁-C₆ alkyl optionally substituted by F or Cl; or C₁-C₆ alkoxy optionally substituted by F in formulas (IV), (V), (VI), and (VII);
R₄ represents a carboxylic acid group (-COOH) or an ester thereof, or -C(=O)NR₆R₇, -NR₇C(=O)R₆, - NR₇C(=O)OR₆, -NHC(=O)NR₇R₆ or -NHC (=S) NR₇R₆ in formulas (IV), (V), (VI), and (VII) wherein
R₆ represents H, or a radical of formula -(Alk)ₘ-Q wherein m is 0 or 1
Alk is an optionally substituted divalent straight or branched C₁-C₁₂ alkylene, or C₂-C₁₂ alkenylene, or C₂-C₁₂ alkynylene radical or a divalent C₃-C₁₂ carbocyclic radical, any of which radicals may contain one or more -O-, -S- or -N(R₈)- links wherein R8 represents H or C₁-C₄ alkyl, C₃-C₄ alkenyl, C₃-C₄ alkynyl, or C₃-C₆ cycloalkyl, and
Q represents H; -NR₉R₁₀ wherein R₉ and R₁₀ independently represents H ; C₁-C₄ alkyl; C₃-C₄ alkenyl; C₃-C₄ alkynyl; C₃-C₆ cycloalkyl; an ester group; an optionally substituted carbocyclic or heterocyclic group; or R₉ and R₁₀ form a ring when taken together with the nitrogen to which they are attached, which ring is optionally substituted; and
R7 represents H or C₁-C₆ alkyl; or when taken together with the atom or atoms to which they are attached R₆ and R₇ form an optionally substituted monocyclic heterocyclic ring having 5,6 or 7 ring atoms;
X represents a bond or a divalent radical of formula -(Z)ₙ-(Alk)- or -(Alk)-(Q)ₙ- in formulas (IV), (V), (VI), and (VII), wherein Q represents -O-, -S- or -NH-, Alk is as defined in relation to R₆ and n is 0 or 1;
Y is -CH₂-, -CH₂CH₂-, or -CH₂CH₂CH₂- in formula (V);
Z represents represents N or CR_{z} in formulas (IV) and (V) wherein R_{z} is H, or optionally substituted C₁-C₆ alkyl, C₃-C₇ cycloalkyl or optionally substituted phenyl;and
Ar represents an optionally substituted monocyclic or bicyclic aromatic or heteroaromatic group having from 5 to 10 ring atoms in formulas (VI) and (VII).

The small molecule may also be any tautomeric form of any one of structures (I), (II), (III), (IV), (V), (VI), and (VII).

Unless otherwise specified in the context in which it occurs, the term "substituted" as applied to any moiety of formulas (I) and (II) means substituted with one or more of the following substituents, namely (C₁-C₆)alkyl, trifluoromethyl, (C₁-C₆)alkoxy (including the special case where a ring is substituted on adjacent ring C atoms by methylenedioxy or ethylenedioxy), trifluoromethoxy, (C₁-C₆)alkylthio, phenyl, benzyl, phenoxy, (C₃-C₈)cycloalkyl, -OH, -SH, -NH₂, -F, -Cl, -Br, -CN, -NO₂, oxo (=O), -COOH, -SO₂OH, -CONH₂, -SO₂NH₂, -COR^{A}, -COOR^{A}, -SO₂OR^{A}, -NHCOR^{A}, -NHSO₂R^{A}, -CONHR^{A}, -SO₂NHR^{A}, -NHR^{A}, -NR^{A}R^{B}, -CONR^{A}R^{B} or -SO₂NR^{A}RB wherein R^{A} and R^{B} are independently a (C₁-C₆)alkyl group.

Unless otherwise specified in the context in which it occurs, the term "substituted" as applied to any moiety of formulas (IV) and (V) means substituted with at least one substituent, selected from, for example, (C₁-C₆)alkyl, (C₁-C₆)alkenyl, (C₂-C₆)alkynyl, fluoro-substituted (C₁-C₆)alkyl, fluoro-substituted (C₁-C₆)alkenyl, fluoro-substituted (C₂-C₆)alkynyl, (C₁-C₆)alkoxy and fluoro-substituted (C₁-C₆)alkoxy (including the special case where a ring is substituted on adjacent ring C atoms by alkylenedioxy such as methylenedioxy or ethylenedioxy), (C₁-C₆)alkylthio, phenyl, benzyl, phenoxy, benzyloxy, -OH, -SH, -NH₂, -F, -Cl, -Br, -CN, -NO₂, oxo (=O), -COOH, -SO₂OH, -CONH₂, -SO₂NH₂, -COR^{A}, -COOR^{A}, -SO₂OR^{A}, -NHCOR^{A}, -NHSO₂R^{A}, -CONHR^{A}, -SO₂NHR^{A}, -NHR^{A}, -NR^{A}R^{B}, - CONR^{A}R^{B} or -SO₂NR^{A}R^{B} wherein R^{A} and R^{B} are independently a (C₁-C₆)alkyl or (C₂-C₆)alkoxy group or a monocyclic carbocyclic or heterocyclic group of from 5-7 ring members, or R^{A} and R^{B} form a ring when taken together with the nitrogen to which they are attached.

Unless otherwise specified in the context in which it occurs, the term "substituted" as applied to any moiety of structures (VI) and (VII) means substituted with at least one substituent, for example selected from (C₁-C₆)alkyl, trifluoromethyl, (C₁-C₆)alkoxy (including the special case where a ring is substituted on adjacent ring C atoms by alkylenedioxy such as methylenedioxy or ethylenedioxy), trifluoromethoxy, (C₁-C₆)alkylthio, phenyl, benzyl, phenoxy, benzyloxy, -OH, -SH, -NH₂, -F, -Cl, -Br, -CN, -NO₂, oxo (=O), -COOH, -SO₂OH, -CONH₂, -SO₂NH₂, -COR^{A}, -COOR^{A}, -SO₂OR^{A}, -NHCOR^{A}, -NHSO₂R^{A}, -CONHR^{A} -SO₂NHR^{A}, -NHR^{A}, -NR^{A}R^{B}, -CONR^{A}R^{B} or -SO₂NR^{A}R^{B} wherein R^{A} and R^{B} are independently a (C₁-C₆)alkyl or C₂-C₆ alkoxy group or a monocyclic carbocyclic or heterocyclic group of from 5-7 ring members, or R^{A} and R^{B} form a ring when taken together with the nitrogen to which they are attached.

In the case where "substituted" means substituted by (C₃-C₈) cycloalkyl, phenyl, benzyl, phenoxy, or benzyloxy, the phenyl ring thereof may itself be substituted with any of the foregoing, except (C₃-C₈) cycloalkyl, phenyl, benzyl, phenoxy, or benzyloxy.

As used herein the term "alkylene" refers to a straight or branched alkyl chain having two unsatisfied valencies, for example -CH₂-, -CH₂CH₂-, -CH₂CH₂CH₂-, -CH(CH₃)CH₂-, -CH(CH₂CH₃)CH₂CH₂CH₃, and -C(CH₃)₃.

As used herein the term "alkenylene" refers to a straight or branched alkenyl chain having two unsatisfied valencies, for example -CH=CH-, -CH₂CH=CH-, -C(CH₃)=CH-, and - CH(CH₂CH₃)CH=CHCH₂-.

As used herein the term "alkynylene" refers to a straight or branched alkynyl chain having two unsatisfied valencies, for example -C≡C-,-CH₂C≡C-, and -CH(CH₂CH₃)C≡CCH₂-.

As used herein the unqualified term "carbocyclyl" or "carbocyclic" includes aryl, cycloalkyl and cycloalkenyl and refers to a ring system (monocyclic, bicyclic, tricyclic or bridged) whose ring atoms are all carbon.

As used herein the unqualified term "cycloalkyl" refers to a carbocyclic ring system which contains only single bonds between ring carbons.

As used herein the unqualified term "heterocyclyl" or "heterocyclic" includes "heteroaryl", and in particular means a 5-8 membered aromatic or non-aromatic heterocyclic ring containing one or more hetero-atoms selected from S, N and O, including for example, pyrrolyl, furanyl, thienyl, piperidinyl, imidazolyl, oxazolyl, isoxazolyl, thiazolyl, thiadiazolyl, pyra- zolyl, pyridinyl, pyrrolidinyl, pyrimidinyl, morpholinyl, piperazinyl, indolyl, morpholinyl, benzofuranyl, pyranyl, isoxazolyl, quinuclidinyl, aza-bicyclo[3.2.1]octanyl, benzimidazolyl, methylenedioxyphenyl, maleimido and succinimido groups.

As used herein the term "heteroaryl" refers to a 5- or 6-membered aromatic ring containing one or more he- teroatoms. Illustrative of such groups are thienyl, furyl, pyrrolyl, imidazolyl, benzimidazolyl, thiazolyl, pyrazolyl, isoxazolyl, isothiazolyl, triazolyl, thiadiazolyl, oxadiazolyl, pyridinyl, pyridazinyl, pyrimi- dinyl, pyrazinyl, triazinyl.

In certain embodiments, the therapeutic agent as disclosed herein, particularly the therapeutic agent capable of interacting with at least one of the CD80 or CD86 polypeptides, may be selected from the group comprising, consisting essentially of or consisting of an antibody or a fragment or derivative thereof, a soluble receptor, and an aptamer.

As used herein, the term "antibody" is used in its broadest sense and generally refers to any immunologic binding agent, such as a whole antibody, including without limitation a chimeric, humanized, human, recombinant, transgenic, grafted and single chain antibody, and the like, or any fusion proteins, conjugates, fragments, or derivatives thereof that contain one or more domains that selectively bind to an antigen of interest. The term antibody thereby includes a whole immunoglobulin molecule, a monoclonal antibody, a chimeric antibody, a humanized antibody, a human antibody, or an immunologically effective fragment of any of these. The term thus specifically encompasses intact monoclonal antibodies, polyclonal antibodies, multivalent (e.g., 2-, 3- or more-valent) and/or multi-specific antibodies (e.g., bi- or more-specific antibodies) formed from at least two intact antibodies, and antibody fragments insofar they exhibit the desired biological activity (particularly, ability to specifically bind an antigen of interest), as well as multivalent and/or multi-specific composites of such fragments. The term "antibody" is not only inclusive of antibodies generated by methods comprising immunisation, but also includes any polypeptide, e.g., a recombinantly expressed polypeptide, which is made to encompass at least one complementarity-determining region (CDR) capable of specifically binding to an epitope on an antigen of interest. Hence, the term applies to such molecules regardless whether they are produced *in vitro,* in cell culture, or *in vivo.*

The term "immunoglobulin sequence" - whether used herein to refer to a heavy chain antibody or to a conventional 4-chain antibody - is used as a general term to include both the full-size antibody, the individual chains thereof, as well as all parts, domains or fragments thereof (including but not limited to antigen-binding domains or fragments such as VHH domains or VH/VL domains, respectively). In addition, the term "sequence" as used herein (for example in terms like "immunoglobulin sequence", "antibody sequence", "variable domain sequence", "VHH sequence" or "protein sequence"), should generally be understood to include both the relevant amino acid sequence as well as nucleic acid sequences or nucleotide sequences encoding the same, unless the context requires a more limited interpretation.

The term "epitope" includes any polypeptide determinant capable of specifically binding to an immunoglobulin or T-cell receptor. Epitope determinants may include chemically active surface groupings of molecules such as amino acids, sugar side chains, phosphoryl, or sulfonyl, and may have specific three dimensional structural characteristics, and/or specific charge characteristics. An epitope is a region of an antigen that is bound by an antibody. An antibody is said to specifically bind an antigen when it preferentially recognizes its target antigen in a complex mixture of proteins and/or macromolecules.

The terms "binding region", "binding site" or "interaction site" shall herein have the meaning of a particular site, part, domain or stretch of amino acid residues that is responsible for binding to an antigen of interest. Such binding region essentially consists of specific amino acid residues of the antibodies described herein, which residues are in contact with the target molecule.

The term "specificity" refers to the number of different types of antigens or antigenic determinants to which a particular antigen-binding molecule or antigen-binding protein (such as an antibody) molecule can bind. The specificity of an antigen-binding protein can be determined based on affinity and/or avidity. The affinity, represented by the equilibrium constant for the dissociation of an antigen with an antigen-binding protein (KD), is a measure for the binding strength between an antigenic determinant and an antigen-binding site on the antigen-binding protein: the lesser the value of the KD, the stronger the binding strength between an antigenic determinant and the antigen-binding molecule (alternatively, the affinity can also be expressed as the affinity constant (KA), which is 1/KD). As will be clear to the skilled person, affinity can be determined in a manner known *per se,* depending on the specific antigen of interest. Avidity is the measure of the strength of binding between an antigen-binding molecule (such as an antibody) and the pertinent antigen. Avidity is related to both the affinity between an antigenic determinant and its antigen binding site on the antigen-binding molecule and the number of pertinent binding sites present on the antigen-binding molecule. Typically, antigen-binding proteins (such as antibodies) will bind with a dissociation constant (KD) of 10⁻⁵ to 10⁻¹² moles/liter (M) or less, and preferably 10⁻⁷ to 10⁻¹² moles/liter (M) or less and more preferably 10⁻⁸ to 10⁻¹² moles/liter, and/or with an association constant(KA) of at least 10⁷ M⁻¹, preferably at least 10⁸ M⁻¹, more preferably at least 10⁹ M⁻¹, such as at least 10¹² M⁻¹. Any KD value greater than 10⁻⁴ M is generally considered to indicate non-specific binding. Preferably, an antibody will bind to the desired antigen with an KD less than 500 nM, preferably less than 200 nM, more preferably less than 10 nM, such as less than 500 pM. Specific binding of an antigen-binding protein to an antigen or antigenic determinant can be determined in any suitable manner known *per se,* including, for example, Scatchard analysis and/or competitive binding assays, such as radioimmunoassays (RIA), enzyme immunoassays (EIA) and sandwich competition assays, and the different variants thereof known per *se* in the art.

A full-length antibody as it exists naturally is an immunoglobulin molecule comprising 2 heavy (H) chains and 2 light (L) chains interconnected by disulfide bonds. The amino terminal portion of each chain includes a variable region of about 100-110 amino acids primarily responsible for antigen recognition via the complementarity determining regions (CDRs) contained therein. The carboxy-terminal portion of each chain defines a constant region primarily responsible for effector function.

The CDRs are interspersed with regions that are more conserved, termed framework regions (FR). Each light chain variable region (LCVR) and heavy chain variable region (HCVR) is composed of 3 CDRs and 4 FRs, arranged from amino-terminus to carboxy-terminus in the following order: FR1, CDR1, FR2, CDR2, FR3, CDR3, FR4. The 3 CDRs of the light chain are referred to as "LCDR1, LCDR2, and LCDR3" and the 3 CDRs of the heavy chain are referred to as "CDR1, HCDR2, and HCDR3." The CDRs contain most of the residues which form specific interactions with the antigen. The numbering and positioning of CDR amino acid residues within the LCVR and HCVR regions is in accordance with the well-known Kabat numbering convention, which refers to a system of numbering amino acid residues which are more variable (i.e., hypervariable) than other amino acid residues in the heavy and light chain regions of an antibody (Kabat, et al., Ann. NYAcad. Sci. 190:382-93 (1971); Kabat, et al., Sequences of Proteins of Immunological Interest, Fifth Edition, U.S. Department of Health and Human Services, NIH Publication No. 91-3242 (1991)). The positioning of CDRs in the variable region of an antibody follows Kabat numbering or simply, "Kabat."

Light chains are classified as kappa or lambda, and are characterized by a particular constant region as known in the art. Heavy chains are classified as gamma, mu, alpha, delta, or epsilon, and define the isotype of an antibody as IgG, IgM, IgA, IgD, or IgE, respectively. IgG antibodies can be further divided into subclasses, e.g., IgG1, IgG2, IgG3, IgG4. Each heavy chain type is characterized by a particular constant region with a sequence well known in the art.

In certain embodiments, an antibody may be any of IgA, IgD, IgE, IgG and IgM classes, and preferably IgG class antibody.

In certain embodiments, the antibody may be a polyclonal antibody, e.g., an antiserum or immunoglobulins purified there from (e.g., affinity-purified).

In other embodiments, the antibody may be a monoclonal antibody or a mixture of monoclonal antibodies. Monoclonal antibodies can target a particular antigen or a particular epitope within an antigen with greater selectivity and reproducibility.

As used herein, the term "monoclonal antibody" refers to an antibody that is derived from a single copy or clone including, for example, any eukaryotic, prokaryotic, or phage clone, and not the method by which it is produced. Monoclonal antibodies preferably exist in a homogeneous or substantially homogeneous population. Monoclonal antibodies and antigen-binding fragments thereof of the present invention can be produced, for example, by recombinant technologies, phage display technologies, synthetic technologies, e.g., CDR-grafting, or combinations of such technologies, or other technologies known in the art.

By means of example and not limitation, monoclonal antibodies may be made by the hybridoma method first described by Kohler et al. 1975 (Nature 256: 495), or may be made by recombinant DNA methods (e.g., as in US 4,816,567). Monoclonal antibodies may also be made using phage antibody libraries using techniques as described by Clackson et al. 1991 (Nature 352: 624-628) and Marks et al. 1991 (J Mol Biol 222: 581-597).

These latter techniques are based on the phage display technology as described *inter alia* in US5837500, US5571698, US5223409, US7118879, US7208293 and US7413537. Briefly, therapeutic candidate molecules, e.g., human antibody fragments (e.g., Fabs), peptides, and small proteins, are displayed on the surface of a small bacterial virus called a bacteriophage (or phage). A collection of displayed molecules is known as a library. Phage display enables to search through these libraries to identify molecules that bind, preferably with high specificity and/or affinity, to targets of interest, e.g. therapeutic targets. Non-limiting examples of phage antibody libraries include HuCAL® (Human Combinatorial Antibody Library, Morphosys), Ylanthia® (Morphosys), the human Fab fragment libraries described in WO200070023, and the macaque antibody library as described in WO 1996040878. The Human Combinatorial Antibody Library (Morphosys) has been prepared as described in WO 199708320 using synthetic consensus sequences which cover the structural repertoire of antibodies encoded in the human genome.

Non-limiting examples of monoclonal antibodies suitable for use as therapeutic agents herein include the monkey monoclonal antibodies 7B6, 16C10, 7C10 and 20C9 which inhibit the interaction of CD80 and/or CD86 polypeptides with CD28 polypeptide as described in WO 1996040878; the anti-human CD80 monoclonal antibody 5B5D1 as deposited in the ECACC collection under No. 95060211, the anti-human CD86 monoclonal antibody 1G10H6D10 as deposited in the ECACC collection under No. 95060210, the monoclonal antibody B7-24 as described in WO199401547 that specifically binds to the B7-1 molecule but not to B7-2.

Further examples of anti-CD80 antibodies include IDEC114 (Biogen Idec) and Galiximab (Cancer and Leukemia Group B).

In certain embodiments, the therapeutic agent may be an antibody fragment.

The term "antibody fragment" or "antigen -binding moiety" comprises a portion or region of a full length antibody, generally the antigen binding or variable domain thereof. Examples of antibody fragments include Fab, Fab', F(ab)2, Fv , sFv fragments, single domain (sd)Fv, such as V_{H} domains , V_{L} domains and V_{HH} domains, diabodies, linear antibodies, single-chain antibody molecules, in particular heavy-chain antibodies; and multivalent and/or multispecific antibodies formed from antibody fragment(s), e.g., dibodies, tribodies, and multibodies. The above designations Fab, Fab', F(ab')2, Fv, scFv *etc.* are intended to have their art-established meaning.

The term "antigen-binding portion" or "antigen-binding region" refers to one or more fragments of an antibody that retain the ability to specifically bind to an antigen. It has been shown that the antigen-binding function of an antibody may be performed by fragments of a full-length antibody. These may also be bispecific, dual specific, or multi-specific formats; specifically binding to two or more different antigens. Examples of binding fragments encompassed within the term "antigen-binding portion" of an antibody include (i) a Fab fragment, a monovalent fragment consisting of the VL, VH, CL and CHI domains; (ii) a F(ab')2 fragment, a bivalent fragment comprising two Fab fragments linked by a disulfide bridge at the hinge region; (iii) a Fd fragment consisting of the VH and CHI domains; (iv) a Fv fragment consisting of the VL and VH domains of a single arm of an antibody, (v) a dAb fragment (Ward et al., Nature, 341 : 544-546 (1989); PCT publication WO 90/05144), which comprises a single variable domain; and (vi) an isolated complementarity determining region (CDR). Furthermore, although the two domains of the Fv fragment, VL and VH, are coded for by separate genes, they may be joined, using recombinant methods, by a synthetic linker that enables them to be made as a single protein chain in which the VL and VH regions pair to form monovalent molecules (known as single chain Fv (scFv) (Bird et al., Science, 242: 423-426 (1988); and Huston et al., Proc. Natl. Acad. Sci., 85: 5879-5883 (1988)). Such single chain antibodies are also intended to be encompassed within the term "antigen-binding portion" of an antibody. Other forms of single chain antibodies, such as diabodies are also encompassed.

Diabodies are bivalent, bispecific antibodies in which VH and VL domains are expressed on a single polypeptide chain, but using a linker that is too short to allow for pairing between the two domains on the same chain, thereby forcing the domains to pair with complementary domains of another chain and creating two antigen binding sites (Holliger, et al., Proc. Natl. Acad. Sci., 90: 6444-6448 (1993); Poljak, et al., Structure 2: 1121-1123 (1994)). Such antibody binding portions are known in the art (Kontermann and Dubel eds., Antibody Engineering (2001) Springer- Verlag. New York. 790 pp. (ISBN 3-540-41354-5).

Still further, an antibody or antigen-binding portion thereof may be part of a larger immunoadhesion molecule, formed by covalent or noncovalent association of the antibody or antibody portion with one or more other proteins or peptides. Examples of such immunoadhesion molecules include use of the streptavidin core region to make a tetrameric scFv molecule (Kipriyanov, S.M., et al., Human Antibodies and Hybridomas, 6: 93-101 (1995)) and use of a cysteine residue, a marker peptide and a C-terminal polyhistidine tag to make bivalent and biotinylated scFv molecules (Kipriyanov, et al., Mol. Immunol., 31 : 1047-1058 (1994)). Antibody portions, such as Fab and F(ab')2 fragments, may be prepared from whole antibodies using conventional techniques, such as papain or pepsin digestion, respectively, of whole antibodies. Moreover, antibodies, antibody portions and immunoadhesion molecules may be obtained using standard recombinant DNA techniques.

In certain embodiments, the therapeutic agent may be a Nanobody®. The terms "Nanobody®" and "Nanobodies®" are trademarks of Ablynx NV (Belgium). The term "Nanobody" is well-known in the art and as used herein in its broadest sense encompasses an immunological binding agent obtained (1) by isolating the V_{HH} domain of a naturally occurring heavy-chain antibody, preferably a heavy-chain antibody derived from camelids; (2) by expression of a nucleotide sequence encoding a naturally occurring V_{HH} domain; (3) by "humanization" of a naturally occurring V_{HH} domain or by expression of a nucleic acid encoding a such humanized V_{HH} domain; (4) by "camelization" of a naturally occurring V_{H} domain from any animal species, and in particular from a mammalian species, such as from a human being, or by expression of a nucleic acid encoding such a camelized V_{H} domain; (5) by "camelisation" of a "domain antibody" or "dAb" as described in the art, or by expression of a nucleic acid encoding such a camelized dAb; (6) by using synthetic or semi-synthetic techniques for preparing proteins, polypeptides or other amino acid sequences known *per se;* (7) by preparing a nucleic acid encoding a Nanobody using techniques for nucleic acid synthesis known *per se,* followed by expression of the nucleic acid thus obtained; and/or (8) by any combination of one or more of the foregoing. "Camelids" as used herein comprise old world camelids (*Camelus bactrianus* and *Camelus dromaderius)* and new world camelids (for example *Lama paccos, Lama glama* and *Lama vicugna*). Exemplary Nanobodies suitable for use herein are the Nanobodies raised against B7-1 and/or B7-2 described in Table B-1 on page 371-372 and Table B-2 on page 372-374 of the publication of WO2008071447.

The amino acid sequence and structure of a Nanobody can be considered - without however being limited thereto - to be comprised of four framework regions or "FR's", which are referred to in the art and herein as "Framework region 1" or "FR1"; as "Framework region 2" or "FR2"; as "Framework region 3" or "FR3"; and as "Framework region 4" or "FR4", respectively; which framework regions are interrupted by three complementary determining regions or "CDR's", which are referred to in the art as "Complementarity Determining Region 1"or "CDR1"; as "Complementarity Determining Region 2" or "CDR2"; and as "Complementarity Determining Region 3" or "CDR3", respectively. The total number of amino acid residues in a Nanobody can be in the region of 110-120, and preferably 112-115. It should however be noted that parts, fragments, analogs or derivatives of a Nanobody are not particularly limited as to their length and/or size, as long as such parts, fragments, analogs or derivatives meet the further requirements outlined herein and are preferably suitable for the purposes described herein.

Preferred CDR sequences and framework sequences for Nanobodies against B7-1 and/or B7-2 are indicated in Table A-1a on pages 148 and 149 of the publication of WO2008071447. Nanobodies suitable for use herein may have at least one, preferably at least two, most preferably all three, of the CDR1, CDR2 and CDR3 sequences present chosen from the group consisting of the CDR1, CDR2 and CDR3 sequences, respectively, listed in said Table A-1a; or from the group of CDR1, CDR2 and CDR3 sequences, respectively, that have at least 80%, preferably at least 90%, more preferably at least 95%, even more preferably at least 99% sequence identity with at least one of the CDR1, CDR2 and CDR3 sequences, respectively, listed in said Table A-1a; and/or from the group consisting of the CDR1, CDR2 and CDR3 sequences, respectively, that have 3, 2, or only 1 amino acid difference with at least one of the CDR1, CDR2 and CDR3 sequences, respectively, listed in said Table A-1a, preferably chosen from the group consisting of the CDR1, CDR2 and CDR3 sequences, respectively, listed in said Table A-1a. Preferably, Nanobodies suitable for use herein may have at least the CDR3 sequence present chosen from the group consisting of the CDR3 sequences listed in said Table A-1a. Preferred combinations of CDR sequences, preferred combinations of framework sequences, and preferred combinations of framework and CDR sequences for Nanobodies against B7-1 and/or B7-2 are indicated in Table A-1a on pages 148 and 149 of the publication of WO2008071447.

The amino acid residues of a Nanobody are numbered according to the general numbering for VH domains given by Kabat et al. ("Sequence of proteins of immunological interest", US Public Health Services, NIH Bethesda, MD, Publication No. 91). According to this numbering, FR1 of a Nanobody comprises the amino acid residues at positions 1-30, CDR1 of a Nanobody comprises the amino acid residues at positions 31-35, FR2 of a Nanobody comprises the amino acids at positions 36-49, CDR2 of a Nanobody comprises the amino acid residues at positions 50-65, FR3 of a Nanobody comprises the amino acid residues at positions 66-94, CDR3 of a Nanobody comprises the amino acid residues at positions 95-102, and FR4 of a Nanobody comprises the amino acid residues at positions 103-113. [In this respect, it should be noted that - as is well known in the art for VH domains and for VHH domains - the total number of amino acid residues in each of the CDR's may vary and may not correspond to the total number of amino acid residues indicated by the Kabat numbering (that is, one or more positions according to the Kabat numbering may not be occupied in the actual sequence, or the actual sequence may contain more amino acid residues than the number allowed for by the Kabat numbering). This means that, generally, the numbering according to Kabat may or may not correspond to the actual numbering of the amino acid residues in the actual sequence. Generally, however, it can be said that, according to the numbering of Kabat and irrespective of the number of amino acid residues in the CDR's, position 1 according to the Kabat numbering corresponds to the start of FR1 and vice versa, position 36 according to the Kabat numbering corresponds to the start of FR2 and vice versa, position 66 according to the Kabat numbering corresponds to the start of FR3 and vice versa, and position 103 according to the Kabat numbering corresponds to the start of FR4 and vice versa.]. Alternative methods for numbering the amino acid residues of VH domains, which methods can also be applied in an analogous manner to VHH domains from Camelids and to Nanobodies, are the method described by Chothia et al. (Nature 342, 877-883 (1989)), the so-called "AbM definition" and the so-called "contact definition". However, in the present description, claims and figures, the numbering according to Kabat as applied to VHH domains by Riechmann and Muyldermans will be followed, unless indicated otherwise.

The Nanobodies may be "humanized". For example, humanized Nanobodies suitable for use herein are Nanobodies which can bind to B7-1 and/or B7-2 and which: i) are a humanized variant of one of the amino acid sequences listed in Table B-1 on pages 371-372 of WO2008071447 or ; and/or ii) have at least 80% amino acid identity with at least one said amino acid sequences, in which for the purposes of determining the degree of amino acid identity, the amino acid residues that form the CDR sequences are disregarded; and in which: iii) preferably one or more of the amino acid residues at positions 11, 37, 44, 45, 47, 83, 84, 103, 104 and 108 according to the Kabat numbering are chosen from the Hallmark residues mentioned in Table A-3 on page 259 of WO2008071447. In addition, or alternatively, other potentially useful humanizing substitutions can be ascertained by comparing the sequence of the framework regions of a naturally occurring VHH sequence with the corresponding framework sequence of one or more closely related human VH sequences, after which one or more of the potentially useful humanizing substitutions (or combinations thereof) thus determined can be introduced into said VHH sequence (in any manner known *per se)* and the resulting humanized VHH sequences can be tested for affinity for the target, for stability, for ease and level of expression, and/or for other desired properties. In this way, by means of a limited degree of trial and error, other suitable humanizing substitutions (or suitable combinations thereof) can be determined by the skilled person. Also, based on the foregoing, (the framework regions of) a Nanobody may be partially humanized or fully humanized.

For a general description of heavy chain antibodies and the variable domains thereof, reference is *inter alia* made to the following references, which are mentioned as general background art: WO 94/04678, WO 95/04079 and WO 96/34103 of the Vrije Universiteit Brussel; WO 94/25591, WO 99/37681, WO 00/40968, WO 00/43507, WO 00/65057, WO 01/40310, WO 01/44301, EP 1134231 and WO 02/48193 of Unilever; WO 97/49805, WO 01/21817, WO 03/035694, WO 03/054016 and WO 03/055527 of the Vlaams Instituut voor Biotechnologie (VIB); WO 03/050531 of Algonomics N.V. and applicant; WO 01/90190 by the National Research Council of Canada; WO 03/025020 (= EP 1 433 793) by the Institute of Antibodies; as well as WO 04/041867, WO 04/041862, WO 04/041865, WO 04/041863, WO 04/062551 by applicant and the further published patent applications by applicant; Hamers-Casterman et al., Nature 1993 June 3; 363 (6428): 446-8; Davies and Riechmann, FEBS Lett. 1994 Feb 21; 339(3): 285-90; Muyldermans et al., Protein Eng. 1994 Sep; 7(9): 1129-3; Davies and Riechmann, Biotechnology (NY) 1995 May; 13(5): 475-9; Gharoudi et al., 9th Forum of Applied Biotechnology, Med. Fac. Landbouw Univ. Gent. 1995; 60/4a part I: 2097-2100; Davies and Riechmann, Protein Eng. 1996 Jun; 9(6): 531-7; Desmyter et al., Nat Struct Biol. 1996 Sep; 3(9): 803-11; Sheriff et al., Nat Struct Biol. 1996 Sep; 3(9): 733-6; Spinelli et al., Nat Struct Biol. 1996 Sep; 3(9): 752-7; Arbabi Ghahroudi et al., FEBS Lett. 1997 Sep 15; 414(3): 521-6; Vu et al., MoI Immunol. 1997 Nov-Dec; 34(16-17): 1121-31; Atarhouch et al., Journal of Camel Practice and Research 1997; 4: 177-182; Nguyen et al., J. MoI. Biol. 1998 Jan 23; 275(3): 413-8; Lauwereys et al., EMBO J. 1998 Jul 1; 17(13): 3512-20; Frenken et al., Res Immunol. 1998 Jul-Aug; 149(6): 589-99; Transue et al., Proteins 1998 Sep 1; 32(4): 515-22; Muyldermans and Lauwereys, J. MoI. Recognit. 1999 Mar- Apr; 12 (2): 131-40; van der Linden et al., Biochim. Biophys. Acta 1999 Apr 12; 1431(1): 37-46.; Decanniere et al., Structure Fold. Des. 1999 Apr 15; 7(4): 361-70; Ngyuen et al., MoI. Immunol. 1999 Jun; 36(8): 515-24; Woolven et al., Immunogenetics 1999 Oct; 50 (1-2): 98- 101; Riechmann and Muyldermans, J. Immunol. Methods 1999 Dec 10; 231 (1-2): 25-38; Spinelli et al., Biochemistry 2000 Feb 15; 39(6): 1217-22; Frenken et al., J. Biotechnol. 2000 Feb 28; 78(1): 11-21; Nguyen et al., EMBO J. 2000 Mar 1; 19(5): 921-30; van der Linden et al., J. Immunol. Methods 2000 Jun 23; 240 (1-2): 185-95; Decanniere et al., J. MoI. Biol. 2000 Jun 30; 300 (1): 83-91; van der Linden et al., J. Biotechnol. 2000 Jul 14; 80(3): 261-70; Harmsen et al., MoI. Immunol. 2000 Aug; 37(10): 579-90; Perez et al., Biochemistry 2001 Jan 9; 40(1): 74-83; Conrath et al., J. Biol. Chem. 2001 Mar 9; 276 (10): 7346-50; Muyldermans et al., Trends Biochem Sci. 2001 Apr;26(4):230-5; Muyldermans S., J. Biotechnol. 2001 Jun; 74 (4): 277-302; Desmyter et al., J. Biol. Chem. 2001 Jul 13 ;276 (28): 26285-90; Spinelli et al., J. MoI. Biol. 2001 Aug 3; 311 (1): 123-9; Conrath et al., Antimicrob Agents Chemother. 2001 Oct; 45 (10): 2807-12; Decanniere et al., J. MoI. Biol. 2001 Oct 26; 313(3): 473-8; Nguyen et al., Adv Immunol. 2001; 79: 261-96; Muruganandam et al., FASEB J. 2002 Feb; 16 (2): 240-2; Ewert et al., Biochemistry 2002 Mar 19; 41 (11): 3628-36; Dumoulin et al., Protein Sci. 2002 Mar; 11 (3): 500-15; Cortez-Retamozo et al., Int. J. Cancer. 2002 Mar 20; 98 (3): 456-62; Su et al., MoI. Biol. Evol. 2002 Mar; 19 (3): 205-15; van der Vaart JM., Methods MoI Biol. 2002; 178: 359-66; Vranken et al., Biochemistry 2002 Jul 9; 41 (27): 8570-9; Nguyen et al., Immunogenetics 2002 Apr; 54 (1): 39-47; Renisio et al., Proteins 2002 Jun 1; 47 (4): 546-55; Desmyter et al., J. Biol. Chem. 2002 Jun 28; 277 (26): 23645-50; Ledeboer et al., J. Dairy Sci. 2002 Jun; 85 (6): 1376-82; De Genst et al., J. Biol. Chem. 2002 Aug 16; 277 (33): 29897-907; Ferrat et al., Biochem. J. 2002 Sep 1; 366 (Pt 2): 415-22; Thomassen et al., Enzyme and Microbial Technol. 2002; 30: 273-8; Harmsen et al., Appl. Microbiol. Biotechnol. 2002 Dec; 60 (4): 449-54; Jobling et al., Nat Biotechnol. 2003 Jan; 21 (1): 77-80; Conrath et al., Dev. Comp. Immunol. 2003 Feb; 27 (2): 87-103; Pleschberger et al., Bioconjug. Chem. 2003 Mar-Apr; 14 (2): 440-8; Lah et al., J. Biol. Chem. 2003 Apr 18; 278 (16): 14101-11; Nguyen et al., Immunology. 2003 May; 109 (1): 93-101; Joosten et al., Microb. Cell Fact. 2003 Jan 30; 2 (1): 1; Li et al., Proteins 2003 Jul 1; 52 (1): 47-50; Loris et al., Biol Chem. 2003 Jul 25; 278 (30): 28252-7; van Koningsbruggen et al., J. Immunol. Methods. 2003 Aug; 279 (1-2): 149-61; Dumoulin et al., Nature. 2003 Aug 14; 424 (6950): 783-8; Bond et al., J. MoI. Biol. 2003 Sep 19; 332 (3): 643-55; Yau et al., J. Immunol. Methods. 2003 Oct 1; 281 (1-2): 161-75; Dekker et al., J. Virol. 2003 Nov; 77 (22): 12132-9; Meddeb-Mouelhi et al., Toxicon. 2003 Dec; 42 (7): 785-91; Verheesen et al., Biochim. Biophys. Acta 2003 Dec 5; 1624 (1-3): 21-8; Zhang et al., J MoI Biol. 2004 Jan 2; 335 (1): 49-56; Stijlemans et al., J Biol Chem. 2004 Jan 9; 279 (2): 1256-61; Cortez- Retamozo et al., Cancer Res. 2004 Apr 15; 64 (8): 2853-7; Spinelli et al., FEBS Lett. 2004 Apr 23; 564 (1-2): 35-40; Pleschberger et al., Bioconjug. Chem. 2004 May-Jun; 15 (3): 664- 71; Nicaise et al., Protein Sci. 2004 Jul; 13 (7): 1882-91 ; Omidfar et al., Tumour Biol. 2004 Jul-Aug; 25 (4): 179-87; Omidfar et al., Tumour Biol. 2004 Sep-Dec; 25(5-6): 296-305; Szynol et al., Antimicrob Agents Chemother. 2004 Sep;48(9):3390-5; Saerens et al., J. Biol. Chem. 2004 Dec 10; 279 (50): 51965-72; De Genst et al., J. Biol. Chem. 2004 Dec 17; 279 (51): 53593-601; Dolk et al., Appl. Environ. Microbiol. 2005 Jan; 71(1): 442-50; Joosten et al., Appl Microbiol Biotechnol. 2005 Jan; 66(4): 384-92; Dumoulin et al., J. MoI. Biol. 2005 Feb 25; 346 (3): 773-88; Yau et al., J Immunol Methods. 2005 Feb; 297 (1-2): 213-24; De Genst et al., J. Biol. Chem. 2005 Apr 8; 280 (14): 14114-21; Huang et al., Eur. J. Hum. Genet. 2005 Apr 13; Dolk et al., Proteins. 2005 May 15; 59 (3): 555-64; Bond et al., J. MoI. Biol. 2005 May 6;348(3):699-709; Zarebski et al., J. Mol. Biol. 2005 Apr 21.

In accordance with the terminology used in the above references, the variable domains present in naturally occurring heavy chain antibodies will also be referred to as "VHH domains", in order to distinguish them from the heavy chain variable domains that are present in conventional 4-chain antibodies (which will be referred to herein as "VH domains") and from the light chain variable domains that are present in conventional 4-chain antibodies (which will be referred to herein as "VL domains"). As mentioned in the prior art referred to above, VHH domains have a number of unique structural characteristics and functional properties which make isolated VHH domains (as well as Nanobodies based thereon, which share these structural characteristics and functional properties with the naturally occurring VHH domains) and proteins containing the same highly advantageous for use as functional antigen-binding domains or proteins. In particular, and without being limited thereto, VHH domains (which have been "designed" by nature to functionally bind to an antigen without the presence of, and without any interaction with, a light chain variable domain) and Nanobodies can function as a single, relatively small, functional antigen-binding structural unit, domain or protein. This distinguishes the VHH domains from the VH and VL domains of conventional 4-chain antibodies, which by themselves are generally not suited for practical application as single antigen-binding proteins or domains, but need to be combined in some form or another to provide a functional antigen-binding unit (as in for example conventional antibody fragments such as Fab fragments; in ScFv's fragments, which consist of a VH domain covalently linked to a VL domain).

In certain embodiments, the therapeutic agent may be a domain antibody (dAb). For the term "dAb", reference is for example made to Ward et al. (Nature 1989 Oct. 12; 341 (6242): 544-6), to Holt et al., Trends Biotechnol., 2003, 21(11):484-490; as well as to for example WO 06/030220, WO 06/003388 and other published patent applications of Domantis Ltd. It should also be noted that, although potentially but not necessarily less preferred in the context of the present invention because they are not of mammalian origin, single domain antibodies or single variable domains can be derived from certain species of shark (for example, the so-called "IgNAR domains", see for example WO 05/18629). Exemplary dAbs suitable for use herein include the dAbs described in US 13/021,127, in particular domain antibodies which bind the same epitope as domain antibody 1 h-239-891 (D70C), including D70C, more particularly dAbs that comprise a first single variable domain having a binding specificity to a first antigen and a second single variable domain having a binding activity to a second antigen, wherein the first antigen is CD28 and wherein the second antigen is an antigen other than CD28, and dAbs described in claim 7 of the US 13/021,127 publication.

In further embodiments, the antibody or antibody fragment may be multispecific (such as a bispecific, trispecific, etc. antibody) comprising at least two (such as two, three, etc.) binding sites, each directed against a different antigen or antigenic determinant. Exemplary multispecific antibody fragments suitable for use as therapeutic agents herein include the trispecific diabody capable of binding to CD40 and to both CD80 and CD86, the bispecific diabody capable of binding to human CD40 and to human CD86, and the trispecific triabody capable of binding to CD40, CD80 and CD86 as described in WO 1998003670.

In some embodiments, the therapeutic agent may be a dual variable domain immunoglobulin (DVD-Ig™).

The term antibody includes antibodies originating from or comprising one or more portions derived from any animal species, preferably vertebrate species, including, e.g., birds and mammals. Without limitation, the antibodies may be chicken, turkey, goose, duck, guinea fowl, quail or pheasant. Also without limitation, the antibodies may be human, murine (e.g., mouse, rat, etc.), porcine, donkey, rabbit, goat, sheep, guinea pig, monkey (e.g., cynomolus monkeys), camel (*e.g., Camelus bactrianus* and *Camelus dromaderius)* also including camel heavy-chain antibodies, llama *(e.g., Lama paccos, Lama glama* or *Lama vicugna*) also including llama heavy-chain antibodies, or horse.

The term antibody as used herein also encompasses "chimeric antibodies" which originate from at least two animal species. More specifically, the term "chimeric antibody" or "chimeric antibodies" refers to antibodies which comprise heavy and light chain variable region sequences from one species and constant region sequences from another species, such as for example antibodies having murine heavy and light chain variable regions linked to human, non-human primate, canine, equine, or feline constant regions. Chimeric antibodies comprise a portion of the heavy and/or light chain that is identical to or homologous with corresponding sequences from antibodies derived from a particular species or belonging to a particular antibody class or subclass, while the remainder of the chain(s) is identical to or homologous with corresponding sequences in antibodies from another species or belonging to another antibody class or subclass, as well as fragments of such antibodies, exhibiting the desired biological activity (See e.g., U.S. Pat. No. 4,816,567; and Morrison et al., Proc. Natl. Acad. Sci. USA 81 :6851-6855 (1984)). Chimeric antibodies are made through merging DNA encoding a portion, such as the Fv region, of a monoclonal antibody from one species, e.g. mouse or monkey, with the antibody-producing DNA from another species, e.g. human.

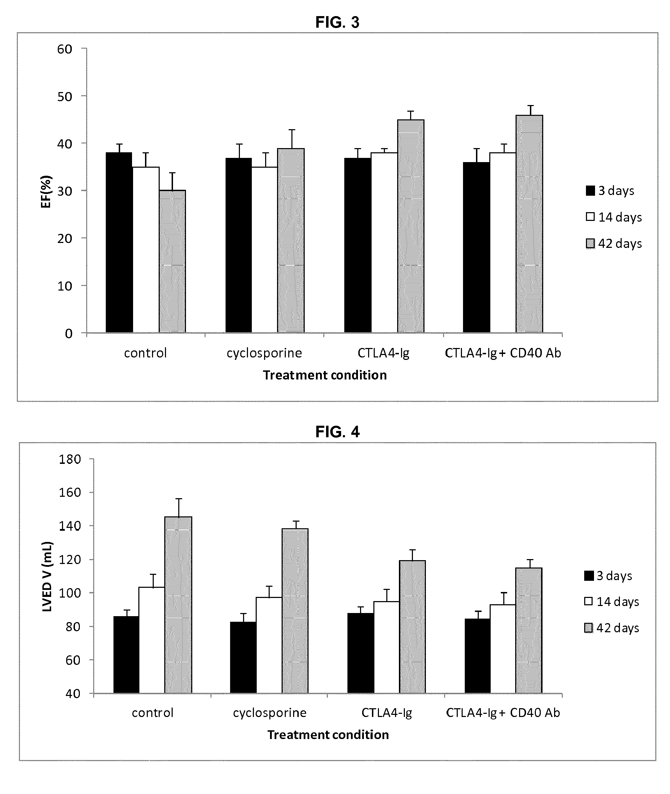

Non-limiting examples of chimeric antibodies suitable for use as therapeutic agents herein include the primatized antibodies derived from the monkey monoclonal antibodies specific to human CD80 and/or CD86, in particular 16C10, 7C10, 20C9 and 7B6, as described in WO 1996040878, including the primatized antibody derived from 7C10 monkey monoclonal antibody, which primatized antibody has the amino acid sequence set forth in Figures 8a and 8b of WO 1996040878; the primatized antibody derived from 7B6 monkey monoclonal antibody, which primatized antibody has the amino acid sequence set forth in Figures 9a and 9b of WO 1996040878; and the primatized antibody derived from 16C10 monkey monoclonal antibody, which primatized antibody has the amino acid sequence set forth in Figures 4a and 4b of WO 1996040878. These primatized antibodies comprise the variable heavy and light domains of the monkey antibody, and human constant domains.

In certain embodiments, the therapeutic agent may be a "fully human antibody". As used herein, the term "fully human antibody" refers to an antibody of which the encoding genetic information is of human origin. Accordingly, the term "fully human antibody" refers to antibodies having variable and constant regions derived only from human germline immunoglobulin sequences. The term "fully human antibody" is thus not to include antibodies in which CDR sequences derived from the germline of other mammalian species, such as a mouse, have been grafted onto human framework sequences. Fully human antibodies may be derived from phage human antibody libraries as described above, or they may be obtained through immunization of transgenic mice which have been engineered to replace the murine immunoglobulin encoding region as described in Lonberg and Husznar 1995 (Int. Rev. Immunol. 13 (1): 65-93). Fully human antibodies that are made using phage display are preferably produced by recombinant expression in a human cell line resulting in antibodies with a human glycosylation pattern. Non-limiting examples of fully human antibodies are HuCAL® antibodies (Morphosys). The genetic information for constructing a HuCAL® antibody is extracted from the HuCAL® antibody library (Morphosys) and introduced into human PER.C6® cells in the form of a vector (i.e., transfection). The transfected cells translate the genetic information into protein. The protein is further modified by glycosylation and the resulting antibody molecule is finally secreted by the cells into the culture medium.

The term antibody as used herein also encompasses "humanized antibodies", which are antibodies derived from non-human species whose protein sequence have been modified so as to increase their similarity to antibodies produced naturally in humans. More particularly, the term "humanized antibody" refers to antibodies which comprise heavy and light chain variable region sequences from a non -human species (e.g., a mouse) but in which at least a portion of the VH and/or VL sequence has been altered to be more "human-like", i.e., more similar to human germline variable sequences. One type of humanized antibody is a CDR-grafted antibody, in which non-human CDR sequences are introduced into human VH and VL sequences to replace the corresponding human CDR sequences.

The humanized antibody is an antibody or a variant, derivative, analog or fragment thereof which immunospecifically binds to an antigen of interest and which comprises a framework (FR) region having substantially the amino acid sequence of a human antibody and a complementary determining region (CDR) having substantially the amino acid sequence of a non-human antibody. A humanized antibody comprises substantially all, or at least one, and typically two, variable domains (Fab, Fab', F(ab') 2, FabC, Fv) in which all or substantially all of the CDR regions correspond to those of a non-human immunoglobulin (i.e., donor antibody) and all or substantially all of the framework regions are those of a human immunoglobulin consensus sequence. A humanized antibody also comprises at least a portion of an immunoglobulin constant region (Fc), typically that of a human immunoglobulin. A humanized antibody may contain both the light chain as well as at least the variable domain of a heavy chain. The antibody also may include the CHI, hinge, CH2, CH3, and CH4 regions of the heavy chain. Alternatively, a humanized antibody may only contain a humanized light chain, or a humanized heavy chain. An exemplary humanized antibody contains a humanized variable domain of a light chain and a humanized variable domain of a heavy chain.

Also, for example, humanized antibodies may be derived from conventional antibodies (i.e. an immunoglobulin molecule comprising 2 heavy (H) chains and 2 light (L) chains interconnected by disulfide bonds) from the family *Camelidae,* in particular from the llama *(e.g., Lama paccos, Lama glama* or *Lama vicugna*), whose variable domains exhibit a high degree of amino acid sequence identity with the variable domains of human antibodies. A suitable platform for the production of such humanized antibodies is the SIMPLE Antibody™ platform (ArGEN-X) as described in WO 2011080350. Accordingly, the humanized antibody may comprise a camelid VH domain and a camelid VL domain of either the lambda light chain class (Vλ) or the kappa light chain class (Vκ), characterised in that at least one amino acid substitution is present in said VH domain and/or said VL domain, said amino acid substitution(s) being selected from the following:
(a) amino acid substitution(s) in said camelid VH domain, wherein an amino acid at one or more positions selected from the group consisting of H1 , H5, H7, H11 , H12, H13, H14, H29, H30, H37, H40, H48, H67, H68, H69, H71 , H74, H77, H78, H80, H81 , H82b, H83, H84, H85, H86, H89, H93, H94 and H108 of the camelid VH domain, according to Kabat, is replaced with an amino acid found at the corresponding position in a human VH domain sequence; and
(b) amino acid substitutions in said camelid VL domain, wherein for a camelid VL domain of the lambda light chain class (Vλ) an amino acid at one or more positions selected from the group consisting of L1 , L2, L3, L7, L8, L9, L11 , L14, L15, L17, L18, L19, L20, L38, L39, L40, L42, L44, L46, L47, L49, L58, L59, L60, L66, L67, L69, L70, L71 , L72, L74, L76, L78, L80, L84 and L103 of the camelid Vλ domain, according to Kabat, is replaced with an amino acid found at the corresponding position in a human Vλ domain sequence; and for a camelid VL domain of the kappa light chain class (Vκ) an amino acid at one or more positions selected from the group consisting of K1, K3, K4, K7, K9, K11 , K12, K13, K14, K15, K18, K19, K36, K42, K43, K45, K46, K58, K63, K70, K77, K78, K79, K80, K83, K100, K103, K104 and K106 of the camelid Vκ domain, according to Kabat, is replaced with an amino acid found at the corresponding position in a human Vκ domain sequence. In particular, for humanisation towards human VH3, VH1, or VH4, one or more of the respective amino acid replacements as described in Table 4 on page 35 of the WO 2011080350 publication may be made, for humanisation towards human Vκ1, Vκ2, Vκ4, Vλ1, Vλ2, Vλ3, Vλ5, or Vλ6, one or more of the respective amino acid replacements as described in Table 5 on pages 38-41 of the WO 2011080350 publication may be made. One or more or any combination of the CH1 domain, hinge region, CH2 domain, CH3 domain, optionally CH4 domain and CL domain in such antibodies may be fully or substantially human with respect to its amino acid sequence.

In certain embodiments, the therapeutic agent may be an intrabody. The term "intrabody" generally refer to an intracellular antibody or antibody fragment. Antibodies, in particular single chain variable antibody fragments (scFv), can be modified for intracellular localization. Such modification may entail for example, the fusion to a stable intracellular protein, such as, e.g., maltose binding protein, or the addition of intracellular trafficking/localization peptide sequences, such as, e.g., the endoplasmic reticulum retention or retrieval signal sequence KDEL.

A skilled person will understand that an antibody can include one or more amino acid deletions, additions and/or substitutions (e.g., conservative substitutions), insofar such alterations preserve its binding of the respective antigen. For example, mutations may be introduced into the antibody, in particular in the Fc region, to extend *in vivo* half-life without compromising immunogenicity as described in US patent 8,323,962.

An antibody may also include one or more native or artificial modifications of its constituent amino acid residues (e.g., glycosylation, etc.).

Methods of producing polyclonal and monoclonal antibodies as well as fragments thereof are well known in the art, as are methods to produce recombinant antibodies or fragments thereof (see for example, Harlow and Lane, "Antibodies: A Laboratory Manual", Cold Spring Harbour Laboratory, New York, 1988; Harlow and Lane, "Using Antibodies: A Laboratory Manual", Cold Spring Harbour Laboratory, New York, 1999, ISBN 0879695447; "Monoclonal Antibodies: A Manual of Techniques", by Zola, ed., CRC Press 1987, ISBN 0849364760; "Monoclonal Antibodies: A Practical Approach", by Dean & Shepherd, eds., Oxford University Press 2000, ISBN 0199637229; Methods in Molecular Biology, vol. 248: "Antibody Engineering: Methods and Protocols", Lo, ed., Humana Press 2004, ISBN 1588290921).

Methods for immunising animals, e.g., non-human animals such as laboratory or farm animals, using immunising antigens (such as, e.g., the herein disclosed CD80 or CD86 polypeptides) optionally fused to or covalently or non-covalently linked, bound or adsorbed to a presenting carrier, and preparation of antibody or cell reagents from immune sera is well-known *per se* and described in documents referred to elsewhere in this specification. The animals to be immunised may include any animal species, preferably warm-blooded species, more preferably vertebrate species, including, e.g., birds and mammals. Without limitation, the antibodies may be chicken, turkey, goose, duck, guinea fowl, quail or pheasant. Also without limitation, the antibodies may be human, murine (e.g., mouse, rat, etc.), porcine, donkey, rabbit, goat, sheep, guinea pig, camel, llama or horse. The term "presenting carrier" or "carrier" generally denotes an immunogenic molecule which, when bound to a second molecule, augments immune responses to the latter, usually through the provision of additional T cell epitopes. The presenting carrier may be a (poly)peptidic structure or a non-peptidic structure, such as inter alia glycans, polyethylene glycols, peptide mimetics, synthetic polymers, etc. Exemplary non-limiting carriers include human Hepatitis B virus core protein, multiple C3d domains, tetanus toxin fragment C or yeast Ty particles. Following immunization, the antibody-producing cells from the animals may be isolated and used to generate monoclonal antibody-producing hybridoma cells using techniques well-know in the art.

Methods for producing recombinant antibodies or fragments thereof need a host organism or cell. The terms "host cell" and "host organism" may suitably refer to cells or organisms encompassing both prokaryotes, such as bacteria, and eukaryotes, such as yeast, fungi, protozoan, plants and animals. Contemplated as host organisms or cells for the production of antibodies include *inter alia* unicellular organisms, such as bacteria *(e.g., E. coli*), and (cultured) animal cells (e.g., mammalian cells or human cells). The advantages of producing antibodies in bacteria are amongst other the relatively safe and straightforward handling of bacterial cells and the rapid replication cycles of microorganisms. Bacteria are particularly suitable for the production of antibody fragments with a simple structure. For the production of full-length immunoglobulins or more complex antibody fragments in prokaryotic cells, the bacterial cells may be transformed with at least two nucleic acids each encoding a different portion of the antibody fragment or the immunoglobulin, e.g., the heavy chain or the light chain, as described in WO 2009021548 for full-length immunoglobulins. In the bacterial cell, the genetic information encoding the antibody is read and translated into a protein. The resulting antibodies accumulate in the periplasmic space and can be harvested upon lysis of the bacterial cells. A further separation step may be performed to purify the antibodies. WO 2009021548 describes an *E*. *Coli*-based secretion system wherein the bacteria release the antibodies in the surrounding culture medium due to the introduction of a signal sequence into the antibody encoding construct. This enables the easy and convenient purification of the antibodies from the cell culture medium. An exemplary mammalian cell line that can be used for the production of antibodies is the Chinese hamster ovary (CHO) cell line. An exemplary human cell line suitable for the production of antibodies includes the PER.C6® cell line as deposited under ECAC no. 96022940 and described in WO 2000063403 or a derivative thereof. Human cell lines are particularly suitable for the production of fully human antibodies because they produce antibodies with a human glycosylation pattern.

Unless indicated otherwise, all methods, steps, techniques and manipulations that are not specifically described in detail can be performed and have been performed in a manner known *per se,* as will be clear to the skilled person. Reference is for example again made to standard handbooks as well as to the general background art referred to herein and to the further references cited therein.

In certain embodiments, the antibody may be a polyclonal antibody, a monoclonal antibody, a chimeric antibody, a humanized antibody, a primatized antibody, a human antibody, a Nanobody, or mixtures thereof.

The term "soluble receptor" generally refers to the soluble (i.e., circulating, not bound to a cell) form of a cell-surface molecule, e.g., a cell-surface receptor, or a fragment or derivative thereof. For example, a cell-surface molecule can be made soluble by attaching a soluble fusion partner, e.g., an immunoglobulin (Ig) moiety, or a portion thereof, to the extracellular domain, or by removing its transmembrane domain.

Particularly intended herein are "soluble CTLA-4 molecules" which denote non-cell-surface-bound (i.e., circulating) CTLA-4 molecules or any functional fragment of a CTLA-4 molecule that binds CD80 and/or CD86 including, but not limited to: CTLA4Ig fusion proteins (e.g., encoded by DNA deposited with ATCC accession number 68629), wherein the extracellular domain of CTLA-4 is fused to an immunoglobulin (Ig) moiety, such as IgCgammal, IgCgamma2, IgCgamma3, IgCgamma4, IgCmu, IgCalphal, IgCalpha2, IgCdelta or IgCepsilon, or fragments and derivatives thereof; proteins with the extracellular domain of CTLA-4 fused or joined with a portion of a biologically active or chemically active protein, such as the papillomavirus E7 gene product (CTLA4-E7), melanoma-associated antigen p97 (CTLA4-p97) or HIV env protein (CTLA4-env gp120) (as described in US Patent No. 5,844,095), or fragments and derivatives thereof; hybrid (chimeric) fusion proteins such as CD28/CTLA4Ig (as described in US Patent No. 5,434,131), or fragments and derivatives thereof; CTLA-4 molecules with the transmembrane domain removed (Oaks, M. K. , et al., 2000. Cellular Immunology 201:144-153), or fragments and derivatives thereof. Examples of CTLA4Ig molecules are described inter alia in WO2005/016266. "Soluble CTLA-4 molecules" also include fragments, portions or derivatives thereof, and soluble CTLA-4 mutant molecules having CTLA-4 binding activity (e.g., L104EA29YIg). "L104EA29YIg" is a fusion protein that is a soluble CTLA4 mutant molecule comprising an extracellular domain of wild-type CTLA4 with amino acid changes A29Y (a tyrosine amino acid residue substituting for an alanine at position 29) and L104E (a glutamic acid amino acid residue substituting for a leucine at position +104), or a portion thereof that binds a B7 molecule, joined to an Ig tail (DNA encoding L104EA29YIg was deposited on June 20,2000 with ATCC number PTA-2104).

Particular examples of soluble CTLA-4 molecules include molecules composed of the Fc region of the immunoglobulin IgG1 fused to the extracellular domain of CTLA-4, such as Abacept (marketed as Orencia®, Bristol Myers Squibb), Belatacept (marketed as Nulojix®, Bristol Myers Squibb), and XPro9523 (Xencor).

For example, CTLA4-KDEL fusion protein as described in Tan et al. (2005. Blood 106:2936-2943) comprises the extracellular domain of CTLA-4 fused to the endoplasmic reticulum retention or retrieval signal sequence, KDEL. The CTLA4-KDEL fusion protein may bind to newly synthesized CD80/86 molecules in the ER and prevent them form reaching the cell surface.

In certain embodiments, the soluble receptor may be a soluble CTLA4 molecule, preferably a CTLA4Ig fusion protein.

The term "aptamer" refers to single-stranded or double-stranded oligo-DNA, oligo-RNA or oligo-DNA/RNA or any analogue thereof that can specifically bind to a target molecule. Advantageously, aptamers can display fairly high specificity and affinity (e.g., K_{A} in the order 1×10⁹ M⁻¹) for their targets. Aptamer production is described inter alia in US 5,270,163; Ellington & Szostak 1990 (Nature 346: 818-822); Tuerk & Gold 1990 (Science 249: 505-510); or "The Aptamer Handbook: Functional Oligonucleotides and Their Applications", by Klussmann, ed., Wiley-VCH 2006, ISBN 3527310592, incorporated by reference herein. The term also encompasses photoaptamers, i.e., aptamers that contain one or more photoreactive functional groups that can covalently bind to or crosslink with a target molecule.

In certain embodiments, the therapeutic agent as disclosed herein capable of preventing or inhibiting the expression of at least one of the CD80 or CD86 polypeptides by a cell or capable of preventing or inhibiting the presentation of at least one of the CD80 or CD86 polypeptides on the surface of a cell may be selected from the group comprising, consisting essentially of or consisting of an intrabody or a fragment or derivative thereof, an antisense agent, and an RNA interference (RNAi) agent. Particularly intended may be such antisense agents capable of binding to (annealing with) a sequence region in *CD80* or *CD86* pre-mRNA or mRNA sequence. Particularly intended may be such RNAi agents configured to target *CD80* or *CD86* mRNA.

In some embodiments, the therapeutic agent may thus be an intrabody directed to the CD80 and/or CD86 polypeptides, which may bind these molecules intracellularly and prevent them from reaching the cell surface.

The term "antisense" generally refers to an agent (e.g., an oligonucleotide) configured to specifically anneal with (hybridise to) a given sequence in a target nucleic acid, such as for example in a target DNA, hnRNA, pre-mRNA or mRNA, and typically comprises, consists essentially of or consists of a nucleic acid sequence that is complementary or substantially complementary to said target nucleic acid sequence. Antisense agents suitable for use herein may typically be capable of annealing with (hybridising to) the respective target nucleic acid sequences at high stringency conditions, and capable of hybridising specifically to the target under physiological conditions.

The terms "complementary" or "complementarity" as used herein with reference to nucleic acids, refer to the normal binding of single-stranded nucleic acids under permissive salt (ionic strength) and temperature conditions by base pairing, preferably Watson-Crick base pairing. By means of example, complementary Watson-Crick base pairing occurs between the bases A and T, A and U or G and C. For example, the sequence 5'-A-G-U-3' is complementary to sequence 5'-A-C-U-3'.

The term "bind" or "binding" as used herein preferably refers to specific binding, i.e., where an agent binds to (anneals with) one or more targets of interest, such as to one or more pre-mRNA molecules or fragments or variants thereof, substantially to the exclusion of other molecules which are random or unrelated, and optionally substantially to the exclusion of other molecules that are structurally related. Binding of an agent to a target may be evaluated *inter alia* using conventional interaction-querying methods, such as *in silico* sequence analysis or nucleic acid hybridisation experiments, e.g., to verify specific hybridisation, e.g., under high stringency conditions.

Specific binding does not necessarily require that an agent binds exclusively to its intended target(s). For example, an agent may be said to specifically bind to a given pre-mRNA of interest or fragments or variants thereof if its affinity for such intended target(s) under the conditions of binding is at least about 2-fold greater, preferably at least about 5-fold greater, more preferably at least about 10-fold greater, yet more preferably at least about 25-fold greater, still more preferably at least about 50-fold greater, and even more preferably at least about 100-fold or at least about 1000-fold or more greater, than its affinity for a non-target molecule.

The sequence of an antisense agent need not be 100% complementary to that of its target sequence to bind or hybridise specifically with the latter. An antisense agent may be said to be specifically hybridisable when binding of the agent to a target nucleic acid molecule interferes with the normal function of the target nucleic acid such as to attain an intended outcome (e.g., loss of utility), and there is a sufficient degree of complementarity to avoid non-specific binding of the antisense agent to non-target sequences under conditions in which specific binding is desired, i.e., under physiological conditions in the case of *in vivo* assays or therapeutic treatment, and in the case of *in vitro* assays, under conditions in which the assays are performed. Thus, "specifically hybridisable" and "complementary" may indicate a sufficient degree of complementarity or precise pairing such that stable and specific binding occurs between an antisense agent and a nucleic acid target.

Preferably, to ensure specificity of antisense agents towards the desired target over unrelated molecules, the sequence of said antisense agents may be at least about 80% complementary, preferably at least about 90% complementary, more preferably at least about 95% complementary, such as, e.g., about 96%, about 97%, about 98%, about 99% and up to 100% complementary to the respective target sequence.

Antisense agents as intended herein preferably comprise or denote antisense molecules such as more preferably antisense nucleic acid molecules or antisense nucleic acid analogue molecules. Preferably, antisense agents may refer to antisense oligonucleotides or antisense oligonucleotide analogues.

The term "oligonucleotide" as used herein refers to a nucleic acid (including nucleic acid analogues and mimetics) oligomer or polymer as defined herein. Preferably, an oligonucleotide, such as more particularly an antisense oligonucleotide, is (substantially) single-stranded. Oligonucleotides as intended herein may be preferably between about 10 and about 100 nucleoside units (i.e., nucleotides or nucleotide analogues) in length, preferably between about 15 and about 50, more preferably between about 20 and about 40, also preferably between about 20 and about 30. Preferably, oligonucleotides as intended herein may comprise one or more or all non-naturally occurring heterocyclic bases and/or one or more or all non-naturally occurring sugar groups and/or one or more or all non-naturally occurring inter-nucleoside linkages, the inclusion of which may improve properties such as, for example, enhanced cellular uptake, increased stability in the presence of nucleases and increased hybridization affinity, increased tolerance for mismatches, *etc.* Further, oligonucleotides as intended herein may be configured to not activate RNAse H, in accordance with known techniques (see, e.g., U.S. Pat. 5,149,797).

Antisense agents such as oligonucleotides as taught herein may be further conjugated (e.g., covalently or non-covalently, directly or via a suitable linker) to one or more moieties or conjugates that enhance the activity, cellular distribution or cellular uptake of the oligonucleotide. Such moieties include but are not limited to lipid moieties such as a cholesterol moiety, cholic acid, a thioether, e.g., hexyl-S-tritylthiol, a thiocholesterol, an aliphatic chain, e.g., dodecandiol or undecyl residues, a phospholipid, e.g., di-hexadecyl-rac-glycerol or triethylammonium 1,2-di-O-hexadecyl-rac-glycero-3-H-phosphonate, a polyamine or a polyethylene glycol chain, or adamantane acetic acid, a palmityl moiety, or an octadecylamine or hexylamino-carbonyl- oxycholesterol moiety.

It is not necessary for all positions in a given agent to be uniformly modified, and in fact more than one of the aforementioned modifications may be incorporated in a single agent or even at a single nucleoside within an oligonucleotide. Further included are antisense compounds that are chimeric compounds. "Chimeric" antisense compounds or "chimeras" are antisense molecules, particularly oligonucleotides, which contain two or more chemically distinct regions, each made up of at least one monomer unit, i.e., a nucleotide in the case of an oligonucleotide compound. These oligonucleotides typically contain at least one region wherein the oligonucleotide is modified so as to confer upon the increased resistance to nuclease degradation, increased cellular uptake, and an additional region for increased binding affinity for the target nucleic acid.

An RNAi (RNA interference) agent typically comprises, consists essentially of or consists of a double-stranded portion or region (notwithstanding the optional and potentially preferred presence of single-stranded overhangs) of annealed complementary strands, one of which (sense strand) has a sequence corresponding to a target nucleotide sequence (hence, to at least a portion of an mRNA) of the target gene to be down-regulated. The other strand (antisense strand) of the RNAi agent is complementary to said target nucleotide sequence.

Whereas the sequence of an RNAi agent, such as more particularly of a sense strand thereof, need not be completely identical to a target sequence to be down-regulated, the number of mismatches between a target sequence and a nucleotide sequence of the RNAi agent is preferably no more than 1 in 5 bases, or 1 in 10 bases, or 1 in 20 bases, or 1 in 50 bases.

Preferably, to ensure specificity of RNAi agents towards the desired target over unrelated molecules, the sequence of said RNAi agents, such as more particularly of a sense strand thereof, may be at least about 80% identical, preferably at least about 90% identical, more preferably at least about 95% identical, such as, e.g., about 96%, about 97%, about 98%, about 99% and up to 100% identical to the respective target sequence.

An RNAi agent may be formed by separate sense and antisense strands or, alternatively, by a common strand providing for fold-back stem-loop or hairpin design where the two annealed strands of an RNAi agent are covalently linked.

An siRNA molecule may be typically produced, e.g., synthesised, as a double stranded molecule of separate, substantially complementary strands, wherein each strand is about 18 to about 35 bases long, preferably about 19 to about 30 bases, more preferably about 20 to about 25 bases and even more preferably about 21 to about 23 bases.

shRNA is in the form of a hairpin structure. shRNA can be synthesized exogenously or can be formed by transcribing from RNA polymerase III promoters *in vivo.* Preferably, shRNAs can be engineered in host cells or organisms to ensure continuous and stable suppression of a desired gene. It is known that siRNA can be produced by processing a hairpin RNA in cells.

RNAi agents as intended herein may include any modifications as set out herein for nucleic acids and oligonucleotides, in order to improve their therapeutic properties.

In embodiments, at least one strand of an RNAi molecules may have a 3' overhang from about 1 to about 6 bases in length, e.g., from 2 to 4 bases, more preferably from 1 to 3 bases. For example, one strand may have a 3' overhang and the other strand may be either blunt-ended or may also have a 3'overhang. The length of the overhangs may be the same or different for each strand. The 3' overhangs can be stabilised against degradation. For example, the RNA may be stabilised by including purine nucleotides, such as A or G nucleotides. Alternatively, substitution of pyrimidine nucleotides by modified analogues, e.g., substitution of U 3' overhangs by 2'-deoxythymidine is tolerated and does not affect the efficiency of RNAi.

An exemplary but non-limiting siRNA molecule may by characterized by any one or more, and preferably by all of the following criteria:
- at least about 80% sequence identity, such as more particularly sequence identity of a sense strand of the siRNA molecule, to target mRNA, more preferably at least about 90 % or at least about 95% or at least about 97% sequence identity to target mRNA;
- having a sequence which targets an area of the target gene present in mature mRNA (e.g., an exon or alternatively spliced intron);
- showing a preference for targeting the 3' end of the target gene.

The exemplary siRNA may be further characterised by one or more or all of the following criteria:
- having a double-stranded nucleic acid length of between 16 to 30 bases and preferably of between 18 to 23 bases, and preferably of 19 nucleotides;
- having GC content between about 30 and about 50 %;
- having a TT(T) sequence at 3' end;
- showing no secondary structure when adopting the duplex form;
- having a Tm (melting temperature) of lower than 20°C;
- having the nucleotides indicated here below in the sequence of the nucleotides, wherein "h" is A, C, T/U but not G; wherein "d" is A, G, T/U but not C, and wherein "w" is A or T/U, but not GorC:

| | | - | - | 1 | 2 | 3 | 4 | 5 | 6 | 7 | 8 | 9 | 10 | 11 | 12 | 13 | 14 | 15 | 16 | 17 | 18 | 19 | - | - | |
|---|---|---|---|---|---|---|---|---|---|---|---|---|---|---|---|---|---|---|---|---|---|---|---|---|---|
| mRNA | P'5 | A | A | | | A | | | | | | | U | | | h | | | | | | w | | | 3'-OH |
| si-ASense | OH-3' | T | T | | | U | | | | | | | A | | | d | | | | | | w | | | 5'-P |
| si-Sense | P-5' | | | | | A | | | | | | | U | | | h | | | | | | w | T | T | 3'-OH |

RNAi agents as intended herein may particularly comprise or denote (i.e., may be selected from a group comprising or consisting of) RNAi nucleic acid molecules or RNAi nucleic acid analogue molecules, such as preferably short interfering nucleic acids and short interfering nucleic acid analogues (siNA) such as short interfering RNA and short interfering RNA analogues (siRNA), and may further denote *inter alia* double-stranded RNA and double-stranded RNA analogues (dsRNA), micro-RNA and micro-RNA analogues (miRNA), and short hairpin RNA and short hairpin RNA analogues (shRNA).

Production of antisense agents and RNAi agents can be carried out by any processes known in the art, such as *inter alia* partly or entirely by chemical synthesis (e.g., routinely known solid phase synthesis; an exemplary and non-limiting method for synthesising oligonucleotides on a modified solid support is described in US 4,458,066; in another example, diethyl-phosphoramidites are used as starting materials and may be synthesised as described by Beaucage et al. 1981 (Tetrahedron Letters 22: 1859-1862)), or partly or entirely by biochemical (enzymatic) synthesis, *e.g.,* by *in vitro* transcription from a nucleic acid construct (template) using a suitable polymerase such as a T7 or SP6 RNA polymerase, or by recombinant nucleic acid techniques, *e.g.,* expression from a vector in a host cell or host organism. Nucleotide analogues can be introduced by *in vitro* chemical or biochemical synthesis. In an embodiment, the antisense agents of the invention are synthesised *in vitro* and do not include antisense compositions of biological origin, or genetic vector constructs designed to direct the *in vivo* synthesis of antisense molecules.

In certain embodiments, the therapeutic agent as intended herein capable of interacting with at least one of the CD80 or CD86 polypeptides or capable of preventing or inhibiting the expression of at least one of the CD80 or CD86 polypeptides by a cell or capable of preventing or inhibiting the presentation of at least one of the CD80 or CD86 polypeptides on the surface of a cell may be a composition comprising a therapeutic agent capable of interacting with at least the CD80 polypeptide or capable of preventing or inhibiting the expression of at least the CD80 polypeptide by a cell or capable of preventing or inhibiting the presentation of at least the CD80 polypeptide on the surface of a cell; and a therapeutic agent capable of interacting with at least the CD86 polypeptide or capable of preventing or inhibiting the expression of at least the CD86 polypeptide by a cell or capable of preventing or inhibiting the presentation of at least the CD86 polypeptide on the surface of a cell.

Preferably, the composition comprises a therapeutic agent capable of interacting with at least the CD80 polypeptide and a therapeutic agent capable of interacting with at least the CD86 polypeptide; or a therapeutic agent capable of preventing or inhibiting the expression of at least the CD80 polypeptide by a cell and a therapeutic agent capable of preventing or inhibiting the expression of at least the CD86 polypeptide by a cell; or a therapeutic agent capable of preventing or inhibiting the presentation of at least the CD80 polypeptide on the surface of a cell and a therapeutic agent capable of preventing or inhibiting the presentation of at least the CD86 polypeptide on the surface of a cell.

Also disclosed herein is a composition capable of interacting with at least the CD80 or CD86 polypeptides comprising a therapeutic agent capable of interacting with at least the CD80 polypeptide and a therapeutic agent capable of interacting with at least the CD86 polypeptide, for use in the treatment of cardiac warm ischemia reperfusion injury. Also disclosed herein is a composition capable of preventing or inhibiting the expression of at least the CD80 or CD86 polypeptides by a cell comprising a therapeutic agent capable of preventing or inhibiting the expression of at least the CD80 polypeptide by a cell and a therapeutic agent capable of preventing or inhibiting the expression of at least the CD86 polypeptide by a cell, for use in the treatment of cardiac warm ischemia reperfusion injury. Also disclosed herein is a composition capable of preventing or inhibiting the presentation of at least one of the CD80 or CD86 polypeptides on the surface of a cell comprising a therapeutic agent capable of preventing or inhibiting the presentation of at least the CD80 polypeptide on the surface of a cell and a therapeutic agent capable of preventing or inhibiting the presentation of at least the CD86 polypeptide on the surface of a cell, for use in the treatment of cardiac warm ischemia reperfusion injury.

In certain embodiments, the therapeutic agent as intended herein capable of interacting with at least one of the CD80 or CD86 polypeptides or capable of preventing or inhibiting the expression of at least one of the CD80 or CD86 polypeptides by a cell or capable of preventing or inhibiting the presentation of at least one of the CD80 or CD86 polypeptides on the surface of a cell may be co-administered with a second therapeutic agent, wherein the second therapeutic agent is capable of interacting with the CD40 polypeptide, and wherein said interaction interferes with the binding of the CD40 polypeptide to the CD40 ligand (CD40L) polypeptide.

Preferably, the therapeutic agent as intended herein capable of interacting with at least one of the CD80 or CD86 polypeptides may be co-administered with the second therapeutic agent, wherein the second therapeutic agent is capable of interacting with the CD40 polypeptide, and wherein said interaction interferes with the binding of the CD40 polypeptide to the CD40 ligand (CD40L) polypeptide.

The therapeutic agent and the second therapeutic agent may be provided as a combined preparation for simultaneous, separate or sequential (in any order) administration. Preferably, the therapeutic agent and the second therapeutic agent may be administered simultaneously. For example, the therapeutic agent and the second therapeutic agent may be comprised in a composition, preferably a pharmaceutical composition, which is administered to the subject. Accordingly, also disclosed herein is a pharmaceutical combination or composition for simultaneous, separate or sequential (in any order) use in the treatment of cardiac warm ischemia reperfusion injury, wherein said composition comprises the therapeutic agent and the second therapeutic agent as taught herein.

Also provided is therefore a method for treating cardiac warm ischemia reperfusion injury in a subject in need of such treatment, comprising administering to said subject a therapeutically effective amount of a therapeutic agent, wherein the agent is capable of interacting with at least one of the CD80 or CD86 polypeptides or capable of preventing or inhibiting the expression of at least one of the CD80 or CD86 polypeptides by a cell or capable of preventing or inhibiting the presentation of at least one of the CD80 or CD86 polypeptides on the surface of a cell, and a therapeutically effective amount of a second therapeutic agent, wherein the second therapeutic agent is capable of interacting with the CD40 polypeptide, and wherein said interaction interferes with the binding of the CD40 polypeptide to the CD40L polypeptide.

Also provided is the use of a therapeutic agent, wherein the agent is capable of interacting with at least one of the CD80 or CD86 polypeptides or capable of preventing or inhibiting the expression of at least one of the CD80 or CD86 polypeptides by a cell or capable of preventing or inhibiting the presentation of at least one of the CD80 or CD86 polypeptides on the surface of a cell, and a second therapeutic agent, wherein the second therapeutic agent is capable of interacting with the CD40 polypeptide, and wherein said interaction interferes with the binding of the CD40 polypeptide to the CD40L polypeptide, for the manufacture of a combined preparation for simultaneous, separate or sequential (in any order) use in the treatment of cardiac warm ischemia reperfusion injury.

In certain embodiments, the therapeutic agent as intended herein capable of interacting with at least one of the CD80 or CD86 polypeptides or capable of preventing or inhibiting the expression of at least one of the CD80 or CD86 polypeptides by a cell or capable of preventing or inhibiting the presentation of at least one of the CD80 or CD86 polypeptides on the surface of a cell may be co-administered with a second therapeutic agent, capable of preventing or inhibiting the expression of the CD40 polypeptide by a cell or capable of preventing or inhibiting the presentation of the CD40 polypeptide on the surface of a cell.

Preferably, the therapeutic agent as intended herein capable of preventing or inhibiting the expression of at least one of the CD80 or CD86 polypeptides by a cell or capable of preventing or inhibiting the presentation of at least one of the CD80 or CD86 polypeptides on the surface of a cell may be co-administered with a second therapeutic agent, capable of preventing or inhibiting the expression of the CD40 polypeptide by a cell or capable of preventing or inhibiting the presentation of the CD40 polypeptide on the surface of a cell.

The therapeutic agent and the second therapeutic agent may be provided as a combined preparation for simultaneous, separate or sequential (in any order) administration. Preferably, the therapeutic agent and the second therapeutic agent may be administered simultaneously. For example, the therapeutic agent and the second therapeutic agent may be comprised in a composition, preferably a pharmaceutical composition, which is administered to the subject. Accordingly, also disclosed herein is a pharmaceutical combination or composition for use in the treatment of cardiac warm ischemia reperfusion injury, wherein said composition comprises the therapeutic agent and the second therapeutic agent as taught herein.

Also provided is therefore a method for treating cardiac warm ischemia reperfusion injury in a subject in need of such treatment, comprising administering to said subject a therapeutically effective amount of a therapeutic agent, wherein the agent is capable of interacting with at least one of the CD80 or CD86 polypeptides or capable of preventing or inhibiting the expression of at least one of the CD80 or CD86 polypeptides by a cell or capable of preventing or inhibiting the presentation of at least one of the CD80 or CD86 polypeptides on the surface of a cell, and a therapeutically effective amount of a second therapeutic agent, wherein the second therapeutic agent is capable of preventing or inhibiting the expression of the CD40 polypeptide by a cell or capable of preventing or inhibiting the presentation of the CD40 polypeptide on the surface of a cell.

Also provided is the use of a therapeutic agent, wherein the agent is capable of interacting with at least one of the CD80 or CD86 polypeptides or capable of preventing or inhibiting the expression of at least one of the CD80 or CD86 polypeptides by a cell or capable of preventing or inhibiting the presentation of at least one of the CD80 or CD86 polypeptides on the surface of a cell, and a second therapeutic agent, wherein the second therapeutic agent is capable of preventing or inhibiting the expression of the CD40 polypeptide by a cell or capable of preventing or inhibiting the presentation of the CD40 polypeptide on the surface of a cell, for the manufacture of a combined preparation for simultaneous, separate or sequential (in any order) use in the treatment of cardiac warm ischemia reperfusion injury.

In further embodiments, the CD40 polypeptide may be comprised by a cell and the CD40L polypeptide may be comprised by another cell.

In particular, the CD40 polypeptide may be comprised by an antigen presenting cell (APC) and the CD40L polypeptide may be comprised by a T-cell, whereby the action of the second therapeutic agent as disclosed herein capable of interacting with the CD40 polypeptide will interfere with the co-stimulatory pathway CD40:CD40L, contributing in an additive or synergistic fashion to the inhibition the T cell responses.

In further embodiments, the CD40 polypeptide may be comprised by an antigen presenting cell (APC), whereby the action of the second therapeutic agent as disclosed herein capable of preventing or inhibiting the expression of the CD40 polypeptide by the cell or capable of preventing or inhibiting the presentation of the CD40 polypeptide on the surface of the cell will interfere with the co-stimulatory pathway CD40:CD40L, contributing in an additive or synergistic fashion to the inhibition the T cell responses.

In certain embodiments, the second therapeutic agent as intended herein may be selected from the group comprising, consisting essentially of, or consisting of a protein, a polypeptide, a peptide, a peptidomimetic, a nucleic acid, a small organic molecule, and a compound or combination of any two or more thereof, as defined elsewhere in this specification.

In certain embodiments, the second therapeutic agent as disclosed herein, particularly the second therapeutic agent capable of interacting with the CD40 polypeptide may be selected from the group comprising, consisting essentially of or consisting of an antibody or a fragment or derivative thereof, a soluble receptor, and an aptamer, as defined elsewhere in this specification.

In certain embodiments, the antibody may be a polyclonal antibody, a monoclonal antibody, a chimeric antibody, a humanized antibody, a primatized antibody, a human antibody, a Nanobody, or mixtures thereof.

Suitable anti-CD40 antibodies include, for example, XmAb® 5485 (Xencor), PG102 (PanGenetics UK Limited, WO2007/129895), Dacetuzumab (SGN-40, Seattle Genetics, Inc./ Genentec), HCD122 (Novartis), ASKP1240 (Astellas Pharma), 5D12 or functional equivalents thereof (DeBoer et al. 1992, J. Immunol. Methods 152:15-23), and FFP106, FFP104, and FFP102 (FFPharma).

In particular embodiments, the anti-CD40 antibody may an antibody as defined in any one of claims 17 to 22 of WO2007/129895. More particularly, the anti-CD40 antibody may comprise a polypeptide comprising an amino acid sequence:

FSX₁SRYSVYWX₂RQPPGKGX₃EWX4GMMWGGGSTDYSTSLKSRLTISKDTSKSQVX₅LKMNS LRTDDTAMYYCVRTDGDY (SEQ ID NO:1), preferably

QVKLQESGPGLVKPSETLSITCTVSGFSX₁SRYSVYWX₂RQPPGKGX₃EWX₄GMMWGGGSTDY STSLKSRLTISKDTSKSQVX₅LKMNSLRTDDTAMYYCVRTDGDYWGQGTTVTVSS (SEQ ID NO:2), wherein:
X₁ is G, A, V, L, I, P, F, M, W, C, N, Q, S, T, Y, D, E, K, R or H, preferably G, A, V, L, P, F, M, W, C, N, Q, S, T, Y, D, E, K, R or H;
X₂ is G, A, V, L, I, P, F, M, W, C, N, Q, S, T, Y, D, E, K, R or H;
X₃ is G, A, V, L, I, P, F, M, W, C, N, Q, S, T, Y, D, E, K, R or H;
X₄ is G, A, V, L, I, P, F, M, W, C, N, Q, S, T, Y, D, E, K, R or H; and
X₅ is G, A, V, L, I, P, F, M, W, C, N, Q, S, T, Y, D, E, K, R or H;
and further comprising an amino acid sequence:
X₆LGX₇X₈ASISCRSSQSLX₉NSNGNTYLHWYLQRPGQSPRLLIYKVSNRFSGVPDRFSGSGSGTD FTLKISRV EAEDX₁₀GVYX₁₁CSQSTHVPWT (SEQ ID NO:3), preferably
ELQLTQSPLSLPVX₆LGX₇XgASISCRSSQSLX₉NSNGNTYLHWYLQRPGQSPRLLIYKVSNRFSG VPDRFSGSGSGTDFTLKISRVEAEDX₁₀GVYX₁₁CSQSTHVPWTFGGGTKLEIKR (SEQ ID NO:4),
wherein:
X₆ is N, Q, S, T, Y, W or C;
X₇ is D, E, N, Q, S, T, Y, W or C;
X₈ is N, Q, S, T, Y, G, A, V, L, I, P, F, M, W or C;
X₉ is G, A, V, L, I, P, F, M;
X₁₀ is G, A, V, L, I, P, F, M; and
X₁₁ is N, Q, S, T, Y, G, A, V, L, I, P, F, M, W or C.

For example, X₁ may be L; X2 may be V; X3 may be L; X4 may be L and X5 may be F. In further examples, X6 may be T or S, X7 may be D or Q, X8 may be Q or P, X9 may be V or A, X10 may be V or L and X11 may be F or Y, preferably X6 may be T, X7 may be Q, X8 may be P, X9 may be A, X10 may be V and X11 may be Y.

In certain embodiments, the second therapeutic agent as disclosed herein capable of preventing or inhibiting the expression of the CD40 polypeptide by a cell or capable of preventing or inhibiting the presentation of the CD40 polypeptide on the surface of a cell may be selected from the group comprising, consisting essentially of or consisting of an intrabody or a fragment or derivative thereof, an antisense agent, and an RNA interference (RNAi) agent, as defined elsewhere in this specification. Particularly intended may be such antisense agents capable of binding to (annealing with) a sequence region in *CD40* pre-mRNA or mRNA sequence. Particularly intended may be such RNAi agents configured to target *CD40* mRNA.

Where the therapeutic agent or the second therapeutic agent is an expressible molecule such as an antibody or a fragment or derivative thereof, a protein or polypeptide, a peptide, a nucleic acid, an antisense agent or an RNAi agent, it shall be understood that the agent itself may be introduced to a subject or may be introduced by means of a recombinant nucleic acid comprising a sequence encoding the agonist operably linked to one or more regulatory sequences allowing for expression of said sequence encoding the agent (e.g., gene therapy or cell therapy).

Hence, an agent may comprise a recombinant nucleic acid comprising a sequence encoding one or more desired proteins, polypeptides, peptides, antisense agents or RNAi agents, operably linked to one or more regulatory sequences allowing for expression of said sequence or sequences encoding the proteins, polypeptides, peptides, antisense agents or RNAi agents, e.g., *in vitro,* in a host cell, host organ and/or host organism (expression constructs). Such recombinant nucleic acid may be comprised in a suitable vector.

By "encoding" is meant that a nucleic acid sequence or part(s) thereof corresponds, by virtue of the genetic code of an organism in question to a particular amino acid sequence, e.g., the amino acid sequence of one or more desired proteins or polypeptides, or to another nucleic acid sequence in a template-transcription product (e.g. RNA or RNA analogue) relationship.

Preferably, a nucleic acid encoding one or more proteins, polypeptides or peptides may comprise one or more open reading frames (ORF) encoding said one or more proteins, polypeptides or peptides. An "open reading frame" or "ORF" refers to a succession of coding nucleotide triplets (codons) starting with a translation initiation codon and closing with a translation termination codon known *per se,* and not containing any internal in-frame translation termination codon, and potentially capable of encoding a protein, polypeptide or peptide. Hence, the term may be synonymous with "coding sequence" as used in the art.

An "operable linkage" is a linkage in which regulatory sequences and sequences sought to be expressed are connected in such a way as to permit said expression. For example, sequences, such as, e.g., a promoter and an ORF, may be said to be operably linked if the nature of the linkage between said sequences does not: (1) result in the introduction of a frame-shift mutation, (2) interfere with the ability of the promoter to direct the transcription of the ORF, (3) interfere with the ability of the ORF to be transcribed from the promoter sequence.

The precise nature of regulatory sequences or elements required for expression may vary between expression environments, but typically include a promoter and a transcription terminator, and optionally an enhancer.

Reference to a "promoter" or "enhancer" is to be taken in its broadest context and includes transcriptional regulatory sequences required for accurate transcription initiation and where applicable accurate spatial and/or temporal control of gene expression or its response to, e.g., internal or external (e.g., exogenous) stimuli. More particularly, "promoter" may depict a region on a nucleic acid molecule, preferably DNA molecule, to which an RNA polymerase binds and initiates transcription. A promoter is preferably, but not necessarily, positioned upstream, *i.e.,* 5', of the sequence the transcription of which it controls. Typically, in prokaryotes a promoter region may contain both the promoter *per se* and sequences which, when transcribed into RNA, will signal the initiation of protein synthesis (e.g., Shine-Dalgarno sequence).

In embodiments, promoters contemplated herein may be constitutive or inducible.

The terms "terminator" or "transcription terminator" refer generally to a sequence element at the end of a transcriptional unit which signals termination of transcription. For example, a terminator is usually positioned downstream of, *i.e.,* 3' of ORF(s) encoding a polypeptide of interest. For instance, where a recombinant nucleic acid contains two or more ORFs, e.g., successively ordered and forming together a multi-cistronic transcription unit, a transcription terminator may be advantageously positioned 3' to the most downstream ORF.

The term "vector" generally refers to a nucleic acid molecule, typically DNA, to which nucleic acid segments may be inserted and cloned, i.e., propagated. Hence, a vector will typically contain one or more unique restriction sites, and may be capable of autonomous replication in a defined host or vehicle organism such that the cloned sequence is reproducible. Vectors may include, without limitation, plasmids, phagemids, bacteriophages, bacteriophage-derived vectors, PAC, BAC, linear nucleic acids, e.g., linear DNA, viral vectors, *etc.,* as appropriate. Expression vectors are generally configured to allow for and/or effect the expression of nucleic acids or ORFs introduced thereto in a desired expression system, *e.g., in vitro,* in a host cell, host organ and/or host organism. For example, expression vectors may advantageously comprise suitable regulatory sequences.

As noted elsewhere, an agent may comprise a protein, polypeptide or peptide. Such may be suitably obtained through expression by host cells or host organisms, transformed with an expression construct encoding and configured for expression of said protein, polypeptide or peptide in said host cells or host organisms, followed by purification of the protein, polypeptide or peptide.

The terms "host cell" and "host organism" may suitably refer to cells or organisms encompassing both prokaryotes, such as bacteria, and eukaryotes, such as yeast, fungi, protozoan, plants and animals. Contemplated as host cells are *inter alia* unicellular organisms, such as bacteria *(e.g., E. coli, Salmonella tymphimurium, Serratia marcescens,* or *Bacillus subtilis),* yeast *(e.g., Saccharomyces cerevisiae* or *Pichia pastoris),* (cultured) plant cells *(e.g.,* from *Arabidopsis thaliana* or *Nicotiana tobaccum*) and (cultured) animal cells (e.g., vertebrate animal cells, mammalian cells, primate cells, human cells or insect cells). Contemplated as host organisms are *inter alia* multi-cellular organisms, such as plants and animals, preferably animals, more preferably warm-blooded animals, even more preferably vertebrate animals, still more preferably mammals, yet more preferably primates; particularly contemplated are such animals and animal categories which are non-human.

Such protein, polypeptide or peptide may be suitably isolated. The term "isolated" with reference to a particular component (such as for instance a nucleic acid, protein, polypeptide or peptide) generally denotes that such component exists in separation from - for example, has been separated from or prepared and/or maintained in separation from - one or more other components of its natural environment. For instance, an isolated human or animal protein or complex may exist in separation from a human or animal body where it naturally occurs. The term "isolated" as used herein may preferably also encompass the qualifier "purified". By means of example, the term "purified" with reference to nucleic acids, proteins, polypeptides or peptides does not require absolute purity. Instead, it denotes that such nucleic acids, proteins, polypeptides or peptides are in a discrete environment in which their abundance (conveniently expressed in terms of mass or weight or concentration) relative to other nucleic acids, proteins, polypeptides or peptides is greater than in a biological sample. A discrete environment denotes a single medium, such as for example a single solution, gel, precipitate, lyophilisate, etc. Purified nucleic acids, proteins, polypeptides or peptides may be obtained by known methods including, for example, laboratory or recombinant synthesis, chromatography, preparative electrophoresis, centrifugation, precipitation, affinity purification, etc.

The various therapeutic agents of the present disclosure or pharmaceutically acceptable derivatives thereof, such as pharmaceutically acceptable salts thereof, which may be collectively denoted as active substances for convenience, may be formulated into and administered as pharmaceutical compositions or formulations with one or more pharmaceutically acceptable carriers/excipients. Such pharmaceutical compositions or formulations may be comprised in a kit of parts.

The term "pharmaceutically acceptable" as used herein is consistent with the art and means compatible with the other ingredients of a pharmaceutical composition and not deleterious to the recipient thereof.

As used herein, "carrier" or "excipient" includes any and all solvents, diluents, buffers (such as, e.g., neutral buffered saline, phosphate buffered saline, or optionally Tris-HCl, acetate or phosphate buffers), solubilisers (such as, e.g., Tween 80, Polysorbate 80), colloids, dispersion media, vehicles, fillers, chelating agents (such as, e.g., EDTA or glutathione), amino acids (such as, e.g., glycine), proteins, disintegrants, binders, lubricants, wetting agents, emulsifiers, sweeteners, colorants, flavourings, aromatisers, thickeners, agents for achieving a depot effect, coatings, antifungal agents, preservatives (such as, e.g., Thimerosal™, benzyl alcohol), antioxidants (such as, e.g., ascorbic acid, sodium metabisulfite), tonicity controlling agents, absorption delaying agents, adjuvants, bulking agents (such as, e.g., lactose, mannitol) and the like. The use of such media and agents (excipients) for pharmaceutical active substances is well known in the art. Except insofar as any conventional media or agent is incompatible with the active substance, its use in the therapeutic compositions may be contemplated. Suitable pharmaceutical carriers are described *inter alia* in Remington's Pharmaceutical Sciences, 18th ed., Mack Publishing Co., Easton, PA (1990).

Illustrative, non-limiting carriers for use in formulating the pharmaceutical compositions include, for example, oil-in-water or water-in-oil emulsions, aqueous compositions with or without inclusion of organic co-solvents suitable for intravenous (IV) use, liposomes or surfactant-containing vesicles, particulate preparations with polymeric compounds such as *inter alia* polylactic acid or polyglycolic acid, microspheres, microbeads and microsomes, powders, tablets, capsules, suppositories, aqueous suspensions, aerosols, and other carriers apparent to one of ordinary skill in the art.

Pharmaceutical carriers may comprise sterile liquids, such as water and oils, including those of petroleum, animal, vegetable or synthetic origin, such as peanut oil, soybean oil, mineral oil, sesame oil and the like.

The pharmaceutical compositions may be formulated for essentially any route of administration, such as without limitation, oral administration (such as, e.g., oral ingestion or inhalation), intranasal administration (such as, e.g., intranasal inhalation or intranasal mucosal application), pulmonary (such as, e.g., by inhalation or insufflation of powders or aerosols), parenteral administration (such as, e.g., subcutaneous, intravenous, intra-arterial, intramuscular, intraperitoneal, or intrasternal injection or infusion, or intracranial, e.g., intrathecal or intraventricular administration), intra-osseous and/or intralesional administration, epidermal and transdermal, or transmucosal (such as, e.g., oral, sublingual, intranasal) administration, topical administration (including *inter alia* ophthalmic administration), rectal, vaginal or intra-tracheal instillation, and the like. In this way, the therapeutic effects attainable by the methods and active substances, compositions or formulations as described herein can be, for example, systemic, local, tissue-specific, *etc.,* depending of the specific needs of a given application.

For example, for oral administration, pharmaceutical compositions may be formulated in the form of pills, tablets, lacquered tablets, coated (e.g., sugar-coated) tablets, granules, hard and soft gelatin capsules, aqueous, alcoholic or oily solutions, syrups, emulsions or suspensions. In an example, without limitation, preparation of oral dosage forms may be is suitably accomplished by uniformly and intimately blending together a suitable amount of the active compound in the form of a powder, optionally also including finely divided one or more solid carrier, and formulating the blend in a pill, tablet or a capsule. Exemplary but non-limiting solid carriers include calcium phosphate, magnesium stearate, talc, sugars (such as, e.g., glucose, mannose, lactose or sucrose), sugar alcohols (such as, e.g., mannitol), dextrin, starch, gelatin, cellulose, polyvinylpyrrolidine, low melting waxes and ion exchange resins. Compressed tablets containing the pharmaceutical composition can be prepared by uniformly and intimately mixing the active ingredient with a solid carrier such as described above to provide a mixture having the necessary compression properties, and then compacting the mixture in a suitable machine to the shape and size desired. Moulded tablets maybe made by moulding in a suitable machine, a mixture of powdered compound moistened with an inert liquid diluent. Suitable carriers for soft gelatin capsules and suppositories are, for example, fats, waxes, semisolid and liquid polyols, natural or hardened oils, etc.

For example, for oral or nasal aerosol or inhalation administration, pharmaceutical compositions may be formulated with illustrative carriers, such as, e.g., in solution with saline, polyethylene glycol or glycols, DPPC, methylcellulose, or in mixture with powdered dispersing agents, further employing benzyl alcohol or other suitable preservatives, absorption promoters to enhance bioavailability, fluorocarbons, and/or other solubilising or dispersing agents known in the art. Suitable pharmaceutical formulations for administration in the form of aerosols or sprays are, for example, solutions, suspensions or emulsions of the active substances in a pharmaceutically acceptable solvent, such as ethanol or water, or a mixture of such solvents. If required, the formulation can also additionally contain other pharmaceutical auxiliaries such as surfactants, emulsifiers and stabilizers as well as a propellant. Illustratively, delivery may be by use of a single-use delivery device, a mist nebuliser, a breath-activated powder inhaler, an aerosol metered-dose inhaler (MDI) or any other of the numerous nebuliser delivery devices available in the art. Additionally, mist tents or direct administration through endotracheal tubes may also be used.

Examples of carriers for administration via mucosal surfaces depend upon the particular route, e.g., oral, sublingual, intranasal, etc. When administered orally, illustrative examples include pharmaceutical grades of mannitol, starch, lactose, magnesium stearate, sodium saccharide, cellulose, magnesium carbonate and the like, with mannitol being preferred. When administered intranasally, illustrative examples include polyethylene glycol, phospholipids, glycols and glycolipids, sucrose, and/or methylcellulose, powder suspensions with or without bulking agents such as lactose and preservatives such as benzalkonium chloride, EDTA. For example, the phospholipid 1,2 dipalmitoyl-sn-glycero-3-phosphocholine (DPPC) may be used as an isotonic aqueous carrier.

For example, for parenteral administration, pharmaceutical compositions may be advantageously formulated as solutions, suspensions or emulsions with suitable solvents, diluents, solubilisers or emulsifiers, etc. Suitable solvents are, without limitation, water, physiological saline solution or alcohols, e.g. ethanol, propanol, glycerol, in addition also sugar solutions such as glucose, invert sugar, sucrose or mannitol solutions, or alternatively mixtures of the various solvents mentioned. The injectable solutions or suspensions may be formulated according to known art, using suitable nontoxic, parenterally-acceptable diluents or solvents, such as mannitol, 1,3-butanediol, water, Ringer's solution or isotonic sodium chloride solution, or suitable dispersing or wetting and suspending agents, such as sterile, bland, fixed oils, including synthetic mono- or diglycerides, and fatty acids, including oleic acid. The active substances can also be lyophilised and the lyophilisates obtained used, for example, for the production of injection or infusion preparations. For example, one illustrative example of a carrier for intravenous use includes a mixture of 10% USP ethanol, 40% USP propylene glycol or polyethylene glycol 600 and the balance USP Water for Injection (WFI). Other illustrative carriers for intravenous use include 10% USP ethanol and USP WFI; 0.01-0.1% triethanolamine in USP WFI; or 0.01-0.2% dipalmitoyl diphosphatidylcholine in USP WFI; and 1-10% squalene or parenteral vegetable oil-in-water emulsion. Water or saline solutions and aqueous dextrose and glycerol solutions may be preferably employed as carriers, particularly for injectable solutions. Illustrative examples of carriers for subcutaneous or intramuscular use include phosphate buffered saline (PBS) solution, 5% dextrose in WFI and 0.01-0.1% triethanolamine in 5% dextrose or 0.9% sodium chloride in USP WFI, or a 1 to 2 or 1 to 4 mixture of 10% USP ethanol, 40% propylene glycol and the balance an acceptable isotonic solution such as 5% dextrose or 0.9% sodium chloride; or 0.01-0.2% dipalmitoyl diphosphatidylcholine in USP WFI and 1 to 10% squalene or parenteral vegetable oil-in-water emulsions.

Where aqueous formulations are preferred, such may comprise one or more surfactants. For example, the composition can be in the form of a micellar dispersion comprising at least one suitable surfactant, e.g., a phospholipid surfactant. Illustrative examples of phospholipids include diacyl phosphatidyl glycerols, such as dimyristoyl phosphatidyl glycerol (DPMG), dipalmitoyl phosphatidyl glycerol (DPPG), and distearoyl phosphatidyl glycerol (DSPG), diacyl phosphatidyl cholines, such as dimyristoyl phosphatidylcholine (DPMC), dipalmitoyl phosphatidylcholine (DPPC), and distearoyl phosphatidylcholine (DSPC); diacyl phosphatidic acids, such as dimyristoyl phosphatidic acid (DPMA), dipahnitoyl phosphatidic acid (DPPA), and distearoyl phosphatidic acid (DSPA); and diacyl phosphatidyl ethanolamines such as dimyristoyl phosphatidyl ethanolamine (DPME), dipalmitoyl phosphatidyl ethanolamine (DPPE) and distearoyl phosphatidyl ethanolamine (DSPE). Typically, a surfactant:active substance molar ratio in an aqueous formulation will be from about 10:1 to about 1:10, more typically from about 5:1 to about 1:5, however any effective amount of surfactant may be used in an aqueous formulation to best suit the specific objectives of interest.

In certain embodiments, the pharmaceutical compositions or formulations may be configured for and delivered as bolus intravenous injection.

When rectally administered in the form of suppositories, these formulations may be prepared by mixing the active substances with a suitable non-irritating excipient, such as cocoa butter, synthetic glyceride esters or polyethylene glycols, which are solid at ordinary temperatures, but liquidify and/or dissolve in the rectal cavity to release the drug.

Suitable carriers for microcapsules, implants or rods are, for example, copolymers of glycolic acid and lactic acid.

One skilled in this art will recognize that the above description is illustrative rather than exhaustive. Indeed, many additional formulations techniques and pharmaceutically-acceptable excipients and carrier solutions are well-known to those skilled in the art, as is the development of suitable dosing and treatment regimens for using the particular compositions described herein in a variety of treatment regimens.

Further, there are several well-known methods of introducing nucleic acids (e.g., antisense and RNAi agents) into animal cells, any of which may be used herein. At the simplest, the nucleic acid can be directly injected into the target cell / target tissue. Other methods include fusion of the recipient cell with bacterial protoplasts containing the nucleic acid, the use of compositions like calcium chloride, rubidium chloride, lithium chloride, calcium phosphate, DEAE dextran, cationic lipids or liposomes or methods like receptor-mediated endocytosis, biolistic particle bombardment ("gene gun" method), infection with viral vectors, electroporation, and the like. Other techniques or methods which are suitable for delivering nucleic acid molecules to target cells include the continuous delivery of an NA molecule from poly (lactic-Co-Glycolic Acid) polymeric microspheres or the direct injection of protected (stabilized) NA molecule(s) into micropumps delivering the product. Another possibility is the use of implantable drug-releasing biodegradable microspheres. Also envisaged is encapsulation of NA in various types of liposomes (immunoliposomes, PEGylated (immuno) liposomes), cationic lipids and polymers, nanoparticules or dendrimers, poly (lactic-Co-Glycolic Acid) polymeric microspheres, implantable drug-releasing biodegradable microspheres, etc; and co-injection of NA with protective agent like the nuclease inhibitor aurintricarboxylic acid. It shall be clear that also a combination of different above-mentioned delivery modes or methods may be used.

Further ways of delivery of nucleic acids such as antisense agents and RNAi agents may employ previously published methods. For example, intracellular delivery of the nucleic acids may be via a composition comprising an admixture of the nucleic acid molecule and an effective amount of a block copolymer. An example of this method is described in US 2004/0248833.

Other methods of delivery of nucleic acids to the nucleus are described in Mann et al. 2001 (Proc Natl Acad Science 98(1): 42-47) and in Gebski et al. 2003 (Human Molecular Genetics 12(15): 1801-1811).

A method for introducing a nucleic acid molecule into a cell by way of an expression vector either as naked DNA or complexed to lipid carriers, is described in US 6,806,084.

It may be desirable to deliver a nucleic acid molecule in a colloidal dispersion system. Colloidal dispersion systems include macromolecule complexes, nanocapsules, microspheres, beads, and lipid-based systems including oil-in- water emulsions, micelles, mixed micelles, and liposomes or liposome formulations. Liposomes are artificial membrane vesicles which are useful as delivery vehicles *in vitro* and *in vivo.* These formulations may have net cationic, anionic or neutral charge characteristics and are useful characteristics with *in vitro, in vivo* and *ex vivo* delivery methods. It has been shown that large unilamellar vesicles (LUV), which range in size from 0.2-4.0 PHI.m can encapsulate a substantial percentage of an aqueous buffer containing large macromolecules. RNA, and DNA can be encapsulated within the aqueous interior and be delivered to cells in a biologically active form (Fraley et al. 1981 (Trends Biochem ScL 6: 77).

In order for a liposome to be an efficient gene transfer vehicle, the following characteristics should be present: (1) encapsulation of the nucleic acid molecule of interest at high efficiency while not compromising their biological activity; (2) preferential and substantial binding to a target cell in comparison to non-target cells; (3) delivery of the aqueous contents of the vesicle to the target cell cytoplasm at high efficiency; and (4) accurate and effective expression of genetic information (Mannino et al. 1988 (Biotechniques 6: 682).

The composition of the liposome is usually a combination of phospholipids, particularly high-phase-transition-temperature phospholipids, usually in combination with steroids, especially cholesterol. Other phospholipids or other lipids may also be used. The physical characteristics of liposomes depend on pH, ionic strength, and the presence of divalent cations.

Alternatively, the nucleic acid molecule may be combined with other pharmaceutically acceptable carriers or diluents to produce a pharmaceutical composition. Suitable carriers and diluents include isotonic saline solutions, for example phosphate-buffered saline. The composition may be formulated for parenteral, intramuscular, intravenous, subcutaneous, intraocular, oral or transdermal administration.

The routes of administration described are intended only as a guide since a skilled practitioner will be able to determine readily the optimum route of administration and any dosage for any particular animal and condition. Multiple approaches for introducing functional new genetic material into cells, both *in vitro* and *in vivo* have been attempted (Friedmann 1989 (Science 244: 1275-1280)). These approaches include integration of the gene to be expressed into modified retroviruses (Friedmann 1989, *supra;* Rosenberg. Cancer Res. 1991, vol. 51(18), 5074S-79S); integration into non-retrovirus vectors (Rosenfeld et al. Cell, 1992, vol. 68,143-55; Rosenfeld et al. Science,1991, vol. 252, 431-4); or delivery of a transgene linked to a heterologous promoter-enhancer element via liposomes (Friedmann 1989, *supra;* Brigham et al. Am. J. Med. Sci., 1989, vol. 298, 278-81; Nabel et al. Science, 1990, vol. 249, 1285-8; Hazinski et al. Am. J. Resp. Cell. Molec. Biol., 1991, vol. 4, 206-9; Wang & Huang. Proc. Natl. Acad. Sci. USA., 1987, vol. 84, 7851-5); coupled to ligand-specific, cation-based transport systems (Wu & Wu. J. Biol. Chem., 1988, vol. 263, 14621-4)) or the use of naked DNA, expression vectors (Nabel et al. 1990, *supra;* Wolff et al. Science, 1990, vol. 247, 1465-8). Direct injection of transgenes into tissue produces only localized expression (Rosenfeld. 1992, *supra;* Rosenfeld et al. *1991, supra;* Brigham et al. 1989, *supra;* Nabel 1990, *supra;* Hazinski et al. 1991, supra). The Brigham et al. group *(*Am. J. Med. Sci., 1989, vol. 298, 278-81; Clin. Res., 1991, vol. 39, abstract) have reported *in vivo* transfection only of lungs of mice following either intravenous or intratracheal administration of a DNA liposome complex. An example of a review article of human gene therapy procedures is: Anderson. Science, 1992, vol. 256, 808-13.

The pharmaceutical formulations as disclosed herein, which may conveniently be presented in unit dosage form, may be prepared according to conventional techniques well known in the pharmaceutical industry. Such techniques may generally include the step of bringing into association the active ingredients with the pharmaceutical carrier(s) or excipient(s). In general the formulations are prepared by uniformly and intimately bringing into association the active ingredients with liquid carriers or finely divided solid carriers or both, and then, if necessary, shaping the product.

The dosage or amount of the present active agents used, optionally in combination with one or more other active compound to be administered, depends on the individual case and is, as is customary, to be adapted to the individual circumstances to achieve an optimum effect. Thus, it depends on the nature and the severity of the disorder to be treated, and also on the sex, age, body weight, general health, diet, mode and time of administration, and individual responsiveness of the human or animal to be treated, on the route of administration, efficacy, metabolic stability and duration of action of the compounds used, on whether the therapy is acute or chronic or prophylactic, or on whether other active compounds are administered in addition to the agent(s) of the invention.

Without limitation, depending on the type and severity of the disease, a typical daily dosage might range from about 1 ng/kg to 100 mg/kg of body weight or more, depending on the factors mentioned above. For repeated administrations over several days or longer, depending on the condition, the treatment is sustained until a desired suppression of disease symptoms occurs. A preferred dosage of the active substance described herein may be in the range from about 0.05 mg/kg to about 10 mg/kg of body weight. Thus, one or more doses of about 0.5 mg/kg, 2.0 mg/kg, 4.0 mg/kg or 10 mg/kg (or any combination thereof) may be administered to the patient. Such doses may be administered intermittently, e.g., every week or every two or three weeks.

In an embodiment, a pharmaceutical composition may comprise between about 10 nM and about 1 µM, preferably between about 20 nM and about 600 nM, such as, e.g., about 100 nM or about 200 nM, or about 300 nM, or about 400 nM or about 500 nM of antisense agent or RNAi agent as taught herein.

In certain embodiments, the active substances as described herein may be administered daily during the treatment. In certain embodiments, the active substances as described herein may be administered at least once a day during the treatment, for example the active substances as described herein may be administered at least twice a day during the treatment, for example the active substances as described herein may be administered at least three times a day during the treatment. In certain embodiments, the active substances as described herein may be administered continuously during the treatment for instance in an aqueous drinking solution.

For the treatment of cardiac warm ischemia reperfusion injury resulting from acute myocardial infarction, it may be desirable to apply the treatment with a therapeutic agent as disclosed herein as soon as possible following the acute myocardial infarction, for example, as soon as about 0.5 hour, about 1 hour, about 2 hours, about 6 hours, about 12 hours, or about 24 hours following the acute myocardial infarction; or prior to, concomitantly with, or as soon as possible following any treatment of the myocardial infarction aimed at restoring blood flow to the ischemic areas of the heart (e.g., anticoagulant therapy), for example, at least about 0.5 hour, about 1 hour or about 2 hours before the treatment aimed at restoring blood flow to the ischemic areas of the heart, or for example, as soon as about 0.5 hour, about 1 hour, about 2 hours, about 6 hours, about 12 hours, or about 24 hours following the treatment aimed at restoring blood flow to the ischemic areas of the heart. Suitable anticoagulant therapeutics include without limitation antithrombotics, such as heparin and coumarin, thrombolytics, such as streptokinase, urokinase, and tissue plasminogen activator, and antithrombocytics.

Accordingly, also disclosed herein is a pharmaceutical combination or composition for simultaneous, separate or sequential (in any order) use in the treatment of cardiac warm ischemia reperfusion injury, wherein said composition comprises a therapeutic agent as described herein and an anticoagulant.

Accordingly, also disclosed in certain embodiments is the therapeutic agent as intended herein capable of interacting with at least one of the CD80 or CD86 polypeptides or capable of preventing or inhibiting the expression of at least one of the CD80 or CD86 polypeptides by a cell or capable of preventing or inhibiting the presentation of at least one of the CD80 or CD86 polypeptides on the surface of a cell, wherein the therapeutic agent is co-administered, such as co-administered simultaneously, separately or sequentially (in any order), with an anticoagulant. In certain embodiments, the additional co-administration, such as simultaneous, separate or sequential (in any order) co-administration, of a second therapeutic agent capable of interacting with the CD40 or capable of preventing or inhibiting the expression of the CD40 polypeptide by a cell or capable of preventing or inhibiting the presentation of the CD40 polypeptide on the surface of a cell, is also contemplated.

For the treatment of cardiac warm ischemia reperfusion injury consequent to therapeutic interventions, such as surgical procedures, e.g., coronary artery bypass graft surgery, coronary angioplasty, vessel ligation and reperfusion during heart transplant surgery and the like, it may be preferable that a therapeutic agent as described herein is administered to a subject undergoing treatment prior to the therapeutic intervention. For example, a therapeutic agent as described herein can be administered to a subject undergoing treatment, e.g., about 1 hour, about 2 hours, about 3 hours, about 4 hours, about 5 hours, about 12 hours, about 24 hours, or about 48 hours prior to the therapeutic intervention. A therapeutic agent as described herein can also be administered to a subject undergoing treatment, for example, about 5 minutes, about 10 minutes, about 15 minutes, about 20 minutes, about 30 minutes or about 45 minutes prior to the therapeutic intervention. A therapeutic agent as described herein can also be administered to a subject undergoing treatment during the therapeutic intervention. A therapeutic agent as described herein can also be administered to a subject undergoing treatment after the therapeutic intervention (i.e. post-intervention), e.g. about 5 minutes, about 10 minutes, about 15 minutes, about 20 minutes, about 30 minutes, about 45 minutes, about 1 hour, about 2 hours, about 3 hours, about 4 hours, about 5 hours, about 6 hours, about 12 hours, about 24 hours, or about 48 hours following the therapeutic intervention.

A therapeutic agent as described herein may be used alone or in combination with any one or more active substances useful for the treatment of cardiac warm ischemia reperfusion injury known in the art (combination therapy), such as one or more immunosuppressant.

Accordingly, also disclosed herein is a pharmaceutical combination or composition for simultaneous, separate or sequential (in any order) use in the treatment of cardiac warm ischemia reperfusion injury, wherein said composition comprises a therapeutic agent as described herein and an immunosuppressant.

Accordingly, also disclosed in certain embodiments is the therapeutic agent as intended herein capable of interacting with at least one of the CD80 or CD86 polypeptides or capable of preventing or inhibiting the expression of at least one of the CD80 or CD86 polypeptides by a cell or capable of preventing or inhibiting the presentation of at least one of the CD80 or CD86 polypeptides on the surface of a cell, wherein the therapeutic agent is co-administered, such as co-administered simultaneously, separately or sequentially (in any order), with an immunosuppressant. In certain embodiments, the additional co-administration, such as simultaneous, separate or sequential (in any order) co-administration, of a second therapeutic agent capable of interacting with the CD40 or capable of preventing or inhibiting the expression of the CD40 polypeptide by a cell or capable of preventing or inhibiting the presentation of the CD40 polypeptide on the surface of a cell, is also contemplated.

The term "immunosuppressant" as used herein is meant to include compounds or compositions which suppress immune responses. Exemplary immunosuppressants include azathioprine, macrolides and cyclosporins, in particular macrolides such as pimecrolimus, tacrolimus and sirolimus and cyclosporins such as cyclosporin A. Alternative immunosuppressants include muromonab CD3, daclizumab, basiliximab, alemtuzumab and other biological immunosuppressants (for example those targeting CD3, CD4 or CD8).

Except when noted, "subject" or "patient" are used interchangeably and refer to animals, preferably warm-blooded animals, more preferably vertebrates, even more preferably mammals, still more preferably primates, and specifically includes human patients and non-human mammals and primates. Preferred patients are human subjects. Hence, in an embodiment, the subject is human.

As used herein, a phrase such as "a subject in need of treatment" includes subjects that would benefit from treatment of a given condition, particularly cardiac warm ischemia reperfusion injury. Such subjects may include, without limitation, those that have been diagnosed with said condition, those prone to contract or develop said condition and/or those in whom said condition is to be prevented. Particularly intended herein are subjects suffering or prone to contract or develop cardiac ischemia, such as subjects suffering a myocardial infarction, or subject undergoing cardiac surgery involving temporarily stopping the blood flow to the myocardium.

The term "therapeutically effective amount" as used herein, refers to an amount of active compound or pharmaceutical agent that elicits the biological or medicinal response in a subject that is being sought by a researcher, veterinarian, medical doctor or other clinician, which may include *inter alia* alleviation of the symptoms of the disease or condition being treated. Methods are known in the art for determining therapeutically effective doses for the present agents.

The term "prophylactically effective amount" refers to an amount of an active compound or pharmaceutical agent that inhibits or delays in a subject the onset of a disorder as being sought by a researcher, veterinarian, medical doctor or other clinician. The term "therapeutically effective amount" as used herein, refers to an amount of active compound or pharmaceutical agent that elicits the biological or medicinal response in a subject that is being sought by a researcher, veterinarian, medical doctor or other clinician, which may include inter alia alleviation of the symptoms of the disease or condition being treated. Methods are known in the art for determining prophylactically effective doses for the present agents.

The ensuing clauses provide additional illustration of certain aspects and embodiments that have been disclosed in accordance with the present invention:
(i) A therapeutic agent for use in the treatment of cardiac warm ischemia reperfusion injury, wherein the therapeutic agent is capable of interacting with at least one of the CD80 or CD86 polypeptides, and wherein said interaction interferes with the binding of the at least one of the CD80 or CD86 polypeptides to the CD28 polypeptide.
(ii) The therapeutic agent for use in the treatment of cardiac warm ischemia reperfusion injury according to clause (i), wherein the at least one of the CD80 or CD86 polypeptides is comprised by an antigen presenting cell (APC) and wherein the CD28 polypeptide is comprised by a T-cell.
(iii) A therapeutic agent for use in the treatment of cardiac warm ischemia reperfusion injury, wherein the therapeutic agent is capable of preventing or inhibiting the expression of at least one of the CD80 or CD86 polypeptides by a cell or capable of preventing or inhibiting the presentation of at least one of the CD80 or CD86 polypeptides on the surface of a cell.
(iv) The therapeutic agent for use in the treatment of cardiac warm ischemia reperfusion injury according to clause (iii), wherein the cell is an antigen presenting cell (APC).
(v) The therapeutic agent for use in the treatment of cardiac warm ischemia reperfusion injury according to any one of clauses (i) to (iv), wherein the agent is selected from the group consisting of a protein, a polypeptide, a peptide, a peptidomimetic, a nucleic acid, a small organic molecule, and a compound or combination of any two or more thereof.
(vi) The therapeutic agent for use in the treatment of cardiac warm ischemia reperfusion injury according to any one of clauses (i) to (v), wherein the agent is selected from the group consisting of an antibody or a fragment or derivative thereof, a soluble receptor, and an aptamer.
(vii) The therapeutic agent for use in the treatment of cardiac warm ischemia reperfusion injury according to clause (vi), wherein the antibody is a polyclonal antibody, a monoclonal antibody, a chimeric antibody, a humanized antibody, a primatized antibody, a human antibody, a Nanobody, or mixtures thereof.
(viii) The therapeutic agent for use in the treatment of cardiac warm ischemia reperfusion injury according to clause (vi), wherein the soluble receptor is a soluble CTLA4 molecule, preferably a CTLA4Ig fusion protein.
(ix) The therapeutic agent for use in the treatment of cardiac warm ischemia reperfusion injury according to any one of clauses (iii) to (v), wherein the agent is selected from the group consisting of an intrabody or a fragment or derivative thereof, an antisense agent, and an RNAi agent.
(x) The therapeutic agent for use in the treatment of cardiac warm ischemia reperfusion injury according to any one of clauses (i) to (ix), wherein the therapeutic agent is co-administered with a second therapeutic agent, wherein the second therapeutic agent is capable of interacting with the CD40 polypeptide, and wherein said interaction interferes with the binding of the CD40 polypeptide to the CD40 ligand (CD40L) polypeptide.
(xi) The therapeutic agent for use in the treatment of cardiac warm ischemia reperfusion injury according to clause (x), wherein the CD40 polypeptide is comprised by an antigen presenting cell (APC) and wherein the CD40L polypeptide is comprised by a T-cell.
(xii) The therapeutic agent for use in the treatment of cardiac warm ischemia reperfusion injury according to any one of clauses (i) to (ix), wherein the therapeutic agent is co-administered with a second therapeutic agent, capable of preventing or inhibiting the expression of the CD40 polypeptide by a cell or capable of preventing or inhibiting the presentation of the CD40 polypeptide on the surface of a cell.
(xiii) The therapeutic agent for use in the treatment of cardiac warm ischemia reperfusion injury according to clause (xii), wherein the cell is an antigen presenting cell (APC).
(xiv) The therapeutic agent for use in the treatment of cardiac warm ischemia reperfusion injury according to any one of clauses (x) to (xiii), wherein the second agent is selected from the group consisting of a protein, a polypeptide, a peptide, a peptidomimetic, a nucleic acid, a small organic molecule, and a compound or combination of any two or more thereof.
(xv) The therapeutic agent for use in the treatment of cardiac warm ischemia reperfusion injury according to any one of clauses (x) to (xiv), wherein the second agent is selected from the group consisting of an antibody or a fragment or derivative thereof, a soluble receptor, and an aptamer.
(xvi) The therapeutic agent for use in the treatment of cardiac warm ischemia reperfusion injury according to any one of clauses (xii) to (xiv), wherein the second agent is selected from the group consisting of an intrabody or a fragment or derivative thereof, an antisense agent, and an RNAi agent.
(xvii) The therapeutic agent for use in the treatment of cardiac warm ischemia reperfusion injury according to any one of clauses (i) to (xvi), wherein the therapeutic agent is co-administered with an anticoagulant.
(xviii) The therapeutic agent for use in the treatment of cardiac warm ischemia reperfusion injury according to any one of clauses (i) to (xvi), wherein the therapeutic agent is co-administered with an immunosuppressant.
(xix) The therapeutic agent for use in the treatment of cardiac warm ischemia reperfusion injury according to any one of clauses (i) to (xviii), wherein the cardiac warm ischemia reperfusion injury is consequent to myocardial infarction, chronic myocardial infarction, acute myocardial infarction (STEMI), treatment of the myocardial infarction aimed at restoring blood flow to the ischemic areas of the heart, cardiovascular arrest, coronary artery bypass graft surgery, coronary angioplasty, transient vasospasm, acute coronary syndromes (NSTEMI), cardioplegia, or vessel ligation and reperfusion during heart transplant surgery.
(xx) The therapeutic agent for use in the treatment of cardiac warm ischemia reperfusion injury according to any one of clauses (i) to (xix), wherein the cardiac warm ischemia reperfusion injury is consequent to acute myocardial infarction.

It is apparent that there have been provided in accordance with the invention products, methods and uses that provide for substantial advantages as set forth above. While the invention has been described in conjunction with specific embodiments thereof, it is evident that many alternatives, modifications, and variations will be apparent to those skilled in the art in light of the foregoing description. Accordingly, it is intended to embrace all such alternatives, modifications, and variations as follows in the spirit and broad scope of the appended claims.

The above aspects and embodiments are further supported by the following non-limiting examples.

### EXAMPLES

### Example 1: Screening for CD80, CD86 and/or CD40 blocking agents

Test blocking agents (e.g., peptides, antibodies, antibody fragments, soluble receptors, small organic molecules, etc.) are screened for binding to human CD80, human CD86 and human CD40 molecules by Bio-Layer Interferometry (BLI), to identify blocking agents which can be useful as therapeutic agents in various embodiments of the invention. Briefly, fusion proteins CD86-Fc, CD80-Fc and CD40-Fc (purchased from R&D systems, Minnesota) are immobilized on anti-human IgG Fc capture biosensors (Fortébio, California). The test blocking agents are then added to the wells and binding to the target molecules is recorded in real-time with an Octet@ RED96 System (Fortébio, California). The real-time kinetic measurements also enable to accurately determine *in vitro* binding properties such as *kₐ* (association rate constant), *k*_{d} (dissociation rate constant), *K*_{D} (dissociation constant). Test blocking agents considered as suitable candidates for further development are such that they bind to their target with a *K*_{D} (dissociation constant) = *k_{d}*/*kₐ* ≤ 10⁻⁷ M.

### Example 2: Inhibition of binding of CD80/CD86 with CD28

In order to assess if test blocking agents can interfere with CD80 and CD86 binding to CD28, Raji cells (10⁵ cells/well) are incubated with increasing concentrations of CD80 and CD86 test blocking agents (e.g., peptides, antibodies, antibody fragments, soluble receptors, small organic molecules, etc.) for one hour. Next, a fusion protein consisting of the extracellular domain of recombinant human CD28 fused to murine IgG2 Fc (purchased from Ancell, Minnesota, USA) is added in the wells at a saturating concentration, without washing the cells in between. After an overnight incubation, cells are washed and stained with a PE-conjugated anti-murine IgG2a antibody (purchased from BD Biosciences, Belgium). Cells are then washed, fixated in paraformaldehyde and measured by flow cytometry, using a BD FACSCanto™ II. Percentage of binding inhibition is calculated based on mean fluorescence intensity values of positive controls obtained without the addition of blocking agents and negative controls obtained without the additions of CD28 fusion protein. The EC50 of each test blocking agent is calculated from the binding curve. Test blocking agents considered as suitable candidates for further development are such that preferably the EC50 is ≤ 1nM.

### Example 3: Inhibition of interaction of CD40 with CD40L

In order to assess if test blocking agents can interfere with CD40 binding to CD40 Ligand, CD40 expressing CHO cells (10⁵ cells/well) are incubated with increasing concentrations of CD40 test blocking agents (e.g., peptides, antibodies, antibody fragments, soluble receptors, small organic molecules, etc.) for one hour. Next, the extracellular domain of recombinant human CD40L fused to His-tag (purchased from Acris Antibodies, California, USA) is added in the wells at a saturating concentration, without washing the cells in between. After an overnight incubation, cells are washed and stained with a PE-conjugated anti-his tag antibody (purchased from Miltenyi Biotec, The Netherlands). Cells are then washed, fixated in paraformaldehyde and measured by flow cytometry, using a BD FACSCanto™ II. Percentage of binding inhibition is calculated based on mean fluorescence intensity values of positive controls obtained without the addition of blocking agents and negative controls obtained without the additions of CD40 ligand protein. The EC50 of each test blocking agent is calculated from the binding curve. Test blocking agents considered as suitable candidates for further development are such that preferably the EC50 is ≤ 1nM.

### Example 4: Cross-reactivity of human targeted blocking agents

To demonstrate that the non-human primate model is appropriate for corroborating the functionality of blocking agents targeting human co-stimulatory molecules (CD80, CD86, CD40) in a setting of myocardial infarction, binding of candidate blocking agents to primate antigen presenting cells (APC) is assessed by flow cytometry.

Dendritic cells are isolated from rhesus monkey and human blood by magnetic activated cell sorting using commercially available kits (purchased from Miltenyi Biotec, The Netherlands). Isolated dendritic cells fractions are incubated in the presence of the blocking agents that are selected in the preceding examples, such as in Example 1. Human APC are used as positive control. Binding of said blocking agents to dendritic cells is revealed through the use of proper detection system. In case the blocking agent is an antibody, an appropriate secondary antibody coupled to a fluorochrome-like phycoerythrin is used. In case the blocking agent is a Nanobody® tagged with c-myc, anti-c-myc tag monoclonal antibody is used, followed by a fluorochrome-labeled antibody against c-myc tag antibody. In case soluble receptor-derived blocking agents, such as CTLA4-Ig, are used, monoclonal mouse anti-human IgG1 antibody (Sigma, #I2513) is added as secondary antibody, followed by an anti-mouse fluorochrome labeled antibody. Binding of blocking agents is analyzed by flow cytometry with a BD FACSCanto™ II. Fluorescence intensity and percentage of labelled cells is assessed with BD FACSDiva™ software.

The experiments confirm that many blocking agents targeting human co-stimulatory molecules (CD80, CD86, CD40), which can be useful as therapeutic agents in various embodiments of the invention, can also bind to non-human primate antigen presenting cells (APC). Therefore, the rhesus monkey model is appropriate for corroborating the functionality of such blocking agents.

### Example 5: One-way allogeneic mixed lymphocyte reaction (MLR)

The *in vitro* functionality of blocking agents targeting human co-stimulatory molecules (CD80, CD86, CD40), which can be useful as therapeutic agents in various embodiments of the invention, i.e. the ability of the blocking agents to interfere with induction of the T-cell response, is assessed by performing one-way allogeneic mixed lymphocytes reactions. Both human and rhesus monkey models are being tested.

Peripheral blood mononuclear cells (PBMCs) from 2 different donors, designated as stimulator and responder population, respectively, are co-cultured in 200 µL RPMI1640 medium (Gibco) supplemented with 10% fetal bovine serum (FBS) for at least 3 days. The stimulator population is inactivated through treatment with mitomycin before being added into the well so that they merely provide foreign allo-antigens to the responder population. The blocking agents that are selected in Example 1 and 2 are added to the wells alone, or in combination (i.e., targeting different co-stimulatory molecules). Each condition is repeated in triplicate. Proliferation of CD4+ and CD8+ T cells from the responder population is measured through the uptake of [³H]-thymidine, added in the culture media for the duration of the co-culture. IL-2 secretion is measured in parallel by performing ELISA on culture supernatants. For this purpose, a commercially available kit from R&D systems (Minneapolis, Minnesota) is used per manufacturer's recommendations. Cyclosporine is used as positive control in the assay. Increasing doses of each blocking agent are added to the co-culture media in order to determine the EC50.

The blocking agent with the lowest EC50 is expected to be the most potent co-stimulatory pathway inhibitor *in vivo.* Blocking agents with EC50's in the nanomolar or preferably sub-nanomolar range and that show comparable results in the non-human primate and human models are selected for conducting *in vivo* experiments.

### Example 6: In vivo treatment of cardiac warm ischemia reperfusion injury in a non-human primate model of myocardial infarction

### Material and methods

### Animal model

21 *Macaca Mulatta* Rhesus monkeys are included in the study after approval by the local Institutional Animal Care and Use Comittee. Animals are fully-grown at the time of infarction, with an average weight of 8 to 10 kg.

### Myocardial Infarction and test articles delivery

Following induction of anesthesia, a myocardial infarction is induced by a 90-minute balloon inflation in the left circumflex coronary artery. Blood flow occlusion is confirmed via fluoroscopy. Animals are defibrillated as necessary. At the end of the ischemic period, the balloon is deflated and the ischemic area is allowed to reperfuse. Animals are then randomized to 4 treatment groups: negative controls (n=6), cyclosporine (n=5),CTLA4-Ig (purchased from R&D systems) (n=5) and CTLA4-Ig combined with a monoclonal anti-CD40 antibody (purchased from R&D systems) (n=5). Shortly before reperfusion, animals receive a bolus intravenous injection of saline solution or water for injection (n=3 per vehicle), 2,5 mg of cyclosporine per kg of body weight dissolved in a saline solution at a concentration of 25mg/mL (cyclosporine group) (Piot et al, 2012), or 10 mg of CTLA4-Ig per kg of body weight diluted to a final concentration of 25mg/ml with sterile water for injection (CTLA4-Ig group) or a combination of 10 mg of CTLA4-Ig and 10 mg anti-CD40 antibody per kg of body weight diluted as described above (CTLA4-Ig + CD40 Ab group).

### Cardiac enzymes measurement

Blood samples are taken repeatedly from reperfusion time point up to 72 hours post-reperfusion. Blood levels of cardiac biomarkers, specifically creatine kinase MB (CKMB) and cardiac troponin I (cTnI), are measured using Meso Scale Discovery assay kits, according to manufacturer's recommendations. These measurements are used to assess the size of the infarct post-myocardial infarction.

### Magnetic resonance imaging (MRI)

The study design includes 4 MRI analyses. A control MRI is performed before induction of myocardial infarction, a baseline MRI is then performed 3 ± 1 days post-infarct, a follow-up MRI is carried out 14 ± 1 days post-infarct and a final MRI is achieved prior to sacrifice at 42 ± 2 days post-infarct. A Siemens Magnetom Sonata 1.5 Tesla MRI apparatus is used. MRI active electrodes, Sp02 and pulse sensor are installed. A CP Body Array Flex Coil is positioned on the animal's chest. The syngo MR A30 software is used to acquire the images. Cine short-axis images are acquired for functional analysis of the heart using a retrogated sequence. It is repeated from the base to the apex for slices of about 8 mm. A dose of 0,2 mmol/kg of gadoteridol is injected intravenously at a concentration of 0,05 mM/ml at a rate of 2 ml/s for image acquisition. Blood oxygen saturation, color of mucous membranes and pulse rate are monitored regularly. Obtained images are exported in Medis QMass MR Research Edition Software for analysis of left ventricle (LV) volumes, left ventricular ejection fraction (LVEF), wall motion, wall thickening and infarct size.

### Necropsy and Tissue Harvest

At the end of the final MRI (42 days post-infarct), animals are euthanized with propofol, given intravenously (IV), until surgical anesthesia level is reached and then a lethal injection of saturated potassium chloride is administered IV as a rapid bolus. Death is confirmed by observation of asystole or ventricular fibrillation on the electrocardiogram (ECG).

### Estimation of scar weight

After sacrifice, hearts are excised. Atria and large arteries are dissected off from the ventricles at the atrio-ventricular groove. Ventricles are then rinsed and breadloafed into about 1 cm thick slices, starting at the apex. Heart slices are then sandwiched between glass panels and pictures of the slices are taken. Slices containing the infarct zones are analyzed using image analysis software, by manually tracing the area of the infarct zones and the total section area. For each image, the infarct area and the total ventricle section are measured and the ratio between both surfaces is calculated. Estimation of infarct weight and volume is deduced by combining this ratio with slices weight and thickness.

### Results

*In vivo* efficacy of CTLA4-Ig is assessed in a non-human primate model of acute myocardial infarction. It is expected from Example 4 and 5 that primate molecules are successfully targeted by CTLA4-Ig targeting human CD80 and CD86 molecules and anti-CD40 antibody. Cyclosporine is used as a positive control as it has already been shown to induce scar size reduction in patients suffering from acute myocardial infarction.

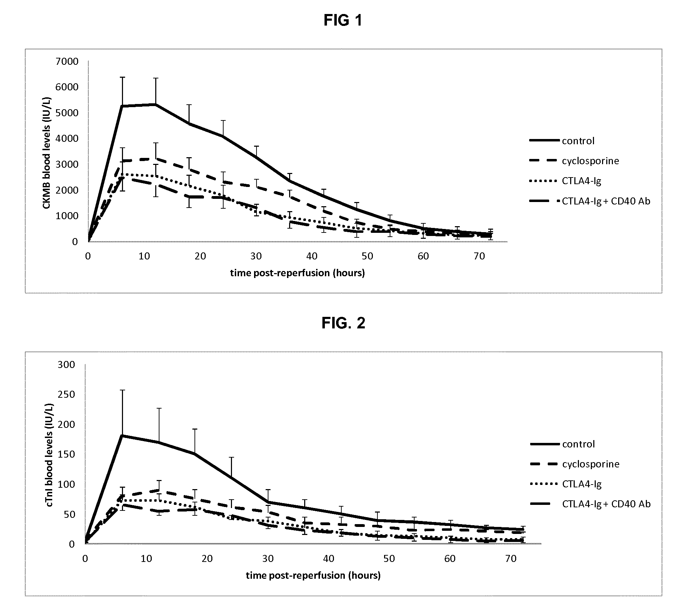

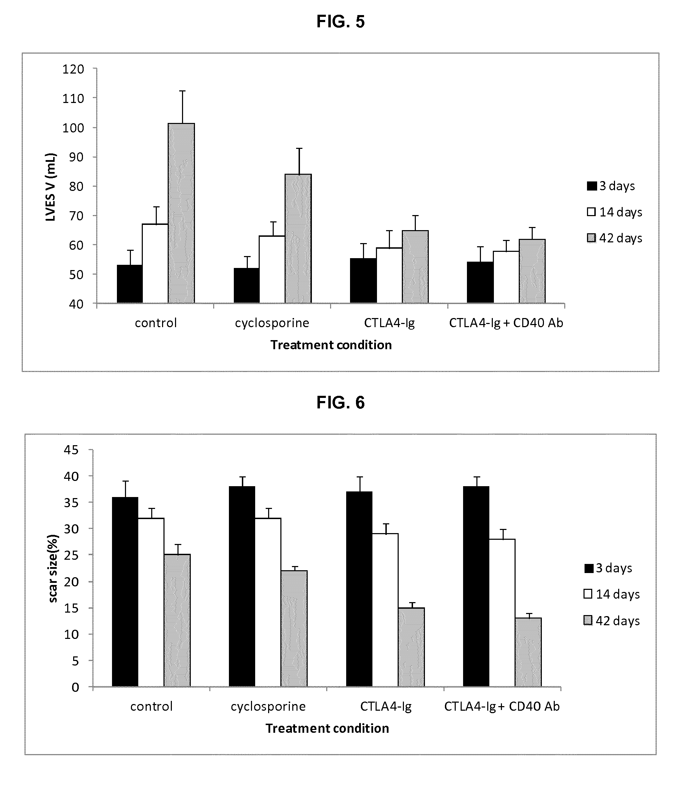

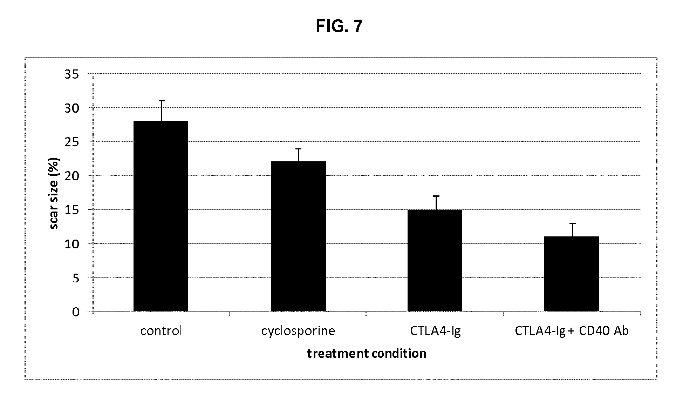

Example results for CTLA4-Ig or the combination of CTLA4-Ig and anti-CD40 antibody which could be expected in certain experiments for the endpoints to be assessed are shown in Figures 1-7. The primary endpoints are reduction of infarct size as measured by blood levels of the cardiac enzymes cardiac troponin I (Fig. 2) and creatine kinase (Fig. 1) shortly post-reperfusion (up to 72 hours), or magnetic resonance imaging (MRI) at longer time intervals (up to 2 months) (Fig. 6). Histological evaluation is also performed at sacrifice (Fig. 7). The secondary endpoints are focused on the improvement of cardiac function as assessed by MRI through the measurement of left ventricular end diastolic volume (Fig. 4), left ventricular end systolic volume (Fig. 5) and ejection fraction (Fig. 3).

Hence, in accordance with some embodiments of the invention, it is postulated that in an illustrative setting of acute myocardial infarction, CTLA4-Ig, through the blocking of the co-stimulatory pathway B7:CD28 and CTLA4-Ig administered in combination with anti-CD40 antibody, can treat cardiac warm ischemia reperfusion injury, thereby inducing improvement of heart function, decreasing heart remodelling and reducing scar size.

## Claims

1. A therapeutic agent for use in the treatment of cardiac warm ischemia reperfusion injury, wherein the therapeutic agent is capable of interacting with at least one of the CD80 or CD86 polypeptides, and wherein said interaction interferes with the binding of the at least one of the CD80 or CD86 polypeptides to the CD28 polypeptide.

2. The therapeutic agent for use in the treatment of cardiac warm ischemia reperfusion injury according to claim 1, wherein the at least one of the CD80 or CD86 polypeptides is comprised by an antigen presenting cell (APC) and wherein the CD28 polypeptide is comprised by a T-cell.

3. A therapeutic agent for use in the treatment of cardiac warm ischemia reperfusion injury, wherein the therapeutic agent is capable of preventing or inhibiting the expression of at least one of the CD80 or CD86 polypeptides by a cell or capable of preventing or inhibiting the presentation of at least one of the CD80 or CD86 polypeptides on the surface of a cell.

4. The therapeutic agent for use in the treatment of cardiac warm ischemia reperfusion injury according to claim 3, wherein the cell is an antigen presenting cell (APC).

5. The therapeutic agent for use in the treatment of cardiac warm ischemia reperfusion injury according to any one of claims 1 to 4, wherein the agent is selected from the group consisting of a protein, a polypeptide, a peptide, a peptidomimetic, a nucleic acid, a small organic molecule, and a compound or combination of any two or more thereof.

6. The therapeutic agent for use in the treatment of cardiac warm ischemia reperfusion injury according to any one of claims 1 to 5, wherein the agent is selected from the group consisting of an antibody or a fragment or derivative thereof, a soluble receptor, and an aptamer.

7. The therapeutic agent for use in the treatment of cardiac warm ischemia reperfusion injury according to claim 6, wherein the antibody is a polyclonal antibody, a monoclonal antibody, a chimeric antibody, a humanized antibody, a primatized antibody, a human antibody, a Nanobody, or mixtures thereof.

8. The therapeutic agent for use in the treatment of cardiac warm ischemia reperfusion injury according to claim 6, wherein the soluble receptor is a soluble CTLA4 molecule, preferably a CTLA4Ig fusion protein.

9. The therapeutic agent for use in the treatment of cardiac warm ischemia reperfusion injury according to any one of claims 3 to 5, wherein the agent is selected from the group consisting of an intrabody or a fragment or derivative thereof, an antisense agent, and an RNAi agent.

10. The therapeutic agent for use in the treatment of cardiac warm ischemia reperfusion injury according to any one of claims 1 to 9, wherein the therapeutic agent is co-administered with a second therapeutic agent, wherein the second therapeutic agent is capable of interacting with the CD40 polypeptide, and wherein said interaction interferes with the binding of the CD40 polypeptide to the CD40 ligand (CD40L) polypeptide.

11. The therapeutic agent for use in the treatment of cardiac warm ischemia reperfusion injury according to claim 10, wherein the CD40 polypeptide is comprised by an antigen presenting cell (APC) and wherein the CD40L polypeptide is comprised by a T-cell.

12. The therapeutic agent for use in the treatment of cardiac warm ischemia reperfusion injury according to any one of claims 1 to 9, wherein the therapeutic agent is co-administered with a second therapeutic agent, capable of preventing or inhibiting the expression of the CD40 polypeptide by a cell or capable of preventing or inhibiting the presentation of the CD40 polypeptide on the surface of a cell.

13. The therapeutic agent for use in the treatment of cardiac warm ischemia reperfusion injury according to claim 12, wherein the cell is an antigen presenting cell (APC).

14. The therapeutic agent for use in the treatment of cardiac warm ischemia reperfusion injury according to any one of claims 10 to 13, wherein the second agent is selected from the group consisting of a protein, a polypeptide, a peptide, a peptidomimetic, a nucleic acid, a small organic molecule, and a compound or combination of any two or more thereof.

15. The therapeutic agent for use in the treatment of cardiac warm ischemia reperfusion injury according to any one of claims 10 to 14, wherein the second agent is selected from the group consisting of an antibody or a fragment or derivative thereof, a soluble receptor, and an aptamer.

16. The therapeutic agent for use in the treatment of cardiac warm ischemia reperfusion injury according to any one of claims 12 to 14, wherein the second agent is selected from the group consisting of an intrabody or a fragment or derivative thereof, an antisense agent, and an RNAi agent.

17. The therapeutic agent for use in the treatment of cardiac warm ischemia reperfusion injury according to any one of claims 1 to 16, wherein the therapeutic agent is co-administered with an anticoagulant.

18. The therapeutic agent for use in the treatment of cardiac warm ischemia reperfusion injury according to any one of claims 1 to 16, wherein the therapeutic agent is co-administered with an immunosuppressant.

19. The therapeutic agent for use in the treatment of cardiac warm ischemia reperfusion injury according to any one of claims 1 to 18, wherein the cardiac warm ischemia reperfusion injury is consequent to myocardial infarction, chronic myocardial infarction, acute myocardial infarction (STEMI), treatment of the myocardial infarction aimed at restoring blood flow to the ischemic areas of the heart, cardiovascular arrest, coronary artery bypass graft surgery, coronary angioplasty, transient vasospasm, acute coronary syndromes (NSTEMI), cardioplegia, or vessel ligation and reperfusion during heart transplant surgery.

20. The therapeutic agent for use in the treatment of cardiac warm ischemia reperfusion injury according to any one of claims 1 to 19, wherein the cardiac warm ischemia reperfusion injury is consequent to acute myocardial infarction.
